# EUROPEAN PATENT APPLICATION

(11) **EP 4 491 229 A2**
(43) Date of publication of application: **15.01.2025**
(21) Application number: 24210935.3
(22) Date of filing: 07.02.2020
(51) Int. Cl.: A61P 27/02

(54) **CODING RNA ADMINISTERED INTO THE SUPRACHOROIDAL SPACE IN THE TREATMENT OF OPHTALMIC DISEASES**

(30) Priority: 08.02.2019 WO PCT/EP2019/053189
(62) Divisional of application: 20702837.4
(71) Applicant: CureVac SE, 72076 Tübingen (DE)
(72) Inventor: Rejman, Joanna, 72076 Tübingen (DE); Baumhof, Patrick, 72076 Tübingen (DE)
(74) Representative: Graf von Stosch Patentanwaltsgesellschaft mbH

(57) **Abstract**

The invention relates *inter alia* to compositions comprising coding RNA for use in treatment of an ophthalmic disease, disorder or condition, wherein said composition is administered into the suprachoroidal space of a subject in need of treatment. Additionally, methods of treating or preventing ophthalmic diseases, disorders or conditions are provided.

## Description

### Introduction

The invention relates *inter alia* to a composition comprising coding RNA for use in treatment or prevention of an ophthalmic disease, disorder or condition, wherein said composition is administered into the suprachoroidal space of a subject in need thereof. Additionally, methods of treating or preventing ophthalmic diseases, disorders or conditions are provided.

Ocular diseases are a major health problem worldwide. An ocular disease can occur in many tissues of the eye (e.g., cornea, sclera, iris and pupil, lens, ciliary muscle, ciliary body, choroid, vitreous, retina and optic nerve, macula, etc.). Ocular diseases can be caused by infections (e.g., viruses, parasites, bacteria, and fungi), cancer, genetic causes, injuries, inflammations, post-surgical complications etc.

RNA-based therapeutics can be used in e.g. passive and active immunotherapy, protein replacement therapy, or genetic engineering. Accordingly, RNA has the potential to provide highly specific and individual treatment options for the therapy of a large variety of ophthalmic diseases, disorders or conditions.

However, the effectiveness of RNA therapeutics is limited by the delivery of the RNA to the site of disease in a spatially and temporally controlled manner. Several hurdles exist in implementing an effective treatment strategy for ocular diseases and disorders, mainly due to the unique anatomy and physiology of the eye. The combination of static barriers such as different layers and regions of the eye, and dynamic barriers such as blood flow, lymphatic clearance, tear dilution, or ribonucleases, pose a significant challenge for ocular RNA drug delivery.

In most applications that require a local administration of RNA, tissue distribution may be a limiting factor. Improved tissue distribution may enable the uptake of the RNA drug by higher number of therapeutically relevant cells, which will then produce the protein encoded by the RNA (e.g. therapeutic protein, a CRISPR-associated endonuclease etc.). Usually, the levels of protein production relate closely with the numbers of transfected cells and, as a result, with the observed activity or therapeutic effect.

Improved tissue distribution is particularly relevant for medical treatments of the eye, where established methods of administration bring along some drawbacks e.g. in terms of distribution of the injected RNA within the eye.

Sub-retinal injection is an invasive method to apply the RNA underneath the retina which is accompanied by the formation of an injection bleb. In that method, the distribution of the injected RNA is largely restricted to the site of injection. Another method is the injection into the vitreous ("intravitreal"), which may lead to the deposition of RNA within the vitreous that fills the space between the lens and the retina. However, injected RNA has to diffuse through the vitreous to reach the therapeutically relevant cells (e.g. retinal cells). The vitreous humour contains no blood vessels, 98-99% of its volume is water, and has a viscosity two to four times that of water, giving it a gelatinous consistency and a charged surface. Such aspects are limiting factors for the diffusion of a potential RNA medicament within the vitreous, and therefore limit the access of RNA to cells in the posterior segment (e.g. retinal cells).

There are various pathological conditions which would highly profit if the RNA drug could be delivered to the posterior segment of the eye. Many inflammatory and proliferative diseases are associated with cells and tissues in the posterior region of the eye. In addition, many choroidal and vascular deficiencies are associated with inflammatory responses, proliferation, and neovascularization and would require long term treatment.

However, as outlined above, it is problematic to deliver effective doses of RNA to the posterior segment using conventional administration methods and routes of delivery. Accordingly, an object of the invention is to provide improved delivery and application methods for RNA or compositions comprising RNA into the eye. A further object of the invention is to provide coding RNA and compositions comprising said coding RNA suitable for ocular delivery.

The objects outlined above are solved by the claimed subject matter of the invention.

### Definitions

For the sake of clarity and readability the following definitions are provided. Any technical feature mentioned for these definitions may be read on each and every embodiment of the invention. Additional definitions and explanations may be specifically provided in the context of these embodiments.

Percentages in the context of numbers should be understood as relative to the total number of the respective items. In other cases, and unless the context dictates otherwise, percentages should be understood as percentages by weight (wt.-%).

Antibody, antibody fragment: As used herein, the term "antibody" includes both an intact antibody and an antibody fragment. Typically, an intact "antibody" is an immunoglobulin that specifically binds to a particular antigen. An antibody may be a member of any immunoglobulin class, including any of the human classes: IgG, IgM, IgE, IgA and IgD. Typically, an intact antibody is a tetramer. Each tetramer consists of two identical pairs of polypeptide chains, each pair having a "light" chain and a "heavy" chain. An "antibody fragment" includes a portion of an intact antibody, such as the antigen-binding or variable region of an antibody. Examples of antibody fragments include Fab, Fab ', F (ab') 2 and Fv fragments; the tribes; Tetra; linear antibodies; single-chain antibody molecules; and multi specific antibodies formed from antibody fragments. E.g., the antibody fragments comprise isolated fragments, "Fv" fragments consisting of heavy and light chain variable regions, recombinant single chain polypeptide molecules in which the light and heavy chain variable regions are linked together by a peptide linker ("ScFv Proteins") and minimal recognition units consisting of amino acid residues that mimic the hypervariable region. Examples of antigen-binding fragments of an antibody include, but are not limited to, Fab fragment, Fab 'fragment, F (ab') 2 fragment, scFv fragment, Fv fragment, dsFv diabody, dAb fragment, fragment Fd ', Fd fragment and an isolated complementarity determining region (CDR). Suitable antibodies that may be encoded by the coding RNA of the invention include monoclonal antibodies, polyclonal antibodies, antibody mixtures or cocktails, human or humanized antibodies, chimeric antibodies, Fab fragments, or bispecific antibodies.

Cationic, cationisable: Unless a different meaning is clear from the specific context, the term "cationic" means that the respective structure bears a positive charge, either permanently or not permanently but in response to certain conditions such as e.g. pH. Thus, the term "cationic" covers both "permanently cationic" and "cationisable". The term "cationisable" as used herein means that a compound, or group or atom, is positively charged at a lower pH and uncharged at a higher pH of its environment. Also in non-aqueous environments where no pH value can be determined, a cationisable compound, group or atom is positively charged at a high hydrogen ion concentration and uncharged at a low concentration or activity of hydrogen ions. It depends on the individual properties of the cationisable or polycationisable compound, in particular the pKa of the respective cationisable group or atom, at which pH or hydrogen ion concentration it is charged or uncharged. In diluted aqueous environments, the fraction of cationisable compounds, groups or atoms bearing a positive charge may be estimated using the so-called Henderson-Hasselbalch equation which is well-known to a person skilled in the art. E.g., if a compound or moiety is cationisable, it is preferred that it is positively charged at a pH value of about 1 to 9, preferably 4 to 9, 5 to 8 or even 6 to 8, more preferably of a pH value of or below 9, of or below 8, of or below 7, most preferably at physiological pH values, e.g. about 7.3 to 7.4, i.e. under physiological conditions, particularly under physiological salt conditions of the cell in vivo. In embodiments, it is preferred that the cationisable compound or moiety is predominantly neutral at physiological pH values, e.g. about 7.0-7.4, but becomes positively charged at lower pH values. In some embodiments, the preferred range of pKa for the cationisable compound or moiety is about 5 to about 7.

Derived from: The term "derived from" as used throughout the present specification in the context of a nucleic acid, i.e. for a nucleic acid "derived from" (another) nucleic acid, means that the nucleic acid, which is derived from (another) nucleic acid, shares e.g. at least about 70%, 80%, 85%, 86%, 87%, 88%, 89%, 90%, 91%, 92%, 93%, 94%, 95%, 96%, 97%, 98%, or about 99% sequence identity with the nucleic acid from which it is derived. The skilled person is aware that sequence identity is typically calculated for the same types of nucleic acids, i.e. for DNA sequences or for RNA sequences. Thus, it is understood, if a DNA is "derived from" an RNA or if an RNA is "derived from" a DNA, in a first step the RNA sequence is converted into the corresponding DNA sequence (in particular by replacing the uracils (U) by thymidines (T) throughout the sequence) or, vice versa, the DNA sequence is converted into the corresponding RNA sequence (in particular by replacing the T by U throughout the sequence). Thereafter, the sequence identity of the DNA sequences or the sequence identity of the RNA sequences is determined. Preferably, a nucleic acid "derived from" a nucleic acid also refers to nucleic acid, which is modified in comparison to the nucleic acid from which it is derived, e.g. in order to increase RNA stability even further and/or to prolong and/or increase protein production. In the context of amino acid sequences, the term "derived from" means that the amino acid sequence, which is derived from (another) amino acid sequence, shares e.g. at least about 70%, 80%, 85%, 86%, 87%, 88%, 89%, 90%, 91%, 92%, 93%, 94%, 95%, 96%, 97%, 98%, or about 99% sequence identity with the amino acid sequence from which it is derived. Thus, it is understood, if e.g. a protein is "derived from" a certain protein, the protein that is "derived from" may represent a variant or fragment of said respective protein, sharing a certain percentage of sequence identity.

CRISPR-associated protein: The term "CRISPR-associated protein" will be recognized and understood by the person of ordinary skill in the art. The term "CRISPR-associated protein" refers to RNA-guided endonucleases that are part of a CRISPR (Clustered Regularly Interspaced Short Palindromic Repeats) system (and their homologs, variants, fragments or derivatives), which is used by prokaryotes to confer adaptive immunity against foreign DNA elements. CRISPR-associated proteins include, without limitation, Cas9, Cpf1 (Cas12), C2c1, C2c3, C2c2, Cas13, CasX and CasY. As used herein, the term "CRISPR-associated protein" includes wild-type proteins as well as homologs, variants, fragments and derivatives thereof. Therefore, when referring to artificial nucleic acid molecules encoding Cas9, Cpf1 (Cas12), C2c1, C2c3, and C2c2, Cas13, CasX and CasY, said artificial nucleic acid molecules may encode the respective wild-type proteins, or homologs, variants, fragments and derivatives thereof. Besides Cas9 and Cas12 (Cpf1), several other CRISPR-associated protein exist that are suitable for genetic engineering in the context of the invention, including Cas13, CasX and CasY; e.g. Cas13 i.e. WP15770004, WP18451595, WP21744063, WP21746774, ERK53440, WP31473346, CVRQ01000008, CRZ35554, WP22785443, WP36091002, WP12985477, WP13443710, ETD76934, WP38617242, WP2664492, WP4343973, WP44065294, ADAR2DD, WP47447901, ERI81700, WP34542281, WP13997271, WP41989581, WP47431796, WP14084666, WP60381855, WP14165541, WP63744070, WP65213424, WP45968377, EHO06562, WP6261414, EKB06014, WP58700060, WP13446107, WP44218239, WP12458151, ERJ81987, ERJ65637, WP21665475, WP61156637, WP23846767, ERJ87335, WP5873511, WP39445055, WP52912312, WP53444417, WP12458414, WP39417390, EOA10535, WP61156470, WP13816155, WP5874195, WP39437199, WP39419792, WP39431778, WP46201018, WP39442171, WP39426176, WP39418912, WP39434803, WP39428968, WP25000926, EFU31981, WP4343581, WP36884929, BAU18623, AFJ07523, WP14708441, WP36860899, WP61868553, KJJ86756, EGQ18444, EKY00089, WP36929175, WP7412163, WP44072147, WP42518169, WP44074780, WP15024765, WP49354263, WP4919755, WP64970887, WP61710138); CasX ( i.e. OGP07438, OHB99618); CasY( i.e. OJI08769, OGY82221, OJI06454, APG80656, OJI07455, OJI09436, PIP58309).

Guide RNA: As used herein, the term "guide RNA" (gRNA) thus relates to any RNA molecule capable of targeting a CRISPR-associated protein as defined above to a target DNA sequence of interest. In the context of the invention, the term guide RNA has to be understood in its broadest sense, and may comprise two-molecule gRNAs ("tracrRNA/crRNA") comprising crRNA ("CRISPR RNA" or "targeter-RNA" or "crRNA" or "crRNA repeat") and a corresponding tracrRNA ("trans-acting CRISPR RNA" or "activator-RNA" or "tracrRNA") molecule, or single-molecule gRNAs. A "sgRNA" typically comprises a crRNA connected at its 3' end to the 5' end of a tracrRNA through a "loop" sequence.

Fragment: The term "fragment" as used throughout the present specification in the context of a nucleic acid sequence or an amino acid (aa) sequence may typically be a shorter portion of a full-length sequence of e.g. a nucleic acid sequence or an amino acid sequence. A fragment typically consists of a sequence that is identical to the corresponding stretch within the full-length sequence. The term "fragment" as used throughout the present specification in the context of proteins or peptides may, typically, comprise a sequence of a protein or peptide as defined herein, which is, with regard to its amino acid sequence (or its encoded nucleic acid molecule), N-terminally and/or C-terminally truncated compared to the amino acid sequence of the original (native) protein (or its encoded nucleic acid molecule). Such truncation may thus occur either on the aa level or correspondingly on the nucleic acid level. A sequence identity with respect to such a fragment as defined herein may therefore preferably refer to the entire protein or peptide as defined herein or to the entire (coding) nucleic acid molecule of such a protein or peptide. Fragments of antigenic proteins or peptides may comprise at least one epitope of those proteins or peptides. Furthermore also domains of a protein, like the extracellular domain, the intracellular domain or the transmembrane domain and shortened or truncated versions of a protein may be understood to comprise a fragment of a protein.

Heterologous: The terms "heterologous" or "heterologous sequence" as used throughout the present specification in the context of a nucleic acid sequence or an amino acid sequence refers to a sequence (e.g. DNA, RNA, amino acid) will be recognized and understood by the person of ordinary skill in the art, and is intended to refer to a sequence that is derived from another gene, from another allele, from another species. Two sequences are typically understood to be "heterologous" if they are not derivable from the same gene or in the same allele. I.e., although heterologous sequences may be derivable from the same organism, they naturally (in nature) do not occur in the same nucleic acid molecule, such as e.g. in the same RNA or protein.

Identity (of a sequence): The term "identity" as used throughout the present specification in the context of a nucleic acid sequence or an amino acid sequence will be recognized and understood by the person of ordinary skill in the art, and is e.g. intended to refer to the percentage to which two sequences are identical. To determine the percentage to which two sequences are identical, e.g. nucleic acid sequences or aa sequences as defined herein, preferably the aa sequences encoded by the nucleic acid sequence as defined herein or the aa sequences themselves, the sequences can be aligned in order to be subsequently compared to one another. Therefore, e.g. a position of a first sequence may be compared with the corresponding position of the second sequence. If a position in the first sequence is occupied by the same residue as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using an algorithm, e.g. an algorithm integrated in the BLAST program.

Nucleic acid: The terms "nucleic acid" or "nucleic acid molecule" will be recognized and understood by the person of ordinary skill in the art, and are e.g. intended to refer to a molecule comprising, preferably consisting of nucleic acid components. The term nucleic acid molecule preferably refers to DNA or RNA. It is preferably used synonymous with the term polynucleotide. Preferably, a nucleic acid or a nucleic acid molecule is a polymer comprising or consisting of nucleotide monomers (natural and/or modified), which are covalently linked to each other by phosphodiester-bonds of a sugar/phosphate-backbone. The term "nucleic acid molecule" also encompasses modified nucleic acid molecules, such as base-modified, sugar-modified or backbone-modified DNA or RNA (e.g. coding RNA) molecules as defined herein.

"Ophthalmic disease disorder or condition" or "ocular disease, disorder or condition": The terms "Ophthalmic disease disorder or condition" or "ocular disease, disorder or condition" will be recognized and understood by the person of ordinary skill in the art, and are e.g. intended to refer to a disease, disorder or condition affecting the eye and/or vision. Ocular/ophthalmic diseases, disorders or conditions can affect one or more of the following parts of the eye: eyelid, lacrimal system and orbit; conjunctiva; sclera, cornea, iris and ciliary body; lens; choroid and retina; vitreous body and globe; optic nerve and visual pathways; and ocular muscles. For example, an ocular or ophthalmic disease, disorder or condition may be caused by a protein deficiency or dysfunctions in the eye or parts of the anatomy associated with vision. An ocular/ophthalmic disease, disorder or condition may be caused by a protein surplus, over expression, and/or over activation in the eye or parts of the anatomy associated with vision.

Nucleic acid sequence/ RNA sequence/ amino acid sequence: The terms "nucleic acid sequence", "RNA sequence" or "amino acid sequence" will be recognized and understood by the person of ordinary skill in the art, and are e.g. intended to refer to particular and individual order of the succession of its nucleotides or amino acids respectively.

About: The term "about" is used when determinants do not need to be identical, i.e. 100% the same. Accordingly, "about" means, that a determinants may diverge by 0.1% to 20%, preferably by 0.1% to 10%; in particular, by 0.5%, 1%, 2%, 3%, 4%, 5%, 6%, 7%, 8%, 9%, 10%, 11%, 12%, 13%, 14%, 15%, 16%, 17%, 18%, 19%, 20%. The skilled person will know that e.g. certain parameters or determinants may slightly vary based on the method how the parameter was determined.

Variant (of a sequence): The term "variant" as used throughout the present specification in the context of a nucleic acid sequence will be recognized and understood by the person of ordinary skill in the art, and is e.g. intended to refer to a variant of a nucleic acid sequence derived from another nucleic acid sequence. E.g., a variant of a nucleic acid sequence may exhibit one or more nucleotide deletions, insertions, additions and/or substitutions compared to the nucleic acid sequence from which the variant is derived. A variant of a nucleic acid sequence may at least 50%, 60%, 70%, 80%, 90%, or 95% identical to the nucleic acid sequence the variant is derived from. The variant is a functional variant in the sense that the variant has retained at least 50%, 60%, 70%, 80%, 90%, or 95% or more of the function of the sequence where it is derived from. A "variant" of a nucleic acid sequence may have at least 70%, 75%, 80%, 85%, 90%, 95%, 98% or 99% nucleotide identity over a stretch of at least 10, 20, 30, 50, 75 or 100 nucleotide of such nucleic acid sequence.

The term "variant" as used throughout the present specification in the context of proteins or peptides will be recognized and understood by the person of ordinary skill in the art, and is e.g. intended to refer to a proteins or peptide variant having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property. "Variants" of proteins or peptides as defined herein may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, e.g., an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra). A "variant" of a protein or peptide may have at least 70%, 75%, 80%, 85%, 90%, 95%, 98% or 99% amino acid identity over a stretch of at least 10, 20, 30, 50, 75 or 100 amino acids of such protein or peptide. Preferably, a variant of a protein comprises a functional variant of the protein, which means that the variant exerts the same effect or functionality or at least 40%, 50%, 60%, 70%, 80%, 90%, or 95% of the effect or functionality as the protein it is derived from.

### Short description of the invention

The present invention is based on the finding that a protein, e.g. a therapeutic protein, encoded by a coding RNA of the invention can efficiently be expressed in a mammalian eye upon administration of said RNA into the suprachoroidal space (SCS). That was surprising, as the art taught that the SCS delivery route would be merely suitable for small molecules, and therefore not preferable for large molecules, as rapid clearance of larger molecules (such as RNA) may occur. For example, Olsen *et al* showed that large biological agents have a more rapid clearance from the SCS than comparable intravitreal injections of the same agent (see Olsen, Timothy W., et al., Investigative ophthalmology & visual science 52.7 (2011): 4749-4756). A composition comprising coding RNA preferably in association or in complexation with e.g. a polymeric carrier, a polycationic protein or peptide, a lipid nanoparticle (LNP), or a liposome, resulted in expression and activity of an encoded protein in the cells of the eye after administration into the SCS (see **Example 2).** As described herein including in the examples, the inventors have successfully delivered coding RNA via the SCS, resulting in robust protein expression in the eye. Example 3 shows that therapeutically relevant cell types (e.g. retinal cells, photoreceptors) were transfected using after administration of a long coding RNA into the SCS (see **Example 3).** The inventors have demonstrated, for the first time, that delivery into the SCS may be used to effectively deliver coding RNA encoding therapeutic proteins or antigenic proteins into the eye, providing an effective yet unexpected solution for this difficult problem of ocular RNA delivery. Moreover, the finding that therapeutically relevant cell types can be transfected with coding RNA opens as yet unexpected treatment options for various diseases associated with defects or deficiencies of cells in the retina (e.g. photoreceptors).

In a **first aspect,** the present invention relates to a composition comprising at least one coding RNA comprising at least one coding sequence encoding at least one peptide or protein **for use** in treatment or prevention of an ophthalmic disease, disorder or condition, wherein said composition is administered into the SCS to a subject in need thereof.

In a **second aspect,** the present invention relates to a kit or kit of parts, preferably **for use** in treatment or prevention of an ophthalmic disease, disorder or condition, wherein said kit or kit of parts for use comprises at least one composition for use according to the first aspect, and optionally, a liquid vehicle for solubilising, and/or technical instructions providing information on administration and dosage of the components.

In a **third aspect,** the invention relates to a method of treating or preventing of an ophthalmic disease, disorder or condition, wherein the method comprises applying or administering to a subject in need thereof the composition of the first aspect, or the kit or kit of parts of the second aspect via administration into the SCS. In particular, a method for gene therapy is provided, wherein the method comprises applying or administering to a subject in need thereof a composition comprising a coding RNA encoding at least one CRISPR-associated endonuclease, and, optionally, at least one guide RNA, via administration into the SCS.

In a **fourth aspect,** the present invention relates to a method of delivering a coding RNA molecule to cells and/or tissues of the eye of a subject, wherein delivering is performed via administration into the SCS.

### Detailed Description of the invention

The present application is filed together with a sequence listing in electronic format, which is part of the description of the present application (WIPO standard ST.25). The information contained in the electronic format of the sequence listing filed together with this application is incorporated herein by reference in its entirety. For many sequences, the sequence listing also provides additional detailed information, e.g. regarding certain structural features, sequence optimizations, GenBank identifiers, or additional detailed information regarding its coding capacity. In particular, such information is provided under numeric identifier <223> in the WIPO standard ST.25 sequence listing. Accordingly, information provided under said numeric identifier <223> is explicitly included herein in its entirety and has to be understood as integral part of the description of the underlying invention.

### Composition for use in treatment or prevention of an ophthalmic disease, disorder, or condition

In a **first aspect,** the invention is *inter alia* directed to a composition comprising at least one coding RNA, wherein said at least one coding RNA comprises at least one coding sequence encoding at least one peptide or protein **for use** in treatment or prevention of an ophthalmic disease, disorder or condition, wherein said composition is administered into the SCS to a subject in need thereof.

The term "treatment" or "treating" of a disease, disorder or condition includes preventing or protecting against the disease, disorder or condition (that is, causing the clinical symptoms not to develop); inhibiting the disease, disorder or condition (i.e., arresting/suppressing the development of clinical symptoms); and/or relieving the disease, disorder or condition (i.e., causing the regression of clinical symptoms). As will be appreciated, it is not always possible to distinguish between "preventing" and "suppressing" a disease, disorder or condition since the ultimate inductive event or events may be unknown or latent. Accordingly, the term "prophylaxis" will be understood to constitute a type of "treatment" that encompasses "preventing" and "suppressing." The term "treatment" thus also includes "prophylaxis". The term "treatment" also includes prevention ".

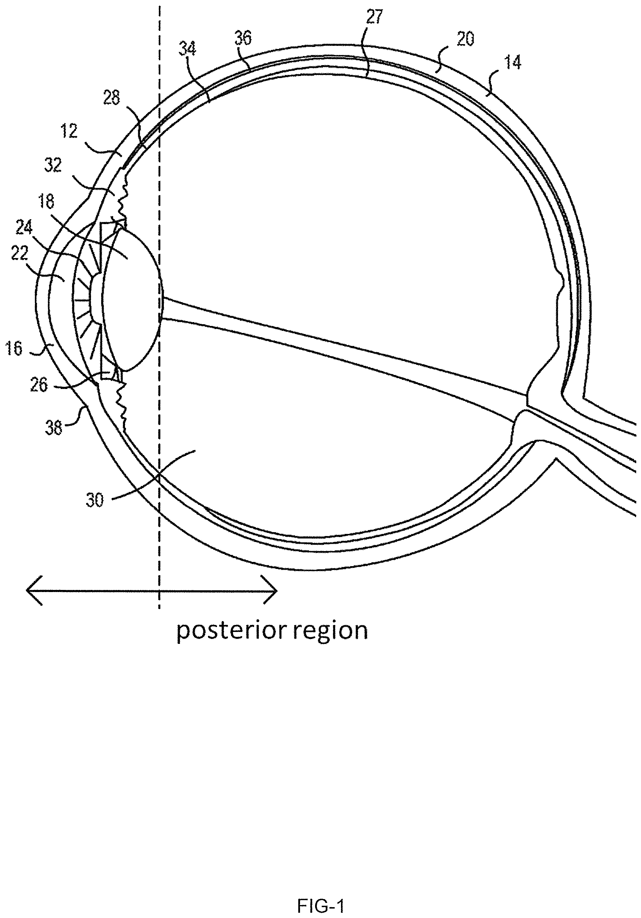

As used herein, "ocular tissue" and "eye" include both the anterior segment of the eye (i.e., the portion of the eye in front of the lens) and the posterior segment of the eye (i.e., the portion of the eye behind the lens) as shown in **Figure 1****.** The eye includes both, an anterior region or segment 12 (the portion of the eye in front of and including the lens) and a posterior region or segment 14 (the portion of the eye behind the lens). The anterior segment 12 is bounded by the cornea 16 and the lens 18, while the posterior segment 14 is bounded by the sclera 20 and the lens 18. The anterior segment 12 is further subdivided into the anterior chamber 22, between the iris 24 and the cornea 16, and the posterior chamber 26, between the lens 18 and the iris 24. The cornea 16 and the sclera 20 collectively form a limbus 38 at the point at which they meet. The exposed portion of the sclera 20 on the anterior segment 12 of the eye is protected by a clear membrane referred to as the conjunctiva 45. Underlying the sclera 20 is the choroid 28 and the retina 27, collectively referred to as retina choroidal tissue. A vitreous humour 30 (also referred to as the "vitreous") is disposed between a ciliary body 32 (including a ciliary muscle and a ciliary process) and the retina 27. The anterior portion of the retina 27 forms an ora serrata 34. The loose connective tissue, or potential space, between the choroid 28 and the sclera 20 is referred to as the suprachoroid, comprising the suprachoroidal space (SCS) 36.

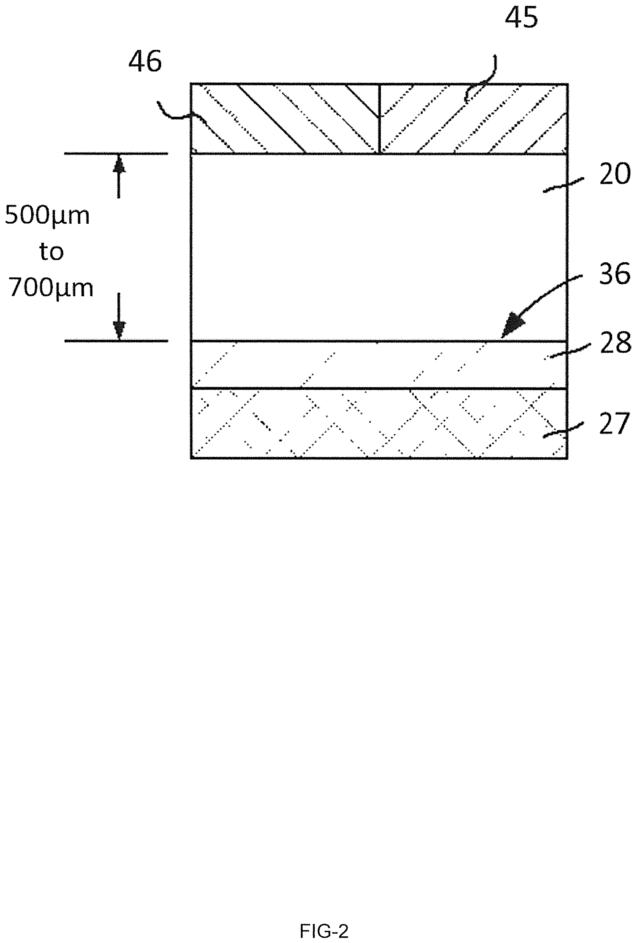

The term "suprachoroidal space" is used interchangeably with suprachoroidal, SCS, suprachoroid and suprachoroidia. The SCS extends from the anterior portion of the eye posterior to the ciliary body to the posterior portion of the eye up to the optic nerve, traversing the circumference of the posterior segment of the eye. The SCS is a space in the eye located between the choroid (the vascular layer of the eye) and the sclera (the outer layer of the eye). As shown in **Figure 2****,** the sclera 20 with surrounding Tenon's Capsule 46 or conjunctiva 45, SCS 36, choroid 28, and retina 27, substantially without fluid and/or tissue separation in the SCS 36.

The coding RNA that is to be administered as a composition into the SCS may be most effective as the coding RNA can be delivered directly, close to, or adjacent to the part of the eye that requires a therapeutic treatment, such as, e.g., the choroid or retina (e.g., for targeting diseases of the posterior segment or region, see for reference **Figure 1****).**

The delivery of RNA macromolecules to the eye is extremely difficult, particularly delivery of an RNA macromolecule to the posterior segment or posterior region. Many inflammatory and proliferative diseases in the posterior region of the eye require long term pharmacological treatment. In addition, many choroidal maladies that are associated with inflammatory responses, proliferation, and neovascularization require long term treatment. It is difficult to deliver effective doses of RNA to the posterior segment using conventional delivery methods and routes of delivery. Administration of a composition comprising RNA to the SCS may enable access to posterior segment ocular tissue while limiting exposure, and potential side effects, to the anterior segment, and other areas of the eye. SCS delivery may also avoid direct entry into the eye (e.g. intravitreal injection, sub-retinal injection) and decreases the risks for endophthalmitis, retinal detachment, and potentially cataract formation. Accordingly, SCS administration of a composition comprising RNA may result in improved expression and/or improved activity of the encoded peptide or protein in the eye, wherein said delivery route has superior safety characteristics as compared to classical routes. Not wishing to be bound by theory, it is believed that upon entering the SCS, the composition comprising coding RNA flows circumferentially from the insertion site toward e.g. the retinochoroidal tissue, macula, and optic nerve in the posterior segment of the eye as well as anteriorly toward the uvea and ciliary body. In addition, a portion of the administered composition comprising at least one coding RNA may remain in the SCS as a depot, or remain in tissue overlying the SCS, e.g. the sclera, near the microneedle insertion site, serving as additional depot of the drug formulation that subsequently can diffuse into the SCS and into other adjacent posterior tissues.

In preferred embodiments, SCS administration of the composition for use may result in (improved) expression and/or (improved) activity of the encoded peptide or protein in cells and/or tissues of the eye.

In preferred embodiments, SCS administration of the composition for use may result in expression of the coding RNA and hence to a measurable amount of peptide or protein in cells and/or tissues of the eye.

The term "expression" has to be understood as translation of the administered coding RNA of the composition into measurable amounts of protein. Protein levels may be detected by various methods in the art, including antibody based detection methods or mass spectrometry. In embodiments, expression of the encoded peptide or protein is essentially restricted to the eye. Accordingly, it is preferred that SCS administration of the composition for use results in expression and hence to a measurable amount of peptide or protein in the eye.

The term "activity" has to be understood as a result of the expressed coding RNA (that is provided by the composition for use of the first aspect). In the context of the invention, activity may be determined by biological assays or animal studies. E.g., the activity of a Cas9 protein may be determined by analyzing the occurrence of genetic repair in the treated eye. In embodiments, activity of the encoded peptide or protein is essentially restricted to the eye. Accordingly, it is preferred that SCS administration of the composition for use results in activity of the encoded peptide or protein in the eye.

Preferably, expression and/or activity may be in cells or tissues in the back of the eye or the posterior segment.

The posterior segment of the eye encompasses, without limitation, sclera, choroid, retina, Bruch's membrane, vitreous humor, optic nerve, and retinal blood vessels.

Accordingly, cells and tissues located in the posterior segment of the eye that may be addressed via SCS delivery of the composition may comprise sclera, choroid, retina, optic nerve, macula, scleral cells, choroid plexus epithelial cells, retinal cells (photoreceptors, bipolar cells, ganglion cells, horizontal cells, and amacrine cells), retinal pigment epithelium, bruch's membrane, or retinal blood vessels.

In preferred embodiments, administration of the composition for use via SCS may result in expression of the peptide or protein (provided by the coding RNA of the composition) mainly located or essentially restricted to therapeutically relevant cells or tissues, including e.g. the choroid, or choroidal tissues, retinal cells, retinal pigment epithelium, Bruch's membrane, while maintaining low levels of expression in other tissues or cells in the eye.

In embodiments, administration of the composition comprising coding RNA for use via SCS may result in expression of the peptide or protein that is substantially increased compared to intravitreal and/or sub-retinal administration.

In embodiments, administration of the composition comprising coding RNA for use via SCS may result in expression of the encoded peptide or protein that is more restricted to the therapeutically relevant cells compared to intravitreal administration and/or sub-retinal administration.

In preferred embodiments, the composition for use is administered into the SCS, wherein administration of the composition leads to translation of the encoded peptide or protein, thereby exerting a therapeutic effect.

Therapeutic effects in that context may be reduced neovascularization, reduced inflammation, neuroprotection, complement inhibition, reduced drusen formation, reduced scar formation, and/or reduction in choriocapillaris.

In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to reduced neovascularization. In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to reduced inflammation. In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to neuroprotection. In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to complement inhibition. In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to reduced drusen formation. In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to reduced scar formation. In preferred embodiments, the invention relates to a composition comprising at least one coding RNA comprising at least one coding sequence encoding at least one therapeutic peptide or protein for use in treatment or prevention of an ophthalmic disease, disorder or condition, wherein said composition is administered into the SCS to a subject in need thereof, wherein administration of said composition leads to reduction in choriocapillaris.

In preferred embodiments, the ophthalmic disease, disorder or condition may be neovascularization, retinal degenerative disease, diabetic eye disease, retinal detachment, optic nerve disease, endocrine disorders, cancer disease, infectious disease, parasitic disease, in particular, pigmentary uveitis (PU), branch retinal vein occlusion (BRVO), central retinal vein occlusion (CRVO), macular edema, cystoid macular edema (CME), uveitic macular edema (UME), cytomegalovirus retinitis, endophthalmitis, scleritis, choriotetinitis, dry eye syndrome, Norris disease, Coat's disease, persistent hyperplastic primary vitreous, familial exudative vitreoretinopathy, Leber congenital amaurosis, X-linked retinoschisis, Leber's hereditary optic neurophathy, uveitis, refraction and accommodation disorders, keratoconus, amblyopia, conjunctivitis, corneal ulcers, dacryocystitis, Duane retraction syndrome, optic neuritis, ocular inflammation, glaucoma, macular degeneration, and uveitis, or any disease, disorder or condition related or associated thereto.

In preferred embodiments, the composition for use is administered into the SCS, wherein the ophthalmic disease, disorder or condition is selected from neovascularization, retinal degenerative disease, diabetic eye disease, retinal detachment, or a cancer disease.

In preferred embodiments, the neovascularization is choroidal neovascularization or retinal neovascularization or a related disease, disorder or condition.

In preferred embodiments, the retinal degenerative disease is age-related macular degeneration (AMD), dry age-related macular degeneration (dry AMD), wet age-related macular degeneration (wet AMD), Stargardt disease, or retinitis pigmentosa (RP), or a related disease, disorder or condition.

In preferred embodiments, the diabetic eye disease is diabetic retinopathy, diabetic macular edema (DME), cataract, and/or glaucoma, or a related disease, disorder or condition.

In preferred embodiments, retinal detachment is from the rhegmatogenous type, the tractional type, and/or the exudative type, or a related disease, disorder or condition.

In embodiments, the cancer disease is melanoma, intraocular lymphoma, Retinoblastoma, choroidal metastasis derived from primary lung cancer, choroidal metastasis derived from primary kidney cancer, choroidal metastasis derived from primary prostate cancer, choroidal metastasis derived from primary breast cancer, metastatic melanomas, or a related disease, disorder or condition.

Further cancer diseases may be eyelid tumors, conjunctival/corneal cancers, uveal cancers, retinal cancers, and orbital/adnexal cancers, in particular Squamous cell carcinoma, squamous neoplasia, Intraocular Melanoma or Retinoblastoma, Sebaceous Carcinoma of the eyelid, Uveal melanoma, iris melanoma, ciliary body melanoma, and choroidal melanoma, or tumor antigens causing a cancer with metastases in the eye (e.g. choroidal metastases, ocular metastases, orbital metastases).

In embodiments, the at least one coding sequence (cds) encodes at least one peptide or protein, wherein said at least one peptide or protein is a therapeutic peptide or protein.

In various embodiments, the length of the encoded peptide or protein may be at least or greater than about 20, 30, 40, 50, 60, 70, 80, 90, 100, 150, 200, 250, 300, 400, 500, 600, 700, 800, 900, 1000, or 1500 amino acids.

In preferred embodiments, the at least one cds encodes at least one therapeutic peptide or protein, wherein said therapeutic peptide or protein may be derived from an antibody, an antibody fragment, an intrabody, a receptor, a binding protein, a CRISPR-associated endonuclease, a transcription factor, an enzyme, a growth factor, a structural protein, cytoplasmic or cytoskeletal proteins, or fragments, variants, or combinations of any of these.

In preferred embodiments, the at least one cds encodes at least one therapeutic peptide or protein, wherein said therapeutic peptide or protein is derived from a CRISPR-associated endonuclease, wherein said CRISPR-associated endonuclease is selected from Cas9, Cpf1, C2c1, C2c3, and C2c2, Cas13, CasX and CasY.

In preferred embodiments, the at least one cds encodes at least one therapeutic peptide or protein, wherein said therapeutic peptide or protein is derived from an antibody, an antibody fragment, wherein said antibody, or antibody fragment is against a platelet derived growth factor (PEDF) or a vascular endothelial growth factor (VEGF).

In preferred embodiments, the at least one cds encodes at least one therapeutic peptide or protein, wherein said therapeutic peptide or protein may be derived from an antibody, an antibody fragment, an intrabody, a receptor, a binding protein, a CRISPR-associated endonuclease, a transcription factor, an enzyme, a growth factor, a structural protein, cytoplasmic or cytoskeletal proteins, or fragments, variants, or combinations of any of these.

In embodiments, the at least one coding RNA of the composition encodes an antibody, an antibody fragment, an intrabody, a receptor, a binding protein, a CRISPR-associated endonuclease, a transcription factor, an enzyme, a growth factor, and/or a structural protein thereby inhibiting, down-regulating, or reducing a protein that is present in excess amount and/or excess activity in an ocular disease, disorder or condition (as defined above).

In other embodiments, the at least one coding RNA of the composition encodes an antibody, an antibody fragment, an intrabody, a receptor, a binding protein, a CRISPR-associated endonuclease, a transcription factor, an enzyme, a growth factor, and/or a structural protein thereby activating, up-regulating or increasing a protein activity that deficient and/or present in low amount and/or low activity in an ocular disease, disorder or condition (as defined above).

Suitably, the coding RNA comprised in the composition for use encodes at least one therapeutic protein as provided in **List 1** below. Therein, Protein/Gene Symbol and the corresponding Name (Symbol: Name) are provided.

### List 1: Suitable therapeutic proteins provided by the coding RNA of the invention

ABCA4: ATP binding cassette subfamily A member 4; ADRB1: adrenoceptor beta 1; ANGPT1: angiopoietin 1; ANGPT2: angiopoietin 2; BEST1: bestrophin 1; CCR3: C-C motif chemokine receptor 3; CD276: CD276 molecule; CD59: CD59 molecule (CD59 blood group); CFD: complement factor D; CHM: CHM, Rab escort protein 1; CHST4: carbohydrate sulfotransferase 4; CNR1: cannabinoid receptor 1; CNTF: ciliary neurotrophic factor; CRYAA: crystallin alpha A; CRYAB: crystallin alpha B; CSF3R: colony stimulating factor 3 receptor; DCN: decorin; DICER1: dicer 1, ribonuclease III; DRD2: dopamine receptor D2; EGFR: epidermal growth factor receptor; EGLN1: egl-9 family hypoxia inducible factor 1; ENG: endoglin; FLT1: fms related tyrosine kinase 1; FLT1(1-758): fms related tyrosine kinase 1 (secreted); FLT1-iso3(sFlt1-14): fms related tyrosine kinase 1 isoform 3 (secreted; P17948-3); FLT1-iso2(sFlt1): fms related tyrosine kinase 1 isoform 2 (secreted; P17948-2); FLT1-iso4: fms related tyrosine kinase 1 isoform 4 (secreted; P17948-4); GUCY1A1: guanylate cyclase 1 soluble subunit alpha 1; GUCY1A2: guanylate cyclase 1 soluble subunit alpha 2; GUCY1B1: guanylate cyclase 1 soluble subunit beta 1; GUCY1B2: guanylate cyclase 1 soluble subunit beta 2 (pseudogene); GUCY2D: guanylate cyclase 2D, retinal; GUCY2F: guanylate cyclase 2F, retinal; HEY1: hes related family bHLH transcription factor with YRPW motif 1; HEPH: hephaestin; HEPHL1: hephaestin like 1; IL1RN: interleukin 1 receptor antagonist; KDR: kinase insert domain receptor; MAG: myelin associated glycoprotein; MERTK: MER proto-oncogene, tyrosine kinase; MYO7A: myosin VIIA; MYOC: myocilin; NOTCH4: notch 4; NR3C1: nuclear receptor subfamily 3 group C member 1; NXNL1: nucleoredoxin like 1; OPA1: OPA1, mitochondrial dynamin like GTPase; OPA3: OPA3, outer mitochondrial membrane lipid metabolism regulator; OPTN: optineurin; PDGFA: platelet derived growth factor subunit A; PDGFB: platelet derived growth factor subunit B; PDGFC: platelet derived growth factor C; PDGFD: platelet derived growth factor D; PDGFRA: platelet derived growth factor receptor alpha; PDGFRB: platelet derived growth factor receptor beta; PGF: placental growth factor; PLXND1: plexin D1; PPP3CA: protein phosphatase 3 catalytic subunit alpha; PPP3CB: protein phosphatase 3 catalytic subunit beta; PPP3CC: protein phosphatase 3 catalytic subunit gamma; PPP3R1: protein phosphatase 3 regulatory subunit B, alpha; PPP3R2: protein phosphatase 3 regulatory subunit B, beta; PRPH2: peripherin 2; PTGDR: prostaglandin D2 receptor; PTGDR2: prostaglandin D2 receptor 2; PTGER1: prostaglandin E receptor 1; PTGER2: prostaglandin E receptor 2; PTGER3: prostaglandin E receptor 3; PTGER4: prostaglandin E receptor 4; PTGFR: prostaglandin F receptor; PTGIR: prostaglandin !2 receptor; RHO: rhodopsin; RLBP1: retinaldehyde binding protein 1; ROCK1: Rho associated coiled-coil containing protein kinase 1; ROCK2: Rho associated coiled-coil containing protein kinase 2; RPE65: RPE65, retinoid isomerohydrolase; RPGR: retinitis pigmentosa GTPase regulator; RS1: retinoschisin 1; SEMA3E: semaphorin 3E; SEMA3: semaphorin 3; SERPINF1: serpin family F member 1; SIRT1: sirtuin 1; SIRT2: sirtuin 2; SIRT3: sirtuin 3; SIRT4: sirtuin 4; SIRT5: sirtuin 5; SIRT6: sirtuin 6; SIRT7: sirtuin 7; SOD1: superoxide dismutase 1; TBXA2R: thromboxane A2 receptor; TEK: TEK receptor tyrosine kinase; TIE1: tyrosine kinase with immunoglobulin like and EGF like domains 1; TNF: tumor necrosis factor; TNFRSF10C: TNF receptor superfamily member 10c; UNC5B: unc-5 netrin receptor B; USH2A: usherin; VEGFA: vascular endothelial growth factor A; VEGFA-isoVEGF165: vascular endothelial growth factor A; VEGFA-isoVEGF165B: vascular endothelial growth factor A; VEGFB: vascular endothelial growth factor B; VEGFC: vascular endothelial growth factor C; VEGFD: vascular endothelial growth factor D; WFS1: wolframin ER transmembrane glycoprotein; aflibercept: aflibercept; etanercept: etanercept; bevacizumab-LC: bevacizumab; bevacizumab-HC: bevacizumab; bevacizumab-RKR: bevacizumab; ranibizumab-LC: ranibizumab; ranibizumab-HC: ranibizumab; ranibizumab-RKR: ranibizumab; anti-CCL11-LC: anti-CCL11; anti-CCL11-HC: anti-CCL11; anti-PDGFB-LC: anti-PDGFB; anti-PDGFB-HC: anti-PDGFB; anti-PDGFA-LC: anti-PDGFB1/anti-PDGFE1; anti-PDGFA-HC: anti-PDGFB1/anti-PDGFE1; bersanlimab-LC: bersanlimab; bersanlimab-HC: bersanlimab; clazakizumab-LC: clazakizumab; clazakizumab-HC: clazakizumab; olokizumab-LC: olokizumab;

Preferred therapeutic peptide or proteins that may be encoded by the cds of the at least one coding RNA are provided in **Table 1.** Additional information regarding each of these suitable amino acid sequences may also be derived from the sequence listing, e.g. the corresponding nucleic acid transcript IDs ("Ensembl Transcript ID") is provided therein under identifier <223> for each sequence.

In preferred embodiments, the ophthalmic disease, disorder or condition may be any deficiency associated with any one of the proteins provided in **List 1.** Accordingly, in embodiments, the ophthalmic disease, disorder or condition may be an ABCA4 deficiency, an ADRB1 deficiency, an ANGPT1 deficiency, an ANGPT2 deficiency, a BEST1 deficiency, a CCR3 deficiency, a CD276 deficiency, a CD59 deficiency, a CFD deficiency, a CHM deficiency, a CHST4 deficiency, a CNR1 deficiency, a CNTF deficiency, a CRYAA deficiency, a CRYAB deficiency, a CSF3R deficiency, a DCN deficiency, a DICER1 deficiency, a DRD2 deficiency, an EGFR deficiency, an EGLN1 deficiency, an ENG deficiency, a FLT1 deficiency, a GUCY1A1 deficiency, a GUCY1A2 deficiency, a GUCY1B1 deficiency, a GUCY1B2 deficiency, a GUCY2D deficiency, a GUCY2F deficiency, a HEY1 deficiency, a HEPH deficiency, a HEPHL1 deficiency, an IL1RN deficiency, a KDR deficiency, a MAG deficiency, a MERTK deficiency, a MYO7A deficiency, a MYOC deficiency, a NOTCH4 deficiency, a NR3C1 deficiency, a NXNL1 deficiency, an OPA1 deficiency, an OPA3 deficiency, an OPTN deficiency, a PDGFA deficiency, a PDGFB deficiency, a PDGFC deficiency, a PDGFD deficiency, a PDGFRA deficiency, a PDGFRB deficiency, a PGF deficiency, a PLXND1 deficiency, a PPP3CA deficiency, a PPP3CB deficiency, a PPP3CC deficiency, a PPP3R1 deficiency, a PPP3R2 deficiency, a PRPH2 deficiency, a PTGDR deficiency, a PTGDR2 deficiency, a PTGER1 deficiency, a PTGER2 deficiency, a PTGER3 deficiency, a PTGER4 deficiency, a PTGFR deficiency, a PTGIR deficiency, a RHO deficiency, a RLBP1 deficiency, a ROCK1 deficiency, a ROCK2 deficiency, a RPE65 deficiency, a RPGR deficiency, a RS1 deficiency, a SEMA3E deficiency, a SEMA3A deficiency, SERPINF1 deficiency, a SIRT1 deficiency, a SIRT2 deficiency, a SIRT3 deficiency, a SIRT4 deficiency, a SIRT5 deficiency, a SIRT6 deficiency, a SIRT7 deficiency, a SOD1 deficiency, a TBXA2R deficiency, a TEK deficiency, a TIE1 deficiency, a TNF deficiency, a TNFRSF10C deficiency, an UNC5B deficiency, an USH2A deficiency, a VEGFA deficiency, a VEGFA-isoVEGF165 deficiency, a VEGFA-isoVEGF165B deficiency, a VEGFB deficiency, a VEGFC deficiency, a VEGFD deficiency, a WFS1 deficiency, a CCL11 deficiency, a DPP4 deficiency, an IL6R deficiency, a TNFRSF10A deficiency, a TNFRSF10B deficiency, a TNFRSF1A deficiency, or a TNFRSF1 B deficiency.

In **Table 1** below, each row corresponds to suitable therapeutic proteins that may be encoded by the coding RNA of the composition for use (row 1 to 125). Column A provides Gene/Protein Symbols (derived from Ensembl Gene ID) of the suitable therapeutic proteins. The "Gene/Protein Symbol" used to describe suitable therapeutic proteins of the invention are also used throughout the description of the invention (as described above) as well as in the ST25 sequence listing under identifier <223>. Column B provides a further description of the respective therapeutic protein. Column C indicates the corresponding Ensembl Gene ID [ENSG00000...]. Column D provides protein SEQ ID NOs of the respective amino acid sequences of the indicated therapeutic proteins.

As an example, row 1 of **Table 1** provides "ABCA4" (row 1, Column A), an "ATP binding cassette subfamily A member 4" (row 1, Column B), that can be identified by the corresponding Ensembl Gene ID "ENSG00000"198691.1 (row 1, Column C), having the amino acid sequences according to **SEQ ID NOs: 2** or **3** (row 1, Column D).

**Table 1: Suitable therapeutic proteins and sequences provided by the coding RNA of the composition for use**

| | **A** | **B** | **C** | **D** |
|---|---|---|---|---|
| 1 | ABCA4 | ATP binding cassette subfamily A member 4 | 198691.1 | 2, 3 |
| 2 | ADRB1 | adrenoceptor beta 1 | 043591.5 | 4 |
| 3 | ANGPT1 | angiopoietin 1 | 154188.9 | 5-8 |
| 4 | ANGPT2 | angiopoietin 2 | 091879.13 | 9-12 |
| 5 | BEST1 | bestrophin 1 | 167995.15 | 13-17 |
| 6 | CCR3 | C-C motif chemokine receptor 3 | 183625.14 | 18, 19 |
| 7 | CD276 | CD276 molecule | 103855.17 | 20-26 |
| 8 | CD59 | CD59 molecule (CD59 blood group) | 085063.15 | 27, 28 |
| 9 | CFD | complement factor D | 197766.7 | 29, 30 |
| 10 | CHM | CHM, Rab escort protein 1 | 188419.13 | 31, 32 |
| 11 | CHST4 | carbohydrate sulfotransferase 4 | 140835.9 | 33 |
| 12 | CNR1 | cannabinoid receptor 1 | 118432.12 | 34-36 |
| 13 | CNTF | ciliary neurotrophic factor | 242689.2 | 37 |
| 14 | CRYAA | crystallin alpha A | 160202.7 | 38-40 |
| 15 | CRYAB | crystallin alpha B | 109846.7 | 41-43 |
| 16 | CSF3R | colony stimulating factor 3 receptor | 119535.17 | 44-46 |
| 17 | DCN | decorin | 011465.16 | 47-54 |
| 18 | DICER1 | dicer 1, ribonuclease III | 100697.14 | 55-57 |
| 19 | DRD2 | dopamine receptor D2 | 149295.13 | 58-61 |
| 20 | EGFR | epidermal growth factor receptor | 146648.17 | 62-67 |
| 21 | EGLN1 | egl-9 family hypoxia inducible factor 1 | 135766.8 | 68 |
| 22 | ENG | endoglin | 106991.13 | 69-71 |
| 23 | FLT1 | fms related tyrosine kinase 1 | 102755.11 | 72-80 |
| 24 | FLT1 (1-758) | fms related tyrosine kinase 1 (secreted) | 102755.11 | 73 |
| 25 | FLT1-iso3(sFlt1-14) | fms related tyrosine kinase 1 isoform 3 | 102755.11 | 74 |
| 26 | FLT1-iso2(sFlt1) | fms related tyrosine kinase 1 isoform 2 | 102755.11 | 75 |
| 27 | FLT1-iso4 | fms related tyrosine kinase 1 isoform 4 | 102755.11 | 77 |
| 28 | GUCY1A1 | guanylate cyclase 1 soluble subunit alpha 1 | 164116.16 | 81-86 |
| 29 | GUCY1A2 | guanylate cyclase 1 soluble subunit alpha 2 | 152402.10 | 87-89 |
| 30 | GUCY1B1 | guanylate cyclase 1 soluble subunit beta 1 | 061918.12 | 90-95 |
| 31 | GUCY1B2 | guanylate cyclase 1 soluble subunit beta 2 (pseudogene) | 123201 | 96 |
| 32 | GUCY2D | guanylate cyclase 2D, retinal | 132518.6 | 97 |
| 33 | GUCY2F | guanylate cyclase 2F, retinal | 101890.4 | 98 |
| 34 | HEY1 | hes related family bHLH transcription factor with YRPW motif 1 | 164683.16 | 99-101 |
| 35 | HEPH | hephaestin | 089472.16 | 102-107 |
| 36 | HEPHL1 | hephaestin like 1 | 181333.11 | 108 |
| 37 | IL1RN | interleukin 1 receptor antagonist | 136689.18 | 109-112 |
| 38 | KDR | kinase insert domain receptor | 128052.9 | 113 |
| 39 | MAG | myelin associated glycoprotein | 105695.14 | 114-117 |
| 40 | MERTK | MER proto-oncogene, tyrosine kinase | 153208.16 | 118-121 |
| 41 | MYO7A | myosin VIIA | 137474.20 | 122-126 |
| 42 | MYOC | myocilin | 034971.16 | 127, 128 |
| 43 | NOTCH4 | notch 4 | 235396.5 | 129-133 |
| 44 | NR3C1 | nuclear receptor subfamily 3 group C member 1 | 113580.14 | 134-137 |
| 45 | NXNL1 | nucleoredoxin like 1 | 171773.2 | 138 |
| 46 | OPA1 | OPA1, mitochondrial dynamin like GTPase | 198836.9 | 139-155 |
| 47 | OPA3 | OPA3, outer mitochondrial membrane lipid metabolism regulator | 125741.4 | 156-158 |
| 48 | OPTN | optineurin | 123240.16 | 159-162 |
| 49 | PDGFA | platelet derived growth factor subunit A | 197461.13 | 163, 164 |
| 50 | PDGFB | platelet derived growth factor subunit B | 100311.16 | 165, 166 |
| 51 | PDGFC | platelet derived growth factor C | 145431.10 | 167-169 |
| 52 | PDGFD | platelet derived growth factor D | 170962.12 | 170, 171 |
| 53 | PDGFRA | platelet derived growth factor receptor alpha | 134853.11 | 172-174 |
| 54 | PDGFRB | platelet derived growth factor receptor beta | 113721.13 | 175, 176 |
| 55 | PGF | placental growth factor | 119630.13 | 177-180 |
| 56 | PLXND1 | plexin D1 | 004399.12 | 181-183 |
| 57 | PPP3CA | protein phosphatase 3 catalytic subunit alpha | 138814.16 | 184-188 |
| 58 | PPP3CB | protein phosphatase 3 catalytic subunit beta | 107758.15 | 189-192 |
| 59 | PPP3CC | protein phosphatase 3 catalytic subunit gamma | 120910.14 | 193-196 |
| 60 | PPP3R1 | protein phosphatase 3 regulatory subunit B alpha | 221823.10 | 197-199 |
| 61 | PPP3R2 | protein phosphatase 3 regulatory subunit B beta | 188386.6 | 200, 201 |
| 62 | PRPH2 | peripherin 2 | 112619.7 | 202 |
| 63 | PTGDR | prostaglandin D2 receptor | 168229.3 | 203, 204 |
| 64 | PTGDR2 | prostaglandin D2 receptor 2 | 183134.4 | 205 |
| 65 | PTGER1 | prostaglandin E receptor 1 | 160951.3 | 206 |
| 66 | PTGER2 | prostaglandin E receptor 2 | 125384.6 | 207, 208 |
| 67 | PTGER3 | prostaglandin E receptor 3 | 050628.20 | 209-218 |
| 68 | PTGER4 | prostaglandin E receptor 4 | 171522.5 | 219 |
| 69 | PTGFR | prostaglandin F receptor | 122420.9 | 220-222 |
| 70 | PTGIR | prostaglandin I2 receptor | 160013.8 | 223-227 |
| 71 | RHO | rhodopsin | 163914.4 | 228 |
| 72 | RLBP1 | retinaldehyde binding protein 1 | 140522.11 | 229, 230 |
| 73 | ROCK1 | Rho associated coiled-coil containing protein kinase 1 | 067900.7 | 231, 232 |
| 74 | ROCK2 | Rho associated coiled-coil containing protein kinase 2 | 134318.13 | 233-236 |
| 75 | RPE65 | RPE65, retinoid isomerohydrolase | 116745.6 | 237 |
| 76 | RPGR | retinitis pigmentosa GTPase regulator | 156313.13 | 238-249 |
| 77 | RS1 | retinoschisin 1 | 102104.8 | 250 |
| 78 | SEMA3E | semaphorin 3E | 170381.13 | 251-254 |
| 79 | SERPINF1 | serpin family F member 1 | 132386.10 | 255, 256 |
| 80 | SIRT1 | sirtuin 1 | 096717.11 | 257-259 |
| 81 | SIRT2 | sirtuin 2 | 068903.19 | 260-267 |
| 82 | SIRT3 | sirtuin 3 | 142082.14 | 268-275 |
| 83 | SIRT4 | sirtuin 4 | 089163.4 | 276 |
| 84 | SIRT5 | sirtuin 5 | 124523.15 | 277-282 |
| 85 | SIRT6 | sirtuin 6 | 077463.14 | 283-287 |
| 86 | SIRT7 | sirtuin 7 | 187531.13 | 288, 289 |
| 87 | SOD1 | superoxide dismutase 1 | 142168.14 | 290, 291 |
| 88 | TBXA2R | thromboxane A2 receptor | 006638.11 | 292-294 |
| 89 | TEK | TEK receptor tyrosine kinase | 120156.20 | 295-298 |
| 90 | TIE1 | tyrosine kinase with immunoglobulin like and EGF like domains 1 | 066056.13 | 299, 300 |
| 91 | TNF | tumor necrosis factor | 232810.3 | 301 |
| 92 | TNFRSF10C | TNF receptor superfamily member 10c | 173535.14 | 302, 303 |
| 93 | UNC5B | unc-5 netrin receptor B | 107731.12 | 304, 305 |
| 94 | USH2A | usherin | 042781.12 | 306, 307 |
| 95 | VEGFA | vascular endothelial growth factor A | 112715.21 | 308-324 |
| 96 | VEGFA-isoVEGF165 | vascular endothelial growth factor A | 112715.21 | 319 |
| 97 | VEGFA-isoVEGF165B | vascular endothelial growth factor A | 112715.21 | 320 |
| 98 | VEGFB | vascular endothelial growth factor B | 173511.9 | 325, 326 |
| 99 | VEGFC | vascular endothelial growth factor C | 150630.3 | 327 |
| 100 | VEGFD | vascular endothelial growth factor D | 165197.4 | 328 |
| 101 | WFS1 | wolframin ER transmembrane glycoprotein | 109501.13 | 329 |
| 102 | aflibercept | aflibercept | | 330 |
| 103 | etanercept | etanercept | | 331 |
| 104 | bevacizumab | bevacizumab | | 332-334 |
| 105 | ranibizumab | ranibizumab | | 335-337 |
| 106 | anti-CCL11 | anti-CCL11 | | 338, 339 |
| 107 | anti-PDGFB | anti-PDGFB | | 340, 341 |
| 108 | anti-PDGFA | anti-PDGFB1/anti-PDGFE1 | | 342, 343 |
| 109 | bersanlimab | bersanlimab | | 344, 345 |
| 110 | clazakizumab | clazakizumab | | 346, 347 |
| 111 | olokizumab | olokizumab | | 348, 349 |
| 112 | sarilumab | sarilumab | | 350, 351 |
| 113 | siltuximab | siltuximab | | 352, 353 |
| 114 | sirukumab | sirukumab | | 354, 355 |
| 115 | DPP4-Fc | dipeptidyl peptidase 4 Fc Chimera | | 356 |
| 116 | IL6R-Fc | interleukin 6 receptor Fc Chimera | | 357 |
| 117 | PDGFRB-Fc | platelet derived growth factor receptor beta Fc Chimera | | 358 |
| 118 | TNFRSF10A-Fc | TNF receptor superfamily member 10a Fc Chimera | | 359 |
| 119 | TNFRSF10B-Fc | TNF receptor superfamily member 10b Fc Chimera | | 360 |
| 120 | TNFRSF1A-Fc | TNF receptor superfamily member 1A Fc Chimera | | 361 |
| 121 | TNFRSF1B-Fc | TNF receptor superfamily member 1B Fc Chimera | | 362 |
| 122 | UNC5B-Fc | UNC5H2/UNC5B Fc Chimera | | 363 |
| 123 | PDGFRB-trap(IgG4) | PDGFRB-trap (IgG4) | | 364 |
| 124 | PDGFRB-trap(IgG1) | PDGFRB-trap (lgG1) | | 365 |
| 125 | SEMA3A | Semaphorin 3A | 075213.10 | 436 |

Accordingly, in preferred embodiments, the peptide or protein is selected from ABCA4, ADRB1, ANGPT1, ANGPT2, BEST1, CCR3, CD276, CD59, CFD, CHM, CHST4, CNR1, CNTF, CRYAA, CRYAB, CSF3R, DCN, DICER1, DRD2, EGFR, EGLN1, ENG, FLT1, FLT1(1-758), FLT1-iso3(sFlt1-14), FLT1-iso2(sFlt1), FLT1-iso4, GUCY1A1, GUCY1A2, GUCY1B1, GUCY1B2, GUCY2D, GUCY2F, HEY1, HEPH, HEPHL1, IL1RN, KDR, MAG, MERTK, MYO7A, MYOC, NOTCH4, NR3C1, NXNL1, OPA1, OPA3, OPTN, PDGFA, PDGFB, PDGFC, PDGFD, PDGFRA, PDGFRB, PGF, PLXND1, PPP3CA, PPP3CB, PPP3CC, PPP3R1, PPP3R2, PRPH2, PTGDR, PTGDR2, PTGER1, PTGER2, PTGER3, PTGER4, PTGFR, PTGIR, RHO, RLBP1, ROCK1, ROCK2, RPE65, RPGR, RS1, SEMA3E, SEMA3A, SERPINF1, SIRT1, SIRT2, SIRT3, SIRT4, SIRT5, SIRT6, SIRT7, SOD1, TBXA2R, TEK, TIE1, TNF, TNFRSF10C, UNC5B, USH2A, VEGFA, VEGFA-isoVEGF165, VEGFA-isoVEGF165B, VEGFB, VEGFC, VEGFD, WFS1, aflibercept, etanercept, bevacizumab, ranibizumab, anti-CCL11, anti-PDGFB, anti-PDGFA, bersanlimab, clazakizumab, olokizumab, sarilumab, siltuximab, sirukumab, DPP4-Fc, IL6R-Fc, PDGFRB-Fc, TNFRSF10A-Fc, TNFRSF10B-Fc, TNFRSF1A-Fc, TNFRSF1B-Fc, UNC5B-Fc, PDGFRB-trap(IgG4), PDGFRB-trap(!gG1), Cas9, Cpf1, C2c1, C2c3, and C2c2, Cas13, CasX and CasY, or a fragment or variant of any of these.

Accordingly, in preferred embodiments, the at least one coding RNA of the composition for use comprises at least one cds, wherein the at least one cds encodes at least one therapeutic peptide or protein comprising or consisting of an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ **ID** NOs: 2 to 365, 436 or a fragment or variant thereof of any of these sequences.

In other preferred embodiments, the at least one coding RNA of the composition for use comprises at least one cds, wherein the at least one cds encodes at least one CRISPR-associated endonuclease comprising or consisting of an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 428-1345, 10999-11001, of published PCT patent application WO2018/172556. Accordingly, SEQ ID NOs: 428-1345, 10999-11001 of WO2018/172556 are herewith incorporated by reference.

In embodiments, the at least one cds encodes at least one peptide or protein, wherein said at least one peptide or protein is a therapeutic peptide or protein.

In other embodiments, the at least one cds encodes at least one antigenic peptide or protein, wherein said antigenic peptide or protein may be derived from tumor antigen, a viral antigen, a bacterial antigen, a fungal antigen, or a parasite antigen.

The term "antigen" as used herein will be recognized and understood by the person of ordinary skill in the art, and is e.g. intended to refer to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. Also fragments, variants and derivatives of peptides or proteins derived from e.g. cancer antigens comprising at least one epitope may be understood as antigens. In the context of the present invention, an antigen may be the product of translation of a provided RNA of the first aspect. The term "antigenic peptide or protein" will be recognized and understood by the person of ordinary skill in the art, and is e.g. intended to refer to a peptide or protein derived from a (antigenic) protein which may stimulate the body's adaptive immune system to provide an adaptive immune response. Therefore an "antigenic peptide or protein" comprises at least one epitope or antigen of the protein it is derived from (e.g. tumor specific antigen).

Suitably, tumor antigens may be derived from antigens causing eyelid tumors, conjunctival/corneal cancers, uveal cancers, retinal cancers, and orbital/adnexal cancers, in particular Squamous cell carcinoma, squamous neoplasia, Intraocular Melanoma or Retinoblastoma, Sebaceous Carcinoma of the eyelid, Uveal melanoma, iris melanoma, ciliary body melanoma, and choroidal melanoma, or tumor antigens causing a cancer with metastases in the eye (e.g. choroidal metastases, ocular metastases, orbital metastases).

According to other embodiments, the coding RNA of the composition for use encodes at least one peptide or protein as defined above and additionally at least one further heterologous peptide or protein element.

Suitably, the at least one further heterologous peptide or protein element may promote/improve secretion of the peptide or protein (e.g. via secretory signal peptides), promote/improve anchoring of the peptide or protein in the plasma membrane (e.g. via transmembrane elements), promote/improve formation of complexes (e.g. via multimerization domains), promote/improve virus-like particle formation (VLP forming sequence), a nuclear localization signal (NLS). In addition, the RNA according may encode peptide linker elements, self-cleaving peptides, immunologic adjuvant sequences or dendritic cell targeting sequences. Suitable multimerization domains may be selected from the list of amino acid sequences according to SEQ ID NOs: 1116-1167 of WO2017/081082, or fragments or variants of these sequences. Suitable transmembrane elements may be selected from the list of amino acid sequences according to SEQ ID NOs: 1228-1343 of WO2017/081082, or fragments or variants of these sequences. Suitable VLP forming sequences may be selected from the list of amino acid sequences according to SEQ ID NOs: 1168-1227 of WO2017/081082, or fragments or variants of these sequences. Suitable peptide linkers may be selected from the list of amino acid sequences according to SEQ ID NOs: 1509-1565 of WO2017/081082, or fragments or variants of these sequences. Suitable self-cleaving peptides may be selected from the list of amino acid sequences according to SEQ ID NOs: 1434-1508 of WO2017/081082, or fragments or variants of these sequences. Suitable immunologic adjuvant sequences may be selected from the list of amino acid sequences according to SEQ ID NOs: 1360-1421 of WO2017/081082, or fragments or variants of these sequences. Suitable dendritic cell targeting sequences may be selected from the list of amino acid sequences according to SEQ ID NOs: 1344-1359 of WO2017/081082, or fragments or variants of these sequences. Suitable secretory signal peptides may be selected from the list of amino acid sequences according to SEQ ID NOs: 1-1115 and SEQ ID NO: 1728 of WO2017/081082, or fragments or variants of these sequences. Suitable nuclear localization signal (NLS) sequences may be selected from the list of amino acid sequences according to SEQ ID NOs: 426, 427, 10575, 381, 382, 384, 11957, 11958-11964 of published PCT patent application WO2018/172556 or fragments or variants of these sequences.

According to preferred embodiments, the coding RNA of the composition for use comprises at least one cds encoding at least one peptide or protein as specified herein, or fragments and variants thereof. In that context, any cds encoding at least one peptide or protein as defined herein or fragments and variants thereof may be understood as suitable cds and may therefore be comprised in the coding RNA.

In embodiments, the length the cds may be at least or greater than about 30, 40, 50, 60, 70, 80, 90, 100, 150, 200, 250, 300, 350, 400, 450, 500, 600, 700, 800, 900, 1000, 1100, 1200, 1300, 1400, 1500, 1600, 1700, 1800, 1900, 2000, 2500, 3000, 3500, 4000, 5000, or 6000 nucleotides. In embodiments, the length of the cds may be in a range of from about 300 to about 2000 nucleotides.

The terms "coding sequence" or "coding region" and "cds" as used herein will be recognized and understood by the person of ordinary skill in the art, and are e.g. intended to refer to a sequence of several nucleotides which may be translated into a peptide or protein. In the context of the present invention a cds is preferably an RNA sequence, consisting of a number of nucleotide triplets, starting with a start codon and preferably terminating with one stop codon. In embodiments, the cds of the RNA may terminate with or two or more stop codons. The first stop codon of the two or more stop codons may be TGA or UGA and the second stop codon of the two or more stop codons may be selected from TAA, TGA, TAG, UAA, UGA or UAG.

In preferred embodiments, the coding RNA of the composition for use may comprise or consist of at least one cds encoding any one of **SEQ ID NOs: 2-365, 436** or SEQ ID NOs: 428-441, 10999-11001, 442-1345 of WO2018/172556 or fragments of variants thereof.

It has to be understood that, on nucleic acid level, any RNA sequence which encodes an amino acid sequences being identical to **SEQ ID NOs: 2-365, 436** or SEQ ID NOs: 428-441, 10999-11001, 442-1345 of WO2018/172556 or fragments or variants thereof, or any RNA sequence which encodes amino acid sequences being at least 70%, 80%, 85%, 86%, 87%, 88%, 89%, 90%, 91%, 92%, 93%, 94%, 95%, 96%, 97%, 98%, or 99% identical to any one of **SEQ ID NOs: 2-365, 436** or SEQ ID NOs: 428-441, 10999-11001, 442-1345 of WO2018/172556 or fragments or variants thereof, may be selected and may accordingly be understood as suitable cds, and may therefore be comprised in the coding RNA of the composition for use.

In preferred embodiments, the coding RNA of the composition for use is an artificial RNA.

The term "artificial RNA" as used herein is intended to refer to an RNA that does not occur naturally. In other words, an artificial RNA may be understood as a non-natural RNA molecule. Such RNA molecules may be non-natural due to its individual sequence (e.g. G/C content modified coding sequence, UTRs) and/or due to other modifications, e.g. structural modifications of modified nucleotides. Artificial RNA may be designed and/or generated by genetic engineering to correspond to a desired artificial sequence of nucleotides. In this context an artificial RNA is a sequence that may not occur naturally, i.e. it differs from the wild type sequence by at least one nucleotide. The term "artificial RNA" is not restricted to mean "one single molecule" but is understood to comprise an ensemble of essentially identical molecules. Accordingly, it may relate to a plurality of essentially identical RNA molecules.

In preferred embodiments, the coding RNA of the composition for use is a modified and/or stabilized RNA.

According to preferred embodiments, the coding RNA of the composition for use may thus be provided as a "stabilized artificial RNA" or "stabilized coding RNA" that is to say an RNA showing improved resistance to in vivo degradation and/or an RNA showing improved stability in vivo (suitably after SCS administration), and/or an RNA showing improved translatability in vivo (suitably after SCS administration). In the following, modifications are described which are suitably to "stabilize" the coding RNA of the composition.

In embodiments, stabilization may be effected by providing a "dried RNA" and/or a "purified RNA" as specified herein.

A purified coding RNA may be a HPLC purified coding RNA, and/or a pharmaceutical grade coding RNA.

Alternatively or in addition to that, stabilization can be effected, e.g., by a modified phosphate backbone of the coding RNA of the composition for use. A backbone modification may be a modification in which phosphates of the backbone of the nucleotides contained in RNA are chemically modified. Nucleotides that may be preferably used comprise e.g. a phosphorothioate-modified phosphate backbone, preferably at least one of the phosphate oxygens contained in the phosphate backbone being replaced by a sulfur atom. Stabilized RNAs may further include, e.g.: non-ionic phosphate analogues, such as, e.g., alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include modifications from the group consisting of methylphosphonates, phosphoramidates and phosphorothioates (e.g. cytidine-5'-O-(1-thiophosphate)).

In the following, suitable modifications are described that are capable of "stabilizing" the coding RNA of the composition for use.

According to embodiments, the coding RNA of the composition for use is a modified coding RNA, wherein the modification refers to chemical modifications comprising backbone modifications as well as sugar modifications or base modifications.

A modified coding RNA may comprise nucleotide analogues/modifications, e.g. backbone modifications, sugar modifications or base modifications. A backbone modification in the context of the invention is a modification, in which phosphates of the backbone of the nucleotides are chemically modified. A sugar modification in the context of the invention is a chemical modification of the sugar of the nucleotides of the RNA.

Furthermore, a base modification in the context of the invention is a chemical modification of the base moiety of the nucleotides of the RNA. In this context, nucleotide analogues or modifications are preferably selected from nucleotide analogues which are applicable for transcription and/or translation.

In particularly preferred embodiments, the nucleotide analogues/modifications which may be incorporated into a modified RNA as described herein are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl-inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl- 1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine, inosine, 1-methylinosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine, 5'-O-(1-thiophosphate)-adenosine, 5'-O-(1-thiophosphate)-cytidine, 5'-O-(1-thiophosphate)-guanosine, 5'-O-(1-thiophosphate)-uridine, 5'-O-(1-thiophosphate)-pseudouridine, 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha-thio-uridine, 4-thio-uridine, 6-aza-uridine, 5-hydroxy-uridine, deoxy-thymidine, 5-methyl-uridine, Pyrrolo-cytidine, inosine, alpha-thio-guanosine, 6-methyl-guanosine, 5-methyl-cytdine, 8-oxo-guanosine, 7-deaza-guanosine, N1-methyladenosine, 2-amino-6-Chloro-purine, N6-methyl-2-amino-purine, Pseudo-iso-cytidine, 6-Chloro-purine, N6-methyladenosine, alpha -thio-adenosine, 8-azido-adenosine, 7-deaza-adenosine.

In some embodiments, at least one chemical modification is selected from pseudouridine, N1-methylpseudouridine, N1-ethylpseudouridine, 2-thiouridine, 4'- thiouridine, 5-methylcytosine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy- pseudouridine, 4-thio-1-methyl-pseudouridine, 4-thio-pseudouridine, 5-aza-uridine, dihydropseudouridine, 5-methoxyuridine and 2'-0-methyl uridine.

In some embodiments, 100% of the uracil in the cds have a chemical modification, preferably a chemical modification is in the 5-position of the uracil.

Particularly preferred in the context of the invention are pseudouridine (ψ), N1-methylpseudouridine (m1ψ), 5-methylcytosine, and 5-methoxyuridine. Accordingly, the coding RNA or the coding sequence may comprise at least one modified nucleotide selected from pseudouridine (ψ), N1- methylpseudouridine (m1ψ), 5-methylcytosine, and 5-methoxyuridine.

In preferred embodiments, the at least one cds of the coding RNA is a codon modified cds, wherein the amino acid sequence encoded by the at least one codon modified cds is preferably not being modified compared to the amino acid sequence encoded by the corresponding wild type cds.

The term "codon modified coding sequence" relates to coding sequences that differ in at least one codon (triplets of nucleotides coding for one amino acid) compared to the corresponding wild type cds. Suitably, a codon modified cds in the context of the invention may show improved resistance to in vivo degradation and/or improved stability in vivo, and/or improved translatability in vivo. Codon modifications in the broadest sense make use of the degeneracy of the genetic code wherein multiple codons encoding the same amino acid may be used interchangeably (cf. **Table 2)** to optimize/modify the cds for in vivo applications as outlined above.

In particularly preferred embodiments, the at least one cds is a codon modified cds, wherein the codon modified cds is selected from C maximized cds, CAI maximized cds, human codon usage adapted cds, G/C content modified cds, and G/C optimized cds, or any combination thereof. Accordingly, the cds of the coding RNA encoding e.g. amino acid sequences according to **SEQ ID NOs: 2-365,436** may be a C maximized cds, a CAI maximized cds, a human codon usage adapted cds, a G/C content modified cds, and a G/C optimized cds, or any combination thereof.

In preferred embodiments, the coding RNA of the composition for use may be modified, wherein the C content of the at least one cds may be increased, preferably maximized, compared to the C content of the corresponding wild type cds (herein referred to as "C maximized coding sequence"). The amino acid sequence encoded by the C maximized cds of the RNA is preferably not modified as compared to the amino acid sequence encoded by the respective wild type nucleic acid cds. The generation of a C maximized nucleic acid sequences may be carried out using a method according to WO2015/062738, the disclosure of WO2015/062738 included herewith by reference. Accordingly, the coding RNA of the composition for use may comprise or consist of at least one cds encoding at least one peptide or protein having an amino acid sequences being at least 70%, 80%, 85%, 86%, 87%, 88%, 89%, 90%, 91%, 92%, 93%, 94%, 95%, 96%, 97%, 98%, or 99% identical to any one of **SEQ ID NOs: 2-365, 436** wherein the cds is a C maximized coding sequence as explained above.

In embodiments, the coding RNA of the composition for use may be modified, wherein the G/C content of the at least one cds may be modified compared to the G/C content of the corresponding wild type cds (herein referred to as "G/C content modified coding sequence"). In this context, the terms "G/C optimization" or "G/C content modification" relate to an RNA that comprises a modified, preferably an increased number of guanosine and/or cytosine nucleotides as compared to the corresponding wild type RNA sequence. Such an increased number may be generated by substitution of codons containing adenosine or thymidine nucleotides by codons containing guanosine or cytosine nucleotides. Advantageously, RNA sequences having an increased G (guanosine)/C (cytosine) content are more stable than sequences having an increased A (adenosine)/U (uracil) content. The amino acid sequence encoded by the G/C content modified cds of the RNA is preferably not modified as compared to the amino acid sequence encoded by the respective wild type sequence. Preferably, the G/C content of the cds of the RNA sequence is increased by at least 10%, 20%, 30%, preferably by at least 40% compared to the G/C content of the cds of the corresponding wild type RNA sequence. Accordingly, the coding RNA of the composition for use may comprise or consist of at least one cds encoding at least one peptide or protein having an amino acid sequences being at least 70%, 80%, 85%, 86%, 87%, 88%, 89%, 90%, 91%, 92%, 93%, 94%, 95%, 96%, 97%, 98%, or 99% identical to any one of **SEQ ID NOs: 2-365, 436** wherein the cds is a "G/C content modified coding sequence" as explained above.

In preferred embodiments, the coding RNA of the composition for use may be modified, wherein the G/C content of the at least one cds may be optimized compared to the G/C content of the corresponding wild type cds (herein referred to as "G/C content optimized coding sequence"). "Optimized" in that context refers to a cds wherein the G/C content is preferably increased to the essentially highest possible G/C content. The amino acid sequence encoded by the G/C content optimized cds of the RNA is preferably not modified as compared to the amino acid sequence encoded by the respective wild type cds. The generation of a G/C content optimized RNA sequence may be carried out using a G/C content optimization method according to WO2002/098443. In this context, the disclosure of WO2002/098443 is included in its full scope in the present invention. Accordingly, the coding RNA of the composition for use may comprise or consist of at least one cds encoding at least one peptide or protein having an amino acid sequences being at least 70%, 80%, 85%, 86%, 87%, 88%, 89%, 90%, 91%, 92%, 93%, 94%, 95%, 96%, 97%, 98%, or 99% identical to any one of **SEQ ID NOs: 2-365, 436** wherein the cds is a "G/C content optimized coding sequence" as explained above.

In embodiments, the coding RNA of the composition for use may be modified, wherein the codons in the at least one cds may be adapted to human codon usage (herein referred to as "human codon usage adapted coding sequence"). Codons encoding the same amino acid occur at different frequencies in a subject, e.g. a human. Accordingly, the cds of the RNA is preferably modified such that the frequency of the codons encoding the same amino acid corresponds to the naturally occurring frequency of that codon according to the human codon usage e.g. as shown in **Table 2.** E.g., in the case of the amino acid Ala, the wild type cds is preferably adapted in a way that the codon "GCC" is used with a frequency of 0.40, the codon "GCT" is used with a frequency of 0.28, the codon "GCA" is used with a frequency of 0.22 and the codon "GCG" is used with a frequency of 0.10 etc. (see **Table 2).** Accordingly, such a procedure (as exemplified for Ala) is applied for each amino acid encoded by the cds of the RNA to obtain sequences adapted to human codon usage. Accordingly, the coding RNA of the composition may comprise or consist of at least one cds encoding at least one peptide or protein having an amino acid sequences being at least 70%, 80%, 85%, 86%, 87%, 88%, 89%, 90%, 91%, 92%, 93%, 94%, 95%, 96%, 97%, 98%, or 99% identical to any one of **SEQ ID NOs: 2-365, 436** wherein the cds is a "human codon usage adapted coding sequence" as explained above.

**Table 2: Human codon usage with respective codon frequencies indicated for each amino acid**

| **Amino acid** | **codon** | **frequency** | | **Amino acid** | **codon** | **frequency** |
|---|---|---|---|---|---|---|
| Ala | GCG | 0.10 | | Pro | CCG | 0.11 |
| Ala | GCA | 0.22 | | Pro | CCA | 0.27 |
| Ala | GCT | 0.28 | | Pro | CCT | 0.29 |
| Ala | GCC* | 0.40 | | Pro | CCC* | 0.33 |
| Cys | TGT | 0.42 | | Gln | CAG* | 0.73 |
| Cys | TGC* | 0.58 | | Gln | CAA | 0.27 |
| Asp | GAT | 0.44 | | Arg | AGG | 0.22 |
| Asp | GAC* | 0.56 | | Arg | AGA* | 0.21 |
| Glu | GAG* | 0.59 | | Arg | CGG | 0.19 |
| Glu | GAA | 0.41 | | Arg | CGA | 0.10 |
| Phe | TTT | 0.43 | | Arg | CGT | 0.09 |
| Phe | TTC* | 0.57 | | Arg | CGC | 0.19 |
| Gly | GGG | 0.23 | | Ser | AGT | 0.14 |
| Gly | GGA | 0.26 | | Ser | AGC* | 0.25 |
| Gly | GGT | 0.18 | | Ser | TCG | 0.06 |
| Gly | GGC* | 0.33 | | Ser | TCA | 0.15 |
| His | CAT | 0.41 | | Ser | TCT | 0.18 |
| His | CAC* | 0.59 | | Ser | TCC | 0.23 |
| Ile | ATA | 0.14 | | Thr | ACG | 0.12 |
| Ile | ATT | 0.35 | | Thr | ACA | 0.27 |
| Ile | ATC* | 0.52 | | Thr | ACT | 0.23 |
| Lys | AAG* | 0.60 | | Thr | ACC* | 0.38 |
| Lys | AAA | 0.40 | | Val | GTG* | 0.48 |
| Leu | TTG | 0.12 | | Val | GTA | 0.10 |
| Leu | TTA | 0.06 | | Val | GTT | 0.17 |
| Leu | CTG* | 0.43 | | Val | GTC | 0.25 |
| Leu | CTA | 007 | | Trp | TGG* | 1 |
| Leu | CTT | 0.12 | | Tyr | TAT | 0.42 |
| Leu | CTC | 0.20 | | Tyr | TAC* | 0.58 |
| Met | ATG* | 1 | | Stop | TGA* | 0.61 |
| Asn | AAT | 0.44 | | Stop | TAG | 0.17 |
| Asn | AAC* | 0.56 | | Stop | TAA | 0.22 |

| | | | | | | |
|---|---|---|---|---|---|---|
| *: most frequent human codon | | | | | | |

In embodiments, the coding RNA of the composition for use may be modified, wherein the codon adaptation index (CAI) may be increased or preferably maximised in the at least one cds (herein referred to as "CAI maximized coding sequence"). Accordingly, it is preferred that all codons of the wild type nucleic acid sequence that are relatively rare in e.g. a human cell are exchanged for a respective codon that is frequent in the e.g. a human cell, wherein the frequent codon encodes the same amino acid as the relatively rare codon. Suitably, the most frequent codons are used for each encoded amino acid (see **Table 2,** most frequent human codons are marked with asterisks). Suitably, the RNA comprises at least one cds, wherein the codon adaptation index (CAI) of the at least one cds is at least 0.5, at least 0.8, at least 0.9 or at least 0.95. Most preferably, the codon adaptation index (CAI) of the at least one cds is 1. E.g., in the case of the amino acid Ala, the wild type cds is adapted in a way that the most frequent human codon "GCC" is always used for said amino acid. Accordingly, such a procedure (as exemplified for Ala) is applied for each amino acid encoded by the cds of the RNA to obtain a CAI maximized cds. Accordingly, the coding RNA of the composition may comprise or consist of at least one cds encoding at least one peptide or protein having an amino acid sequences being at least 70%, 80%, 85%, 86%, 87%, 88%, 89%, 90%, 91%, 92%, 93%, 94%, 95%, 96%, 97%, 98%, or 99% identical to any one of **SEQ ID NOs: 2-365, 436** wherein the cds is a "CAI maximized coding sequence" as explained above.

In certain embodiments, the coding RNA of the composition for use comprises a cds encoding a CRISPR-associated endonuclease as defined herein. In embodiments, the coding RNA of the composition for use comprises at least one cds comprising or consisting of a nucleic acid sequence being at least 70%, 80%, 85%, 86%, 87%, 88%, 89%, 90%, 91%, 92%, 93%, 94%, 95%, 96%, 97%, 98%, or 99% identical to any one of SEQ ID NO: 411; 2540-2553; 11117-11119; 11355-11357; 2554-3457; 1380-1393; 3700-3713; 4860-4873; 6020-6033; 7180-7193; 8340-8353; 11237-11239; 11473-11475; 11591-11593; 11709-11711; 11827-11829; 11945-11947; 1394-2297; 3714-4617; 4874-5777; 6034-6937; 7194-8097; 8354-9257; 412; 3474-3887; 2314-2327; 4634-4647; 5794-5807; 6954-6967; 8114-8127; 413-425; 3490-3503; 3506-3519; 3522-3535; 3538-3551; 3554-3567; 3570-3583; 3586-3599; 3602-3615; 3618-3631; 3634-3647; 3650-3663; 3666-3679; 3682-3695; 9514-9527; 9626-9639; 9738-9751; 9850-9863; 9962-9975, 10074-10087; 10186-10199; 10298-10311; 2330-2343; 2346-2359; 2362-2375; 2378-2391; 2394-2407; 2410-2423; 2426-2439; 2442-2455; 2458-2471; 2474-2487; 2490-2503; 2506-2519; 2522-2535; 9498-9511; 9610-9623; 9722-9735; 9834-9847; 9946-9959; 10058-10071; 10170-10183-10282-10295; 4650-4663; 4666-4679; 4682-4695; 4698-4711; 4714-4727; 4730-4743; 4746-4759; 4762-4775; 4778-4791; 4794-4807; 4810-4823; 4826-4839; 4842-4855; 9530-9543; 9642-9655; 9754-9767; 9866-9879; 9978-9991; 10090-10103; 10202-10215; 10314-10327; 5810-5823; 5826-5839; 5842-5855; 5858-5871; 5874-5887; 5890-5903; 5906-5919; 5922-5935; 5938-5951; 5954-5967; 5970-5983, 5986-5999; 6002-6015; 9546-9559; 9658-9671; 9770-9783; 9882-9895; 9994-10007; 10106-10119; 10218-10231; 10330-10343; 6970-6983; 6986-6999; 7002-7015; 7018-7031; 7034-7047; 7050-7063; 7066 -7079; 7082-7095; 7098-7111; 7114-7127; 7130-7143; 7146-7159; 7162-7175; 9562-9575; 9674-9687; 9786-9799; 9898-9911; 10010-10023; 10122-10135; 10234-10247; 10346-10359; 8130-8143; 8146-8159; 8162-8175; 8178-8191; 8194-8207; 8210-8223; 8226-8239; 8242-8255; 8258-8271; 8274-8287; 8290-8302; 8306-8319; 8322-8335; 9578-9591; 9690-9703; 9802-9815; 9914-9927; 10026-10039; 10138-10151; 10250 -10263; 10362-10375; 9290-9303; 9306-9319; 9322-9335; 9338-9351; 9354-9367; 9370-9383; 9386-9399; 9402-9415; 9418-9431; 9434-9447; 9450-9463; 9466-9479; 9482-9495; 9594 -9607; 9706-9719; 9818-9831; 9930-9943; 10042-10055; 10154-10167; 10266-10279; 10378-10391 of published PCT patent application WO 2018/172556, said sequences herewith incorporated by reference.

In other embodiments, the coding RNA of the composition for use comprises at least one cds comprising or consisting of a nucleic acid sequence being at least 70%, 80%, 85%, 86%, 87%, 88%, 89%, 90%, 91%, 92%, 93%, 94%, 95%, 96%, 97%, 98%, or 99% identical to any one of SEQ ID NOs: 10552; 3458-3459; 3460-3473; 2298-2299; 4618-4619; 5778-5779; 6938-6939; 8098-8099; 9258-9259 ; 2300-2313; 4620-4633; 5780-5793; 6940-6953; 8100-8113; 9260-9273; 3488-3489; 10396; 2328-2329; 10395; 4648-4649; 10397; 5808-5809; 10398; 6968-6969; 10399; 8128-8129; 10400; 9274-9287; 3504-3505; 3520-3521; 3536-3537; 3552-3553; 3568-3669; 3584-3585; 3600-3601; 3616-3617; 3632-3633; 3648-3649; 3664-3665; 3680-3681; 3696-3697; 9528-9529; 9640-9641; 9752-9753; 9864-9865; 9976-9977; 10088-10089; 10200-10201; 10312-10313; 10403; 10410; 10417; 10424; 10431; 10438; 10445; 10452; 10459; 10466; 10473; 10480; 10487; 10494; 10501; 10508; 10515; 10522; 10529; 10536; 10543; 2344-2345; 2360-2361; 2376-2377; 2392-2393; 2408-2409; 2424-2425; 2440-2441; 2456-2457; 2472-2473; 2489-2490; 2504-2505; 2520-2521; 2536-2537; 9512-9513; 9624-9625; 9736-9737; 9848-9849; 9960-9961; 10072-10073; 10184-10185; 10296-10297; 10402; 10409; 10416; 10423; 10430; 10437; 10444; 10451; 10458; 10465; 10472; 10479; 10486; 10493; 10500; 10507; 10514; 10521; 10528; 10535; 10542; 4664-4665; 4680-4681; 4696-4697; 4712-4713; 4728-4729; 4744-4745; 4760^761; 4776-4777; 4792-4793; 4808-4809; 4824-4825; 4840-4841; 4856-4857; 9544-9545; 9656-9657; 9768-9769; 9880-9881; 9992-9993; 10104-10105; 10216-10217; 10328-10329; 10404; 10411; 10418; 10425; 10432; 10439; 10446; 10453; 10460; 10467; 10474; 10481; 10488; 10495; 10502; 10509; 10516; 10523; 10530; 10537; 10544; 5824-5825; 5840-5841; 5856-5857; 5872-5873; 5888-5889; 5904-5905; 5920-5921; 5936-5937; 5952-5953; 5968-5969; 5984-5985; 6000-6001; 6016-6017; 9560-9561; 9672-9673; 9784-9785; 9896-9897; 10008-10009; 10120-10121; 10232-10233; 10344-10345; 10405; 10412; 10419; 10426; 10433; 10440; 10447; 10454; 10461; 10468; 10475; 10482; 10489; 10496; 10503; 10510; 10517; 10524; 10531; 10538; 10545; 7033; 7048-7049; 7064-7065; 7080-7081; 7096-7097; 7112-7113; 7128-7129; 7144-7145; 7160-7161; 7176-7177; 9576-9577; 9688-9689; 9800-9801; 9912-9913; 10024-10025; 10136-10137; 10248-10249; 10360-10361; 10406; 10413; 10420; 10427; 10434; 10441; 10448; 10455; 10462; 10469; 10476; 10483; 10490; 10497; 10504; 10511; 10518; 10525; 10532; 10539; 10546; 8144-8145; 8160-8160; 8176-8177; 8192-8193; 8208-8209; 8224-8225; 8240-8241; 8256-8257; 8272-8273; 8288 -8289; 8304-8305; 8320-8321; 8336-8337; 9592-9593; 9704-9705; 9816-9817; 9928-9929; 10040-10041; 10152-10153; 10264-10265; 10376-10377; 10407; 10414; 10421; 10428; 10435; 10442; 10449; 10456; 10463; 10470; 10477; 10484; 10491; 10498; 10505; 10512; 10519; 10526; 10533; 10540; 10547; 9288-9289; 10401; 10553; 10582-10583;10579-10580; 10585-10586; 10588-10589; 10591-10592; 10594-10595; 10597-10598; 10554-10574; 10601; 10602; 10615; 10616; 10629; 10630; 10643; 10644; 10657; 10658; 10671; 10672; 10685; 10686; 10699; 10700; 10713; 10714; 10727; 10728; 10741; 10742; 10755; 10756; 10769; 10770; 10783; 10784; 10797; 10798; 10811; 10812; 10825; 10826; 10839; 10840; 10853; 10854; 10867; 10868; 10881; 10882; 10603; 10604; 10617; 10618; 10631; 10632; 10645; 10646; 10659; 10660; 10673; 10674; 10687; 10688; 10701; 10702; 10715; 10716; 10729; 10730; 10743; 10744; 10757; 10758; 10771; 10772; 10785; 10786; 10799; 10800; 10813; 10814; 10827; 10828; 10841; 10842; 10855; 10856; 10869; 10870; 10883; 10884; 10605; 10606; 10619; 10620; 10633; 10634; 10647; 10648; 10661; 10662; 10675; 10676; 10689; 10690; 10703; 10704; 10717; 10718; 10731; 10732; 10745; 10746; 10759; 10760; 10773; 10774; 10787; 10788; 10801; 10802; 10815; 10816; 10829; 10830; 10843; 10844; 10857; 10858; 10871; 10872; 10885; 10886; 10607; 10608; 10621; 10622; 10635; 10636; 10649; 10650; 10663; 10664; 10677; 10678; 10691; 10692; 10705; 10706; 10719; 10720; 10733; 10734; 10747; 10748; 10761; 10762; 10775; 10776; 10789; 10790; 10803; 10804; 10817; 10818; 10831; 10832; 10845; 10846; 10859; 10860; 10873; 10874; 10887; 10888; 10609; 10610; 10623; 10624; 10637; 10638; 10651; 10652; 10665; 10666; 10679; 10680; 10693; 10694; 10707; 10708; 10721; 10722; 10735; 10736; 10749; 10750; 10763; 10764; 10777; 10778; 10791; 10792; 10805; 10806; 10819; 10820; 10833; 10834; 10847; 10848; 10861; 10862; 10875; 10876; 10889; 10890; 10611; 10612; 10625; 10626; 10639; 10640; 10653; 10654; 10667; 10668; 10681; 10682; 10695; 10696; 10709; 10710; 10723; 10724; 10737; 10738; 10751; 10752; 10765; 10766; 10779; 10780; 10793; 10794; 10807; 10808; 10821; 10822; 10835; 10836; 10849; 10850; 10863; 10864; 10877; 10878; 10891; 10892; 9304-9305; 9320-9321; 9336-9337; 9352-9353; 9368-9369; 9384-9385; 9400-9401; 9416-9417; 9432-9433; 9448-9449; 9464-9465; 9480-9481; 9496-9497; 9608-9609; 9720-9721; 9832-9833; 9944-9945; 10056-10057; 10168-10169; 10280-10281; 10392-10393; 10408; 10415; 10422; 10429; 10436; 10443; 10450; 10457; 10464; 10471; 10478; 10485; 10492; 10499; 10506; 10513; 10520; 10527; 10534; 10541; 10548 of published PCT patent application WO 2018/172556, said sequences herewith incorporated by reference.

In embodiments, the coding RNA of the composition for use may be monocistronic, bicistronic, or multicistronic.

The term "monocistronic" will be recognized and understood by the person of ordinary skill in the art, and is e.g. intended to refer to an RNA that comprises only one coding sequences. The terms "bicistronic", or "multicistronic" as used herein will be recognized and understood by the person of ordinary skill in the art, and are e.g. intended to refer to an RNA that may have two (bicistronic) or more (multicistronic) coding sequences.

In preferred embodiments, the coding RNA of the composition for use is monocistronic.

In embodiments, the coding RNA of the composition for use is monocistronic and the cds of said RNA encodes at least two different proteins as defined above (e.g. derived from **List 1** or **Table 1).** Accordingly, said cds may e.g. encode at least two, three, four, five, six, seven, eight and more therapeutic proteins, linked with or without an amino acid linker sequence, wherein said linker sequence can comprise rigid linkers, flexible linkers, cleavable linkers, or a combination thereof. Such constructs are herein referred to as "multi-protein-constructs".

In embodiments, the coding RNA of the composition for use may be bicistronic or multicistronic and comprises at least two coding sequences, wherein the at least two coding sequences encode two or more proteins as defined above (e.g. derived from **List 1** or **Table 1).** Accordingly, the coding sequences in a bicistronic or multicistronic RNA suitably encode distinct proteins or peptides as defined herein. Preferably, the coding sequences in said bicistronic or multicistronic constructs may be separated by at least one IRES (internal ribosomal entry site) sequence. In that context, suitable IRES sequences may be selected from the list of nucleic acid sequences according to SEQ ID NOs: 1566-1662 of the patent application WO2017/081082, or fragments or variants of these sequences. In this context, the disclosure of WO2017/081082 relating to IRES sequences is herewith incorporated by reference.

Preferably, the coding RNA of the composition for use may comprise about 50 to about 20000 nucleotides, or about 500 to about 10000 nucleotides, or about 1000 to about 10000 nucleotides, or preferably about 1000 to about 5000 nucleotides, or even more preferably about 1000 to about 3000 nucleotides.

In preferred embodiments, the coding RNA of the composition for use may be an mRNA, a self-replicating RNA, a circular RNA, a viral RNA, or a replicon RNA.

In embodiments, the coding RNA of the composition for use is a circular RNA. As used herein, "circular RNA" or "circRNAs" have to be understood as a circular polynucleotide constructs that encode at least one antigenic peptide or protein as defined herein. Accordingly, in preferred embodiments, said circRNA comprises at least one cds encoding at least one peptide or protein as defined herein. The production of circRNA can be performed using various methods provided in the art. Accordingly, methods for producing circular RNA as provided in US6210931, US5773244, WO1992/001813, WO2015/034925 and WO2016/011222 are incorporated herewith by reference.

In embodiments, the coding RNA of the composition for use is a replicon RNA. The term "replicon RNA" will be recognized and understood by the person of ordinary skill in the art, and is e.g. intended to be an optimized self-replicating RNA. Such constructs may include replicase elements derived from e.g. alphaviruses (e.g. SFV, SIN, VEE, or RRV) and the substitution of the structural virus proteins with the nucleic acid of interest. Alternatively, the replicase may be provided on an independent coding RNA construct. Downstream of the replicase may be a sub-genomic promoter that controls replication of the replicon RNA.

In preferred embodiments, the coding RNA of the composition for use is an mRNA.

The terms "RNA" and "mRNA" will be recognized and understood by the person of ordinary skill in the art, and are e.g. intended to be a ribonucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. The mRNA (messenger RNA) usually provides the nucleotide sequence that may be translated into an amino-acid sequence of a particular peptide or protein after SCS administration.

Suitably, the coding RNA of the composition for use may be modified by the addition of a 5'-cap structure, which preferably stabilizes the RNA and/or enhances expression of the encoded peptide or protein.

A 5'-cap structure is of particular importance in embodiments where the coding RNA is linear, e.g. a linear mRNA or a linear replicon RNA.

In preferred embodiments, the coding RNA, in particular the mRNA of the composition for use comprises a 5'-cap structure, preferably m7G (m7G(5')ppp(5')G), cap0, cap1, cap2, a modified cap0 or a modified cap1 structure.

The term "5'-cap structure" as used herein will be recognized and understood by the person of ordinary skill in the art, and is e.g. intended to refer to a 5' modified nucleotide, particularly a guanine nucleotide, positioned at the 5'-end of an RNA molecule, e.g. an mRNA molecule. Preferably, the 5'-cap structure is connected via a 5'-5'-triphosphate linkage to the RNA.

5'-cap structures which may be suitable in the context of the present invention are cap0 (methylation of the first nucleobase, e.g. m7GpppN), cap1 (additional methylation of the ribose of the adjacent nucleotide of m7GpppN), cap2 (additional methylation of the ribose of the 2nd nucleotide downstream of the m7GpppN), cap3 (additional methylation of the ribose of the 3rd nucleotide downstream of the m7GpppN), cap4 (additional methylation of the ribose of the 4th nucleotide downstream of the m7GpppN), ARCA (anti-reverse cap analogue), modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

A 5'-cap (cap0 or cap1) structure may be formed in chemical RNA synthesis or RNA in vitro transcription (cotranscriptional capping) using cap analogues.

The term "cap analogue" as used herein will be recognized and understood by the person of ordinary skill in the art, and is e.g. intended to refer to a non-polymerizable di-nucleotide or tri-nucleotide that has cap functionality in that it facilitates translation or localization, and/or prevents degradation of a nucleic acid molecule, particularly of an RNA molecule, when incorporated at the 5'-end of the nucleic acid molecule. Non-polymerizable means that the cap analogue will be incorporated only at the 5'-terminus because it does not have a 5' triphosphate and therefore cannot be extended in the 3'-direction by a template-dependent polymerase, particularly, by template-dependent RNA polymerase. Examples of cap analogues include, but are not limited to, a chemical structure selected from the group consisting of m7GpppG, m7GpppA, m7GpppC; unmethylated cap analogues (e.g. GpppG); dimethylated cap analogue (e.g. m2,7GpppG), trimethylated cap analogue (e.g. m2,2,7GpppG), dimethylated symmetrical cap analogues (e.g. m7Gpppm7G), or anti reverse cap analogues (e.g. ARCA; m7,2'OmeGpppG, m7,2'dGpppG, m7,3'OmeGpppG, m7,3'dGpppG and their tetraphosphate derivatives). Further cap analogues have been described previously (WO2008/016473, WO2008/157688, WO2009/149253, WO2011/015347, and WO2013/059475). Further suitable cap analogues in that context are described in WO2017/066793, WO2017/066781, WO2017/066791, WO2017/066789, WO2017/053297, WO2017/066782, WO2018075827 and WO2017/066797 wherein the disclosures referring to cap analogues are incorporated herewith by reference.

In embodiments, a modified cap1 structure is generated using tri-nucleotide cap analogue as disclosed in WO2017/053297, WO2017/066793, WO2017/066781, WO2017/066791, WO2017/066789, WO2017/066782, WO2018075827 and WO2017/066797. In particular, any cap structures derivable from the structure disclosed in claim 1-5 of WO2017/053297 may be suitably used to co-transcriptionally generate a modified cap1 structure. Further, any cap structures derivable from the structure defined in claim 1 or claim 21 of WO2018075827 may be suitably used to co-transcriptionally generate a modified cap1 structure.

In preferred embodiments, the 5'-cap structure may suitably be added co-transcriptionally using tri-nucleotide cap analogue as defined herein in an RNA *in vitro* transcription reaction as defined herein.

Preferred cap-analogues are the di-nucleotide cap analogues m7G(5')ppp(5')G (m7G) or 3'-O-Me-m7G(5')ppp(5')G to co-transcriptionally generate cap0 structures. Further preferred cap-analogues are the tri-nucleotide cap analogues m7G(5')ppp(5')(2'OMeA)pG or m7G(5')ppp(5')(2'OMeG)pG to co-transcriptionally generate cap1 structures.

In other embodiments, the 5'-cap structure is formed via enzymatic capping using capping enzymes (e.g. vaccinia virus capping enzymes and/or cap-dependent 2'-O methyltransferases) to generate cap0 or cap1 or cap2 structures. In embodiments, the 5'-cap structure (cap0 or cap1) may be added using immobilized capping enzymes and/or cap-dependent 2'-O methyltransferases using methods and means disclosed in WO2016/193226.

In a particularly preferred embodiment, the coding RNA of the composition for use comprises a cap1 structure, wherein said cap1 structure may be formed enzymatically or co-transcriptionally (e.g. using m7G(5')ppp(5')(2'OMeA), or m7G(5')ppp(5')(2'OMeG) analogues).

In preferred embodiments, the coding RNA of the composition for use comprises an m7G(5')ppp(5')(2'OMeA) cap structure. In such embodiments, the coding RNA comprises a 5' terminal m7Gcap, and an additional methylation of the ribose of the adjacent nucleotide of m7GpppN, in that case, a 2'O methylated adenosine.

In other preferred embodiments, the coding RNA of the composition for use comprises an m7G(5')ppp(5')(2'OMeG) cap structure. In such embodiments, the coding RNA comprises a 5' terminal m7Gcap, and an additional methylation of the ribose of the adjacent nucleotide, in that case, a 2'O methylated guanosine.

Accordingly, whenever reference is made to suitable RNA or mRNA sequences in the context of the invention, the first nucleotide of said RNA or mRNA sequence, that is, the nucleotide downstream of the m7G(5')ppp structure, may be a 2'O methylated guanosine or a 2'O methylated adenosine.

In embodiments, the A/U content in the environment of the ribosome binding site of the coding RNA of the composition for use may be increased compared to the A/U content in the environment of the ribosome binding site of its respective wild type nucleic acid. This modification (an increased A/U content around the ribosome binding site) increases the efficiency of ribosome binding to the nucleic acid, preferably the RNA. An effective binding of the ribosomes to the ribosome binding site in turn has the effect of an efficient translation of the RNA.

Accordingly, in a particularly preferred embodiment, the coding RNA of the composition for use comprises a ribosome binding site, also referred to as "Kozak sequence" identical to or at least 80%, 85%, 90%, 95% identical to any one of the sequences **SEQ ID NOs: 406, 407,** or fragments or variants thereof.

In preferred embodiments, the coding RNA of the composition for use comprises at least one poly(N) sequence, e.g. at least one poly(A) sequence, at least one poly(U) sequence, at least one poly(C) sequence, or combinations thereof.

In preferred embodiments, the coding RNA of the composition for use comprises at least one poly(A) sequence.

The terms "poly(A) sequence", "poly(A) tail" or "3'-poly(A) tail" as used herein will be recognized and understood by the person of ordinary skill in the art, and are e.g. intended to be a sequence of adenosine nucleotides, typically located at the 3'-end of a coding RNA, of up to about 1000 adenosine nucleotides. Said poly(A) sequence is essentially homopolymeric, e.g. a poly(A) sequence of e.g. 100 adenosine nucleotides has essentially the length of 100 nucleotides. In other embodiments, the poly(A) sequence may be interrupted by at least one nucleotide different from an adenosine nucleotide.

The poly(A) sequence, suitable located downstream of a 3' UTR as defined herein, may comprise about 10 to about 500 adenosine nucleotides, about 10 to about 200 adenosine nucleotides, about 40 to about 200 adenosine nucleotides, or about 40 to about 150 adenosine nucleotides. Suitably, the length of the poly(A) sequence may be at least about or even more than about 10, 50, 64, 75, 100, 200, 300, 400, or 500 adenosine nucleotides. Suitably, the poly(A) sequence of the coding RNA may be long enough to bind at least 2, 3, 4, 5 or more monomers of PolyA Binding Proteins. In preferred embodiments, the poly(A) sequence comprises about 50 to about 250 adenosines. In a particularly preferred embodiment, the poly(A) sequence comprises about 64 adenosine nucleotides.

Preferably, the poly(A) sequence of the coding RNA is obtained from a DNA template during RNA in vitro transcription. In other embodiments, the poly(A) sequence is obtained in vitro by common methods of chemical synthesis without being necessarily transcribed from a DNA template. In other embodiments, poly(A) sequences are generated by enzymatic polyadenylation of the RNA (after RNA *in vitro* transcription) using commercially available polyadenylation kits and corresponding protocols known in the art, or alternatively, by using immobilized poly(A)polymerases e.g. using a methods and means as described in WO2016/174271.

In embodiments, the coding RNA may comprise a poly(A) sequence derived from a template DNA and may comprise at least one additional poly(A) sequence generated by enzymatic polyadenylation, e.g. as described in WO2016/091391.

In embodiments where enzymatic polyadenylation of RNA is used, it has to be understood that RNA or mRNA sequences as described herein, may additionally comprise about 30 to about 500 adenosine nucleotides located at the 3' terminus / the 3'-end.

In preferred embodiments, the coding RNA may comprise at least one poly(C) sequence.

The term "poly(C) sequence" as used herein will be recognized and understood by the person of ordinary skill in the art, and are e.g. intended to be a sequence of cytosine nucleotides, typically located at the 3'-end of an RNA, of up to about 200 cytosine nucleotides.

In preferred embodiments, the poly(C) sequence, suitable located at the 3' terminus downstream of the 3' UTR as defined herein, comprises about 10 to about 200 cytosine nucleotides, about 10 to about 100 cytosine nucleotides, about 20 to about 70 cytosine nucleotides, about 20 to about 60 cytosine nucleotides, or about 10 to about 40 cytosine nucleotides. In a particularly preferred embodiment, the poly(C) sequence comprises about 30 cytosine nucleotides.

Preferably, the poly(C) sequence in the RNA sequence of the present invention is derived from a DNA template by RNA *in vitro* transcription. In other embodiments, the poly(C) sequence is obtained in vitro by common methods of chemical synthesis, or enzymatically, without being necessarily transcribed from a DNA template.

In preferred embodiments, the coding RNA of the composition for use comprises at least one histone stem-loop.

The term "histone stem-loop" (abbreviated as "hsl") as used herein will be recognized and understood by the person of ordinary skill in the art, and is e.g. intended to refer to nucleic acid sequences predominantly found in histone mRNAs.

Histone stem-loop sequences/structures may suitably be selected from histone stem-loop sequences as disclosed in WO2012/019780, the disclosure relating to histone stem-loop sequences/histone stem-loop structures incorporated herewith by reference. A histone stem-loop sequence that may be used within the present invention may preferably be derived from formulae (I) or (II) of WO2012/019780. According to a further preferred embodiment the coding RNA may comprise at least one histone stem-loop sequence derived from at least one of the specific formulae (Ia) or (Ila) of the patent application WO2012/019780.

In particularly preferred embodiment, the coding RNA of the composition for use comprises at least one histone stem-loop sequence, wherein said histone stem-loop sequence comprises a nucleic acid sequence identical or at least 70%, 80%, 90%, 91%, 92%, 93%, 94%, 95%, 96%, 97%, 98%, or 99% identical to **SEQ ID NOs: 404** or **405,** or fragments or variants thereof.

In embodiments, the coding RNA of the composition for use comprises a 3'-terminal sequence element. Said 3'-terminal sequence element comprises a poly(A)sequence and a histone-stem-loop sequence, wherein said sequence element is located at the 3' terminus of the RNA of the invention.

Accordingly, the coding RNA of the composition for use may comprise a 3'-terminal sequence element comprising or consisting of a nucleic acid sequence being identical or at least 70%, 80%, 90%, 91%, 92%, 93%, 94%, 95%, 96%, 97%, 98%, or 99% identical to **SEQ ID NOs: 410-431,** or a fragment or variant thereof.

In various embodiments, the coding RNA of the composition for use may comprise a 5'-terminal sequence element according to **SEQ ID NOs: 408, 409,** or a fragment or variant thereof. Such a 5'-terminal sequence element comprises e.g. a binding site for T7 RNA polymerase. Further, the first nucleotide of said 5'-terminal start sequence may preferably comprise a 2'O methylation, e.g. 2'O methylated guanosine or a 2'O methylated adenosine.

The coding RNA of the composition for use may be composed of a protein-coding region ("coding sequence" or "cds"), and 5'- UTR and/or 3'- UTR. UTRs may harbor regulatory sequence elements or motifs that determine RNA turnover, stability, and/or localization. Moreover, UTRs may harbor sequence elements or motifs that enhance translation. In medical application of RNA, translation of the RNA cds into at least one peptide or protein is of paramount importance to therapeutic efficacy. Certain combinations of 3'-UTRs and/or 5'-UTRs may enhance the expression of operably linked coding sequences encoding peptides or proteins as defined above. Coding RNA harboring said UTR combinations advantageously enable rapid and transient expression of encoded peptides or proteins after administration to a subject, preferably after SCS administration.

**Table A: The sequence information is introduced here since the nucleotide sequences do not meet the minimum length requirements for inclusion into the ST.26 XML sequence listing (10 or more specifically defined nucleotides / 4 or more specifically defined amino acids, please see §8 of the WIPO STANDARD ST.26).**

| **SEQ ID NO:** | **ST.25 - <212> Molecule Type** | **ST.25 - <223> Other Information** | **Sequence** |
|---|---|---|---|
| 408 | mRNA 5'-end | gggaga | gggaga |
| 409 | mRNA 5'-end | aggaga | aggaga |

Accordingly, the coding RNA of the composition for use may comprise certain combinations of 3'-UTRs and/or 5'-UTRs, resulting in translation of the protein (e.g. therapeutic protein, CRISPR-associated endonuclease, as defined above), and hence, in expression of the protein in therapeutically relevant cells or tissues in the eye (e.g., retinal cells).

Suitably, the coding RNA of the composition for use comprises at least one heterologous 5'-UTR and/or at least one heterologous 3'-UTR. Said heterologous 5'-UTRs or 3'-UTRs may be derived from naturally occurring genes or may be synthetically engineered. In preferred embodiments, the coding RNA comprises at least one cds operably linked to at least one (heterologous) 3'-UTR and/or at least one (heterologous) 5'-UTR.

In preferred embodiments, the coding RNA of the composition for use comprises at least one heterologous 3'-UTR.

The term "3'-untranslated region" or "3'-UTR" or "3'-UTR element" will be recognized and understood by the person of ordinary skill in the art, and are e.g. intended to refer to a part of the coding RNA, located 3' (i.e. downstream) of a cds, which is not translated into protein. A 3'-UTR may be part of an RNA, e.g. an mRNA, located between a cds and a terminal poly(A) sequence. A 3'-UTR may comprise elements for controlling gene expression, also called regulatory elements. Such regulatory elements may be, e.g., ribosomal binding sites, miRNA binding sites etc.

Preferably, the coding RNA of the composition for use comprises a 3'-UTR, which may be derivable from a gene that relates to an RNA with enhanced half-life (i.e. that provides a stable RNA).

In some embodiments, a 3'-UTR comprises one or more of a polyadenylation signal, a binding site for proteins that affect an RNA stability of location in a cell, or one or more miRNA or binding sites for miRNAs.

MicroRNAs (or miRNA) are 19-25 nucleotide long noncoding RNAs that bind to the 3'-UTR of nucleic acid molecules and down-regulate gene expression either by reducing nucleic acid molecule stability or by inhibiting translation. E.g., microRNAs are known to regulate RNA, and thereby protein expression, e.g. in liver (miR-122), heart (miR-ld, miR-149), endothelial cells (miR-17-92, miR-126), adipose tissue (let-7, miR-30c), kidney (miR-192, miR-194, miR-204), myeloid cells (miR-142-3p, miR-142-5p, miR-16, miR-21, miR-223, miR-24, miR-27), muscle (miR-133, miR-206, miR-208), and lung epithelial cells (let-7, miR-133, miR-126). The RNA of the invention may comprise one or more microRNA target sequences, microRNA sequences, or microRNA seeds. Such sequences may e.g. correspond to any known microRNA such as those taught in US Publications US2005/0261218 and US2005/0059005, the contents of which are incorporated herein by reference.

Accordingly, miRNA, or binding sites for miRNAs as defined above may be removed from the 3'-UTR or introduced into the 3'-UTR in order to tailor the expression of the RNA expression to desired cell types or tissues, preferably to desired cell types or tissues in the eye.

In preferred embodiments, the coding RNA of the composition for use comprises at least one heterologous 3'-UTR, wherein the at least one heterologous 3'-UTR comprises a nucleic acid sequence derived from a 3'-UTR of a gene selected from PSMB3, ALB7, alpha-globin (referred to as "muag"), CASP1, COX6B1, GNAS, NDUFA1 and RPS9, or from a homolog, a fragment or variant of any one of these genes.

In embodiments, the coding RNA of the composition for use may comprise a 3'-UTR derived from a ALB7 gene, wherein said 3'-UTR derived from an ALB7 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 400** or **401** or a fragment or a variant thereof. In embodiments, the coding RNA of the composition for use may comprise a 3'-UTR derived from a alpha-globin gene, wherein said 3'-UTR derived from a alpha-globin gene ("muag") comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 402** or **403** or a fragment or a variant thereof. In embodiments, the coding RNA of the composition for use may comprise a 3'-UTR derived from a PSMB3 gene, wherein said 3'-UTR derived from a PSMB3 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 388** or **389** or a fragment or a variant thereof. In embodiments, the coding RNA of the composition for use may comprise a 3'-UTR derived from a CASP1 gene, wherein said 3'-UTR derived from a CASP1 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 390** or **391** or a fragment or a variant thereof. In embodiments, the coding RNA of the composition for use may comprise a 3'-UTR derived from a COX6B1 gene, wherein said 3'-UTR derived from a COX6B1 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 392** or **393** or a fragment or a variant thereof. In embodiments, the coding RNA of the composition for use may comprise a 3'-UTR derived from a GNAS gene, wherein said 3'-UTR derived from a GNAS gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 394** or **395** or a fragment or a variant thereof. In embodiments, the coding RNA of the composition for use may comprise a 3'-UTR derived from a NDUFA1 gene, wherein said 3'-UTR derived from a NDUFA1 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 396** or **397** or a fragment or a variant thereof. In embodiments, the coding RNA of the composition for use may comprise a 3'-UTR derived from a RPS9 gene, wherein said 3'-UTR derived from a RPS9 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 398** or **399** or a fragment or a variant thereof.

Accordingly, the coding RNA of the composition for use may suitably comprise at least one 3'-UTR comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 388-403** or a fragment or a variant thereof.

In other embodiments, the coding RNA of the composition for use comprises a 3'-UTR as described in WO2016/107877, the disclosure of WO2016/107877 relating to 3'-UTR sequences herewith incorporated by reference. Suitable 3'-UTRs are SEQ ID NOs: 1 to 24 and SEQ ID NOs: 49 to 318 ofWO2016/107877, or fragments or variants of these sequences. Accordingly, the 3'-UTRs of the RNA may comprise or consist of a corresponding RNA sequence of the nucleic acid sequence according SEQ ID NOs: 1 to 24 and SEQ ID NOs: 49 to 318 of WO2016/107877. In other embodiments, the coding RNA comprises a 3'-UTR as described in WO2017/036580, the disclosure of WO2017/036580 relating to 3'-UTR sequences herewith incorporated by reference. Suitable 3'-UTRs are SEQ ID NOs: 152 to 204 of WO2017/036580, or fragments or variants of these sequences. Accordingly, the 3'-UTR of the RNA may comprise or consist of a corresponding RNA sequence of the nucleic acid sequence according SEQ ID NOs: 152 to 204 of WO2017/036580. In other embodiments, the coding RNA comprises a 3'-UTR as described in WO2016022914, the disclosure of WO2016022914 relating to 3'-UTR sequences herewith incorporated by reference. Particularly preferred 3'-UTRs are nucleic acid sequences according to SEQ ID NOs: 20 to 36 of WO2016022914, or fragments or variants of these sequences. In this context, it is particularly preferred that the 3'-UTR of the RNA comprises or consists of a corresponding RNA sequence of the nucleic acid sequence according to SEQ ID NOs: 20 to 36 of WO2016022914.

In preferred embodiments, the coding RNA of the composition for use comprises at least one heterologous 5'-UTR.

The terms "5'-untranslated region" or "5'-UTR" or "5'-UTR element" will be recognized and understood by the person of ordinary skill in the art, and are e.g. intended to refer to a part the RNA, located 5' (i.e. "upstream") of a cds, which is not translated into protein. A 5'-UTR may be part of an RNA located 5' of the cds. Typically, a 5'-UTR starts with the transcriptional start site and ends before the start codon of the cds. A 5'-UTR may comprise elements for controlling gene expression, called regulatory elements. Such regulatory elements may be, e.g., ribosomal binding sites, miRNA binding sites etc. The 5'-UTR may be post-transcriptionally modified, e.g. by enzymatic or post-transcriptional addition of a 5'-cap structure (see above).

Preferably, the coding RNA of the composition for use comprises a 5'-UTR, which may be derivable from a gene that relates to an RNA with enhanced half-life (i.e. that provides a stable RNA).

In some embodiments, a 5'-UTR comprises one or more of a binding site for proteins that affect an RNA stability of location in a cell, or one or more miRNA or binding sites for miRNAs (as defined above).

Accordingly, miRNA or binding sites for miRNAs as defined above may be removed from the 5'-UTR or introduced into the 5'-UTR in order to tailor the expression of the RNA expression to desired cell types or tissues, preferably in desired cell types or tissues in the eye.

In preferred embodiments, the coding RNA of the composition for use comprises at least one heterologous 5'-UTR, wherein the at least one heterologous 5'-UTR comprises a nucleic acid sequence derived from a 5'-UTR of gene selected from HSD17B4, RPL32, ASAH1, ATP5A1, MP68, NDUFA4, NOSIP, RPL31, SLC7A3, TUBB4B, and UBQLN2, or from a homolog, a fragment or variant of any one of these genes.

In embodiments, the coding RNA of the composition for use may comprise a 5'-UTR derived from a RPL32 gene, wherein said 5'-UTR derived from a RPL32 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 386** or **387** or a fragment or a variant thereof. In embodiments, the coding RNA of the composition for use may comprise a 5'-UTR derived from a HSD17B4 gene, wherein said 5'-UTR derived from a HSD17B4 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 366** or **367** or a fragment or a variant thereof. In embodiments, the coding RNA of the composition for use may comprise a 5'-UTR derived from a ASAH1 gene, wherein said 5'-UTR derived from a ASAH1 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 368** or **369** or a fragment or a variant thereof. In embodiments, the coding RNA of the composition for use may comprise a 5'-UTR derived from a ATP5A1 gene, wherein said 5'-UTR derived from a ATP5A1 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 370** or **371** or a fragment or a variant thereof. In embodiments, the coding RNA of the composition for use may comprise a 5'-UTR derived from a MP68 gene, wherein said 5'-UTR derived from a MP68 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 372** or **373** or a fragment or a variant thereof. In embodiments, the coding RNA of the composition for use may comprise a 5'-UTR derived from a NDUFA4 gene, wherein said 5'-UTR derived from a NDUFA4 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 374** or **375** or a fragment or a variant thereof. In embodiments, the coding RNA of the composition for use may comprise a 5'-UTR derived from a NOSIP gene, wherein said 5'-UTR derived from a NOSIP gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 376** or **377** or a fragment or a variant thereof. In embodiments, the RNA may comprise a 5'-UTR derived from a RPL31 gene, wherein said 5'-UTR derived from a RPL31 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 378** or **379** or a fragment or a variant thereof. In embodiments, the coding RNA of the composition for use may comprise a 5'-UTR derived from a SLC7A3 gene, wherein said 5'-UTR derived from a SLC7A3 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 380** or **381** or a fragment or a variant thereof. In embodiments, the coding RNA of the composition for use may comprise a 5'-UTR derived from a TUBB4B gene, wherein said 5'-UTR derived from a TUBB4B gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 382** or **383** or a fragment or a variant thereof. In embodiments, the coding RNA of the composition for use may comprise a 5'-UTR derived from a UBQLN2 gene, wherein said 5'-UTR derived from a UBQLN2 gene comprises or consists of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NO: 384** or **385** or a fragment or a variant thereof.

Accordingly, the coding RNA of the composition for use may suitably comprise at least one 5'-UTR comprising or consisting of a nucleic acid sequence being identical or at least 70%, 80%, 85%, 86%, 87%, 88%, 89%, 90%, 91%, 92%, 93%, 94%, 95%, 96%, 97%, 98%, or 99% identical to **SEQ ID NOs: 366-403** or a fragment or a variant thereof.

In other embodiments, the coding RNA of the composition for use comprises a 5'-UTR as described in WO2013/143700, the disclosure of WO2013/143700 relating to 5'-UTR sequences herewith incorporated by reference. Particularly preferred 5'-UTRs are nucleic acid sequences derived from SEQ ID NOs: 1-1363, SEQ ID NO: 1395, SEQ ID NO: 1421 and SEQ ID NO: 1422 of WO2013/143700, or fragments or variants of these sequences. In this context, it is preferred that the 5'-UTR of the RNA comprises or consists of a corresponding RNA sequence of the nucleic acid sequence according SEQ ID NOs: 1-1363, SEQ ID NO: 1395, SEQ ID NO: 1421 and SEQ ID NO: 1422 of WO2013/143700. In other embodiments, the coding RNA comprises a 5'-UTR as described in WO2016/107877, the disclosure of WO2016/107877 relating to 5'-UTR sequences herewith incorporated by reference. Particularly preferred 5'-UTRs are nucleic acid sequences according to SEQ ID NOs: 25 to 30 and SEQ ID NOs: 319 to 382 of WO2016/107877, or fragments or variants of these sequences. In this context, it is particularly preferred that the 5'-UTR of the RNA comprises or consists of a corresponding RNA sequence of the nucleic acid sequence according SEQ ID NOs: 25 to 30 and SEQ ID NOs: 319 to 382 of WO2016/107877. In other embodiments, the coding RNA comprises a 5'-UTR as described in WO2017/036580, the disclosure of WO2017/036580 relating to 5'-UTR sequences herewith incorporated by reference. Particularly preferred 5'-UTRs are nucleic acid sequences according to SEQ ID NOs: 1 to 151 of WO2017/036580, or fragments or variants of these sequences. In this context, it is particularly preferred that the 5'-UTR of the RNA comprises or consists of a corresponding RNA sequence of the nucleic acid sequence according to SEQ ID NOs: 1 to 151 of WO2017/036580. In other embodiments, the coding RNA comprises a 5'-UTR as described in WO2016022914, the disclosure of WO2016022914 relating to 5'-UTR sequences herewith incorporated by reference. Particularly preferred 5'-UTRs are nucleic acid sequences according to SEQ ID NOs: 3 to 19 of WO2016022914, or fragments or variants of these sequences. In this context, it is particularly preferred that the 5'-UTR of the RNA comprises or consists of a corresponding RNA sequence of the nucleic acid sequence according to SEQ ID NOs: 3 to 19 of WO2016022914.

Suitably, in preferred embodiments, the coding RNA of the composition for use comprises at least one cds encoding at least one peptide or protein as specified herein (e.g. therapeutic protein, CRISPR-associated endonuclease), operably linked to a 3'-UTR and/or a 5'-UTR selected from the following 5'-UTR/3'-UTR combinations: a-1 (HSD17B4/PSMB3), a-2 (NDUFA4/PSMB3), a-3 (SLC7A3/PSMB3), a-4 (NOSIP/PSMB3), a-5 (MP68/PSMB3), b-1 (UBQLN2/RPS9), b-2 (ASAH1/RPS9), b-3 (HSD17B4/RPS9), b-4 (HSD17B4/CASP1), b-5 (NOSIP/COX6B1), c-1 (NDUFA4/RPS9), c-2 (NOSIP/NDUFA1), c-3 (NDUFA4/COX6B1), c-4 (NDUFA4 /NDUFA1), c-5 (ATP5A1/PSMB3), d-1 (Rpl31/PSMB3), d-2 (ATP5A1/CASP1), d-3 (SLC7A3/GNAS), d-4 (HSD17B4/NDUFA1), d-5 (Slc7a3/Ndufa1), e-1 (TUBB4B/RPS9), e-2 (RPL31/RPS9), e-3 (MP68/RPS9), e-4 (NOSIP/RPS9), e-5 (ATP5A1/RPS9), e-6 (ATP5A1/COX6B1), f-1 (ATP5A1/GNAS), f-2 (ATP5A1/NDUFA1), f-3 (HSD17B4/COX6B1), f-4 (HSD17B4/GNAS), f-5 (MP68/COX6B1), g-1 (MP68/NDUFA1), g-2 (NDUFA4/CASP1), g-3 (NDUFA4/GNAS), g-4 (NOSIP/CASP1), g-5 (RPL31/CASP1), h-1 (RPL31/COX6B1), h-2 (RPL31/GNAS), h-3 (RPL31/NDUFA1), h-4 (Slc7a3/CASP1), h-5 (SLC7A3/COX6B1), i-1 (SLC7A3/RPS9), i-2 (RPL32/ALB7), i-2 (RPL32/ALB7), or i-3 (a-globin gene/-).

In various embodiments, the coding RNA of the composition for use comprises the following elements, preferably in 5'-to 3'-direction:
A) 5'-cap structure, preferably as specified herein;
B) 5'-terminal start element, preferably as specified herein;
C) optionally, a cleavage site for a catalytic nucleic acid molecule, preferably as specified herein;
D) optionally, 5'-UTR, preferably as specified herein;
E) a ribosome binding site, preferably as specified herein;
F) at least one coding sequence, preferably as specified herein;
G) 3'-UTR, preferably as specified herein;
H) optionally, poly(A) sequence, preferably as specified herein;
I) optionally, poly(C) sequence, preferably as specified herein;
J) optionally, histone stem-loop preferably as specified herein;
K) optionally, 3 terminal sequence element, preferably as specified herein.

In preferred embodiments the coding RNA of the composition for use comprises the following elements preferably in 5'-to 3'-direction:
A) 5'-cap structure selected from m7G(5'), m7G(5')ppp(5')(2'OMeA), or m7G(5')ppp(5')(2'OMeG);
B) 5'-terminal start element selected from **SEQ ID NOs: 408** or **409** or fragments or variants thereof;
C) optionally, a cleavage site for a catalytic nucleic acid molecule, preferably as specified herein;
D) optionally, 5'-UTR selected from **SEQ ID NOs: 366-387** or fragments or variants thereof;
E) a ribosome binding site selected from **SEQ ID NOs: 406** or **407** or fragments or variants thereof;
F) at least one coding sequence encoding at least one protein selected from **SEQ ID NOs: 2-365, 436** or fragments or variants thereof;
G) 3'-UTR selected from **SEQ ID NOs: 388-403;**
H) optionally, poly(A) sequence comprising about 50 to about 500 adenosines;
I) optionally, poly(C) sequence comprising about 10 to about 100 cytosines;
J) optionally, histone stem-loop (sequence) selected from **SEQ ID NOs: 404** or **405;**
K) optionally, 3'-terminal sequence element **SEQ ID NOs: 410-431.**

In particularly preferred embodiments the coding RNA of the composition for use comprises the following elements A to G in 5' to 3' direction:
A) 5'-cap structure selected from m7G(5'), m7G(5')ppp(5')(2'OMeA), or m7G(5')ppp(5')(2'OMeG);
B) 5'-terminal start element selected from **SEQ ID NOs: 408** or **409** or fragments or variants thereof;
C) 3'-UTR as specified herein;
D) a ribosome binding site selected from **SEQ ID NOs: 406** or **407** or fragments or variants thereof;
E) at least one coding sequence encoding at least one protein selected from **SEQ ID NOs: 2-365, 436** or fragments or variants thereof;
F) 5'-UTR as specified herein;
G) poly(A) sequence comprising about 50 to about 500 adenosines, preferably about 64 adenosines;
and
wherein the 3'-UTR and/or 5'-UTR element is selected from UTR combinations according to a-1, a-2, a-3, a-4, a-5, b-1, b-2, b-3, b-4, b-5, c-1, c-2, c-3, c-4, c-5, d-1, d-2, d-3, d-4, d-5, e-1, e-2, e-3, e-4, e-5, e-6, f-1, f-2, f-3, f-4, f-5, g-1, g-2, g-3, g-4, g-5, h-1, h-2, h-3, h-4, h-5, i-1, i-2, or i-3, as specified herein, wherein said RNA optionally comprises a poly(C) sequence comprising about 10 to about 100 cytosines, wherein said RNA optionally comprises a histone stem-loop selected from **SEQ ID NOs: 404** or **405.**

In preferred embodiments, the coding RNA of the composition for use comprises or consists of an RNA sequence which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 11011-11042; 11249-11280; 11131-11162; 11367-11398; 11485-11516; 11603-11634; 11721-11752; 11839-11870; 11044-11116; 11282-11354; 11164-11236; 11400-11472; 11518-11590; 11636-11708; 11754-11826; 11872-11944; 11011-11042; 11249-11280; 11044-11116; 11282-11354; 11131-11162; 11367-11398; 11485-11516; 11603-11634; 11721-11752; 11839-11870; 11164-11236; 11400-11472; 11518-11590; 11636-11708; 11754-11826; 11872-11944; 11120-11122; 11240; 11241; 11358; 11359; 11476; 11477; 11594; 11595; 11712; 11713; 11830; 11831; 11948; 11949; 11123-11130; 11360-11366; 11242-11248; 11478-11484; 11596-11602; 11714-11720; 11832-11838; 11950-11956 of published PCT patent application WO2018/172556, said RNA sequences from WO2018/172556 herewith incorporated by reference.

The composition for use may comprise a safe and effective amount of the coding RNA as specified herein. As used herein, "safe and effective amount" means an amount of the coding RNA that is sufficient to results in expression and/or activity of the encoded protein in the eye after administration via SCS delivery. At the same time, a "safe and effective amount" is small enough to avoid serious side-effects of the coding RNA/of the composition via SCS delivery.

A "safe and effective amount" of the coding RNA of the composition for use will furthermore vary in connection with the particular condition to be treated and also with the age and physical condition of the patient to be treated, the severity of the condition, the duration of the treatment, the nature of the accompanying therapy, of the particular pharmaceutically acceptable carrier used etc. Moreover, the "safe and effective amount" of the RNA or the composition as described herein may depend from application route (e.g. intravitreal, sub-retinal, SCS), application device (needle injection, injection device), and/or complexation/formulation (e.g. RNA in association with a polymeric carrier or LNP). Moreover, the "safe and effective amount" of the RNA or the composition may depend from the condition of the treated subject (infant, immunocompromised human subject etc.).

In the context of the invention, a "composition" refers to any type of composition in which the specified ingredients (e.g. RNA encoding peptide or protein as specified above e.g. in association with a polymeric carrier or LNP), may be incorporated, optionally along with any further constituents, usually with at least one pharmaceutically acceptable carrier or excipient. The composition may be a dry composition such as a powder or granules, or a solid unit such as a lyophilized form. Alternatively, the composition may be in liquid form, and each constituent may be independently incorporated in dissolved or dispersed (e.g. suspended or emulsified) form.

In preferred embodiments, the composition for use comprises at least one coding RNA and at least one pharmaceutically acceptable carrier or excipient. Accordingly, the composition for use is preferably pharmaceutical composition. Preferably, the carrier or excipient is suitable for ocular administration, preferably for SCS administration.

The term "pharmaceutically acceptable carrier" or "pharmaceutically acceptable excipient" as used herein preferably includes the liquid or non-liquid basis of the composition for administration. If the composition is provided in liquid form, the carrier may be water, e.g. pyrogen-free water; isotonic saline or buffered (aqueous) solutions, e.g. phosphate, citrate etc. buffered solutions. Water or preferably a buffer, more preferably an aqueous buffer, may be used, containing a sodium salt, preferably at least 50mM of a sodium salt, a calcium salt, preferably at least 0.01mM of a calcium salt, and optionally a potassium salt, preferably at least 3mM of a potassium salt. According to preferred embodiments, the sodium, calcium and, optionally, potassium salts may occur in the form of their halogenides, e.g. chlorides, iodides, or bromides, in the form of their hydroxides, carbonates, hydrogen carbonates, or sulfates, etc. Examples of sodium salts include NaCl, Nal, NaBr, Na₂CO₃, NaHCOs, Na₂SO₄, examples of the optional potassium salts include KCI, KI, KBr, K₂CO₃, KHCOs, K₂SO₄, and examples of calcium salts include CaCl₂, Cal₂, CaBr₂, CaCOs, CaSO₄, Ca(OH)₂.

Furthermore, organic anions of the aforementioned cations may be in the buffer. Accordingly, in embodiments, the composition for use may comprise pharmaceutically acceptable carriers or excipients using one or more pharmaceutically acceptable carriers or excipients to e.g. increase stability, increase cell transfection, permit the sustained or delayed, increase the translation of encoded protein in vivo, and/or alter the release profile of encoded protein in vivo. In addition to traditional excipients such as any and all solvents, dispersion media, diluents, or other liquid vehicles, dispersion or suspension aids, surface active agents, isotonic agents, thickening or emulsifying agents, preservatives, excipients of the present invention can include, without limitation, lipidoids, liposomes, lipid nanoparticles, polymers, lipoplexes, core-shell nanoparticles, peptides, proteins, cells transfected with polynucleotides, hyaluronidase, nanoparticle mimics and combinations thereof. In embodiments, one or more compatible solid or liquid fillers or diluents or encapsulating compounds may be used as well, which are suitable for administration to a subject. The term "compatible" as used herein means that the constituents of the composition are capable of being mixed with the at least one RNA and, optionally, a plurality of RNAs of the composition, in such a manner that no interaction occurs, which would substantially reduce the biological activity or the pharmaceutical effectiveness of the composition under typical use conditions (e.g., SCS administration). Pharmaceutically acceptable carriers or excipients must have sufficiently high purity and sufficiently low toxicity to make them suitable for administration to a subject to be treated. Compounds which may be used as pharmaceutically acceptable carriers or excipients may be sugars, such as, for example, lactose, glucose, trehalose, mannose, and sucrose; starches, such as, for example, corn starch or potato starch; dextrose; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The term "subject", "patient" or "individual" as used herein generally includes humans and non-human animals and preferably mammals, including chimeric and transgenic animals and disease models. Subjects to which administration of the compositions, preferably the pharmaceutical composition, is contemplated include, but are not limited to, humans and/or other primates; mammals, including commercially relevant mammals such as cattle, pigs, horses, sheep, cats, dogs; and/or birds, including commercially relevant birds such as poultry, chickens, ducks, geese, and/or turkeys. Preferably, the term "subject" refers a non-human primate or a human, most preferably a human.

In preferred embodiments, a "subject in need of treatment", or a "subject in need thereof" in the context of the invention is a human subject.

In embodiments, the composition for use may comprise a plurality or at least more than one of the coding RNA species as defined above, wherein each coding RNA species may encode a different peptide or protein.

In embodiments, the composition for use comprises more than one or a plurality, e.g. 2, 3, 4, 5, 6, 7, 8, 9, 10, 11, 12, 13, 14, 15 of different coding RNAs as defined above.

In a further preferred embodiment, the composition for use comprises at least one coding RNA encoding a CRISPR-associated protein, preferably at least one RNA which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 11011-11042; 11249-11280; 11131-11162; 11367-11398; 11485-11516; 11603-11634; 11721-11752; 11839-11870; 11044-11116; 11282-11354; 11164-11236; 11400-11472; 11518-11590; 11636-11708; 11754-11826; 11872-11944; 11011-11042; 11249-11280; 11044-11116; 11282-11354; 11131-11162; 11367-11398; 11485-11516; 11603-11634; 11721-11752; 11839-11870; 11164-11236; 11400-11472; 11518-11590; 11636-11708; 11754-11826; 11872-11944; 11120-11122; 11240; 11241; 11358; 11359; 11476; 11477; 11594; 11595; 11712; 11713; 11830; 11831; 11948; 11949; 11123-11130; 11360-11366; 11242-11248; 11478-11484; 11596-11602; 11714-11720; 11832-11838; 11950-11956, of published PCT patent application WO2018/172556, and additionally, at least one guide RNA. Suitably, SCS administration of the composition leads to expression of the encoded CRISPR-associated protein (provided by the coding RNA) in the eye, preferably in therapeutically relevant target cells of the eye (as defined above), preferably amenable to treatment by knocking in, knocking out, manipulating or modulating the expression of a gene of interest, well-orchestrated by the guide RNA of the composition. The respective guide RNA of the composition may be chosen based on the gene of interest associated with a deficiency, disease or disorder as defined above.

For the production of a composition for use comprising at least 2, 3, 4, 5, 6, 7, 8, 9, 10 RNA constructs, methods as disclosed in published patent application WO2017/1090134 are preferably used and adapted accordingly. In embodiments where guide RNA is comprised in the composition, said guide RNA may alternatively be produced by common chemical RNA synthesis methods.

In embodiments, the composition for use may further comprise an anti-inflammatory drug, a vascular endothelial growth factor (VEGF) modulator (e.g., anti-VEGF antibody), a platelet derived growth factor (PDGF) modulator, an angiogenesis inhibitor, an immunosuppressive agent, a vascular permeability inhibitor, or a combination thereof. In embodiments, the composition may comprise modulators and/or activators of any of the provided protein targets of **List 1.** In other embodiments, the composition for use may comprise at least one of the agents provided in paragraphs [1263] to [1276] of published PCT application WO2017/192565, the content herewith incorporated by reference.

In embodiments, the coding RNA comprised in a composition for use is provided in an amount of about 100ng to about 500µg, about 1µg to about 500µg, about 1µg to about 400µg, about 1µg to about 300µg, about 1µg to about 200µg, about 1µg to about 100µg, about 1µg to about 50µg, about 1µg to about 25µg, about 1µg to about 10pg, specifically, in an amount of about 1µg, about 2µg, about 3µg, about 4µg, about 5µg, about 6µg, about 7µg, about 8µg, about 9µg, about 10pg, about 15µg, about 20pg, about 25µg, about 30pg, about 35µg, about 40pg, about 45µg, about 50pg, about 60pg, about 70pg, about 80pg, about 90pg, about 100pg, about 200pg, about 300pg, about 400pg, about 500µg.

In a preferred embodiment, the at least one coding RNA of the composition for use is complexed or associated with, or at least partially complexed or partially associated with one or more cationic or polycationic compound, preferably cationic or polycationic polymer, cationic or polycationic polysaccharide, cationic or polycationic lipid, cationic or polycationic protein, or cationic or polycationic peptide, or any combinations thereof.

The term "cationic or polycationic compound" as used herein will be recognized and understood by the person of ordinary skill in the art, and are e.g. intended to refer to a charged molecule, which is positively charged at a pH value ranging from about 1 to 9, at a pH value ranging from about 3 to 8, at a pH value ranging from about 4 to 8, at a pH value ranging from about 5 to 8, more preferably at a pH value ranging from about 6 to 8, even more preferably at a pH value ranging from about 7 to 8, most preferably at a physiological pH, e.g. ranging from about 7.2 to about 7.5. Accordingly, a cationic component, e.g. a cationic peptide, cationic protein, cationic polymer, cationic polysaccharide, cationic lipid may be any positively charged compound or polymer which is positively charged under physiological conditions. A "cationic or polycationic peptide or protein" may contain at least one positively charged amino acid, or more than one positively charged amino acid, e.g. selected from Arg, His, Lys or Orn. Accordingly, "polycationic" components are also within the scope exhibiting more than one positive charge under the given conditions.

Cationic or polycationic compounds, being particularly preferred may be selected from the following list of cationic or polycationic peptides or proteins of fragments thereof: protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides, pAntp, plsl, FGF, Lactoferrin, Transportan, Buforin-2, Bac715-24, SynB, SynB(1), pVEC, hCT-derived peptides, SAP, or histones. More preferably, the coding RNA is complexed with one or more polycations, preferably with protamine or oligofectamine, most preferably with protamine.

Further preferred cationic or polycationic compounds, which can be used as complexation agent may include cationic polysaccharides, e.g. chitosan, polybrene etc.; cationic lipids, e.g. DOTMA, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS, DIMRI, DOTAP, DC-6-14, CLIP1, CLIP6, CLIP9, oligofectamine; or cationic or polycationic polymers, e.g. modified polyaminoacids, such as beta-aminoacid-polymers or reversed polyamides, etc., modified polyethylenes, such as PVP etc., modified acrylates, such as pDMAEMA etc., modified amidoamines such as pAMAM etc., modified polybetaaminoester (PBAE), such as diamine end modified 1,4 butanediol diacrylate-co-5-amino-1-pentanol polymers, etc., dendrimers, such as polypropylamine dendrimers or pAMAM based dendrimers, etc., polyimine(s), such as PEI, poly(propyleneimine), etc., polyallylamine, sugar backbone based polymers, such as cyclodextrin based polymers, dextran based polymers, etc., silan backbone based polymers, such as PMOXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

In this context it is particularly preferred that the coding RNA of the composition for use is complexed or at least partially complexed with a cationic or polycationic compound and/or a polymeric carrier, preferably cationic proteins or peptides. In this context, the disclosure of WO2010/037539 and WO2012/113513 is incorporated herewith by reference. Partially means that only a part of the RNA of the composition is complexed with a cationic compound and that the rest of the RNA is present in uncomplexed form ("free").

In this context it is particularly preferred that the coding RNA of the composition is complexed, or at least partially complexed with protamine. Preferably, the molar ratio of the nucleic acid, particularly the RNA of the protamine-complexed RNA to the free RNA may be selected from a molar ratio of about 0.001:1 to about 1:0.001, including a ratio of about 1:1. Suitably, the complexed RNA is complexed with protamine by addition of protamine-trehalose solution to the RNA sample at a RNA:protamine weight to weight ratio (w/w) of 2:1.

Further preferred cationic or polycationic proteins or peptides that may be used for complexation can be derived from formula (Arg)I;(Lys)m;(His)n;(Orn)o;(Xaa)x of the patent application WO2009/030481 or WO2011/026641, the disclosure of WO2009/030481 or WO2011/026641 relating thereto incorporated herewith by reference.

In preferred embodiments, the at least one coding RNA is complexed or at least partially complexed with at least one cationic or polycationic proteins or peptides preferably selected from **SEQ ID NOs: 432-435, 437** or any combinations thereof.

According to embodiments, the composition comprises at least one coding RNA as defined herein, and a polymeric carrier.

The term "polymeric carrier" as used herein will be recognized and understood by the person of ordinary skill in the art, and is e.g. intended to refer to a compound that facilitates transport and/or complexation of another compound (e.g. RNA cargo). A polymeric carrier is typically a carrier that is formed of a polymer. A polymeric carrier may be associated to its cargo (e.g. RNA) by covalent or non-covalent interaction. A polymer may be based on different subunits, such as a copolymer.

Suitable polymeric carriers in that context may include, e.g., polyacrylates, polyalkycyanoacrylates, polylactide, polylactide-polyglycolide copolymers, polycaprolactones, dextran, albumin, gelatin, alginate, collagen, chitosan, cyclodextrins, protamine, PEGylated protamine, PEGylated PLL and polyethylenimine (PEI), dithiobis(succinimidylpropionate) (DSP), Dimethyl-3,3'-dithiobispropionimidate (DTBP), polyethylene imine) biscarbamate (PEIC), poly(L-lysine) (PLL), histidine modified PLL, poly(N-vinylpyrrolidone) (PVP), poly(propylenimine (PPI), poly(amidoamine) (PAMAM), poly(amido ethylenimine) (SS-PAEI), triehtylenetetramine (TETA), poly(β-aminoester), poly(4-hydroxy-L-proine ester) (PHP), poly(allylamine), poly(α-[4-aminobutyl]-L-glycolic acid (PAGA), Poly(D,L-lactic-co-glycolid acid (PLGA), Poly(N-ethyl-4-vinylpyridinium bromide), poly(phosphazene)s (PPZ), poly(phosphoester)s (PPE), poly(phosphoramidate)s (PPA), poly(N-2-hydroxypropylmethacrylamide) (pHPMA), poly(2-(dimethylamino)ethyl methacrylate) (pDMAEMA), poly(2-aminoethyl propylene phosphate) PPE_EA), galactosylated chitosan, N-dodecylated chitosan, histone, collagen and dextran-spermine. In one embodiment, the polymer may be an inert polymer such as, but not limited to, PEG. In one embodiment, the polymer may be a cationic polymer such as, but not limited to, PEI, PLL, TETA, poly(allylamine), Poly(N-ethyl-4-vinylpyridinium bromide), pHPMA and pDMAEMA. In one embodiment, the polymer may be a biodegradable PEI such as, but not limited to, DSP, DTBP and PEIC. In one embodiment, the polymer may be biodegradable such as, but not limited to, histine modified PLL, SS-PAEI, poly(β-aminoester), PHP, PAGA, PLGA, PPZ, PPE, PPA and PPE-EA.

When PEI is present, it may be branched PEl of a molecular weight ranging from 10 to 40 kDA, e.g., 25 kDa branched PEl (Sigma #408727).

In some embodiments, the polymeric carrier comprises PEI. In some embodiments, PEI is branched PEI. PEI may be a branched PEl of a molecular weight ranging from 10 to 40 kDA, e.g., 25 kDa. In some embodiments, PEI is linear PEI. In some embodiments, the PEI nanoparticle has a mean diameter of or less than about 60nm (e.g., of or less than about 55nm, of or less than about 50nm, of or less than about 45nm, of or less than about 40nm, of or less than about 35nm, of or less than about 30nm, or of or less than about 25nm). Suitable nanoparticles may be in the range of 25nm to 60nm, e.g. 30nm to 50nm. As used herein, the mean diameter may be represented by the z-average as determined by dynamic light scattering as commonly known in the art.

A suitable polymeric carrier may be a polymeric carrier formed by disulfide-crosslinked cationic compounds. The disulfide-crosslinked cationic compounds may be the same or different from each other. The polymeric carrier can also contain further components (e.g. lipidoid compound). The polymeric carrier used according to the present invention may comprise mixtures of cationic peptides, proteins or polymers and optionally further components as defined herein, which are crosslinked by disulfide bonds (via -SH groups).

In this context, polymeric carriers according to formula (Ia) {(Arg)I;(Lys)m;(His)n;(Orn)o;(Xaa')x(Cys)y} and formula (Ib) Cys{(Arg)I;(Lys)m;(His)n;(Orn)o;(Xaa)x}Cys of published PCT application WO2012/013326 are preferred, the disclosure of WO2012/013326 relating thereto incorporated herewith by reference.

In embodiments, the polymeric carrier used to complex the at least one coding RNA may be derived from a polymeric carrier molecule according formula (L-P¹-S-[S-P²-S]ₙ-S-P³-L) of published PCT application WO2011/026641, the disclosure of WO2011/026641 relating thereto incorporated herewith by reference.

In embodiments, the polymeric carrier compound is formed by, or comprises, or consists of the peptide elements CysArg12Cys **(SEQ ID NO: 432)** or CysArg12 **(SEQ ID NO: 433)** or TrpArg12Cys **(SEQ ID NO: 434).** In other embodiments, the polymeric carrier compound is formed by, or comprises, or consists of the peptide elements according **to SEQ ID NO: 435** or **437.**

In particularly preferred embodiments, the polymeric carrier compound consists of a (R₁₂C)-(R₁₂C) dimer, a (WR₁₂C)-(WR₁₂C) dimer, or a (CR₁₂)-(CR₁₂C)-(CR₁₂) trimer, wherein the individual peptide elements in the dimer (e.g. (WR12C)), or the trimer (e.g. (CR12)), are connected via -SH groups.

In a preferred embodiments, the at least one coding RNA of the composition for use is complexed or associated with a polyethylene glycol/peptide polymer comprising HG-PEG5000-S-(S-CHHHHHHRRRRHHHHHHC-S-)7-S-PEG5000-OH **(SEQ ID NO: 435** as peptide monomer).

In a preferred embodiments, the at least one coding RNA of the composition for use is complexed or associated with a polyethylene glycol/peptide polymer comprising HG-PEG5000-S-(S-CHHHHHHRRRRHHHHHHC-S-)4-S-PEG5000-OH **(SEQ ID NO: 435** as peptide monomer).

In another preferred embodiments, the at least one coding RNA of the composition for use is complexed or associated with a polyethylene glycol/peptide polymer comprising HG-PEG5000-S-(S-CGHHHHHRRRRHHHHHGC-S-)7-S-PEG5000-OH **(SEQ ID NO: 437** as peptide monomer).

In particularly preferred embodiments, the at least one coding RNA of the composition for use is complexed or associated with a polyethylene glycol/peptide polymer comprising HO-PEG5000-S-(S-CGHHHHHRRRRHHHHHGC-S-)4-S-PEG5000-OH **(SEQ ID NO: 437** as peptide monomer).

In other embodiments, the composition comprises at least one coding RNA complexed or associated with polymeric carriers and, optionally, with at least one lipid or lipidoid component as described in published PCT applications WO2017/212008A1, WO2017/212006A1, WO2017/212007A1, and WO2017/212009A1. In this context, the disclosures of WO2017/212008A1, WO2017/212006A1, WO2017/212007A1, and WO2017212009A1 are herewith incorporated by reference.

In a particularly preferred embodiment, the polymeric carrier is a peptide polymer, preferably a polyethylene glycol/peptide polymer as defined above, and a lipid component, preferably a lipidoid component, more preferably lipidoid component.

A lipidoid compound, also simply referred to as lipidoid (or lipidoit), is a lipid-like compound, i.e. an amphiphilic compound with lipid-like physical properties. The lipidoid compound is preferably a compound which comprises two or more cationic nitrogen atoms and at least two lipophilic tails. In contrast to many conventional cationic lipids, the lipidoid compound may be free of a hydrolysable linking group, in particular linking groups comprising hydrolysable ester, amide or carbamate groups. The cationic nitrogen atoms of the lipidoid may be cationisable or permanently cationic, or both types of cationic nitrogens may be present in the compound. In the context of the present invention the term lipid is considered to also encompass lipidoid compounds.

In preferred embodiments the coding RNA of the composition for use is complexed or associated with a polymeric carrier, preferably with a polyethylene glycol/peptide polymer as defined above, and a lipidoid component.

As mentioned, the cationic lipidoid compound, also simply referred to as lipidoid, is a lipid-like compound, i.e. an amphiphilic compound with lipid-like physical properties.

In one embodiment, the lipidoid is a compound comprising at least two cationic nitrogen atoms and at least two lipophilic tails. As used herein, a "tail" is a substructure of a molecule representing a chain or chain-like structure, such as an optionally substituted hydrocarbyl, acyl or acyloxylalkyl chain of at least four, and more preferably at least six, carbon atoms. The optionally substituted hydrocarbyl, acyl or acyloxyalkyl chain representing the lipophilic tail may be directly connected with a cationic nitrogen atom.

In one specific embodiment, the lipidoid is a compound comprising two identical lipophilic tails, each of which is directly connected with a cationic nitrogen atom. In another specific embodiment, the lipidoid is a compound comprising three identical lipophilic tails, each tail being directly connected with a cationic nitrogen atom. In a further specific embodiment, the lipidoid is a compound comprising four or more identical lipophilic tails, each tail being directly connected with a cationic nitrogen atom. In each of these embodiments, the lipidoid may optionally comprise a further nitrogen atom to which no lipophilic tail is connected. Such lipidoid may also be understood as a compound having a cationic backbone derived from an oligoamine with the lipophilic tails being attached to the, or some of the, cationic nitrogens of the oligoamine.

If the lipophilic tails are substituted hydrocarbyl (e.g. alkyl) chains, the substituent may, e.g., be a methyl or a hydroxyl.

The optionally substituted hydrocarbyl chain may be saturated, such as to resemble an alkyl, or it may be unsaturated, i.e. an alkenyl or alkynyl, each optionally having one, two, three or more carbon-carbon double bonds and/or triple bonds. In the case of tail structures representing or including an acyl or acyloxyalkyl group, these may also comprise one, two or more carbon-carbon double bonds and/or triple bonds in the hydrocarbon segment of the tail.

In one embodiment, the lipidoid compound is free of hydrolysable linking groups, such as ester, amide or carbamate groups. As used herein, a linking group is a group which links the lipophilic tails of the lipidoid molecule to the hydrophilic region comprising the cationic nitrogen atoms. Conventional cationic lipids that have been proposed as carriers or agents to deliver nucleic acids to cells and enhance transfection often - if not typically - exhibit such linkers or linking groups, which are most often hydrolysable and/or enzymatically cleavable. In particular, linkers with ester groups have been proposed, but also linkers with amide groups or carbamate groups, all of which are susceptible to hydrolytic and/or enzymatic cleavage *in vivo.*

As used herein, hydrolysable means that an appreciable degree of hydrolysis occurs in a physiological fluid (such as interstitial fluid) under *in vivo* conditions within seconds, minutes, hours, or days; preferably, the respective compound or group is hydrolysed to at least 50% after not more than 7 days, or even after not more than 2 days.

In some embodiments of the inventions, the lipidoid compound comprises a PEG moiety.

As said, the lipidoid compound is cationic, which means that it is cationisable or permanently cationic. In one embodiment, the lipidoid is cationisable, i.e. it comprises one or more cationisable nitrogen atoms, but no permanently cationic nitrogen atoms. In another embodiment, at least one of the cationic nitrogen atoms of the lipidoid is permanently cationic. Optionally, the lipidoid comprises two permanently cationic nitrogen atoms, three permanently cationic nitrogen atoms, or even four or more permanently cationic nitrogen atoms.

In preferred embodiment the coding RNA of the composition for use is complexed or associated with a polymeric carrier, preferably with a polyethylene glycol/peptide polymer as defined above, and a lipidoid component, wherein the lipidoid component is a compound according to formula A 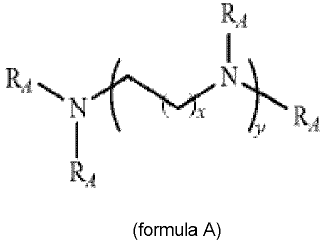 wherein
- R_{A} is independently selected for each occurrence an unsubstituted, cyclic or acyclic, branched or unbranched C₁₋₂₀ aliphatic group; a substituted or unsubstituted, cyclic or acyclic, branched or unbranched C₁₋₂₀ heteroaliphatic group; a substituted or unsubstituted aryl; a substituted or unsubstituted heteroaryl; 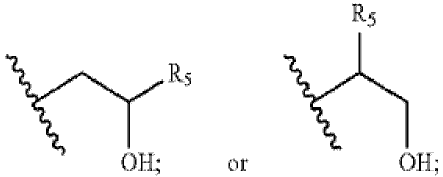 wherein at least one R_{A} is 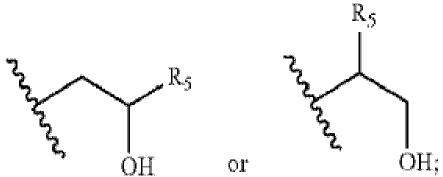
- R₅ is independently selected for each occurrence of from an unsubstituted, cyclic or acyclic, branched or unbranched C₈₋₁₆ aliphatic; a substituted or unsubstituted aryl; or a substituted or unsubstituted heteroaryl;
- each occurrence of x is an integer from 1 to 10;
- each occurrence of y is an integer from 1 to 10;
or a pharmaceutically acceptable salt thereof.

In a preferred embodiment, the lipidoid component may be any one selected from the lipidoids of the lipidoids provided in the table of page 50-54 of published PCT patent application WO2017212009A1, the specific lipidoids provided in said table, and the specific disclosure relating thereto herewith incorporated by reference.

Accordingly, in in a preferred embodiments, the lipidoid component may be any one selected from 3-C12-OH, 3-C12-OH-cat, 3-C12-amide, 3-C12-amide monomethyl, 3-C12-amide dimethyl, RevPEG(10)-3-C12-OH, RevPEG(10)-DLin-pAbenzoic, 3C12amide-TMA cat., 3C12amide-DMA, 3C12amide-NH2, 3C12amide-OH, 3C12Ester-OH, 3C12 Ester-amin, 3C12Ester-DMA, 2C12Amid-DMA, 3C12-lin-amid-DMA, 2C12-sperm-amid-DMA, or 3C12-sperm-amid-DMA (see table of published PCT patent application WO2017/212009A1 (pages 50-54).

In preferred embodiments, the peptide polymer comprising a lipidoid as specified above is used to complex the coding RNA of the composition for use to form complexes having an N/P ratio from about 0.1 to about 20, or from about 0.2 to about 15, or from about 2 to about 15, or from about 2 to about 12, wherein the N/P ratio is defined as the mole ratio of the nitrogen atoms of the basic groups of the cationic peptide or polymer to the phosphate groups of the nucleic acid. In that context, the disclosure of published PCT patent application WO2017/212009A1, in particular claims 1 to 10 of WO2017/212009A1, and the specific disclosure relating thereto is herewith incorporated by reference.

Further suitable lipidoid compounds may be derived from published PCT patent application WO2010/053572. In particular, lipidoid compounds derivable from claims 1 to 297 of published PCT patent application WO2010/053572 may be used in the context of the invention, e.g. incorporated into the peptide polymer as described herein, or e.g. incorporated into the lipid nanoparticle (as described below). Accordingly, claims 1 to 297 of published PCT patent application WO2010053572, and the specific disclosure relating thereto, is herewith incorporated by reference.

In preferred embodiments, the at least one coding RNA of the composition for use is complexed, partially complexed, encapsulated, partially encapsulated, or associated with one or more lipids (e.g. cationic lipids and/or neutral lipids), thereby forming liposomes, lipid nanoparticles (LNPs), lipoplexes, and/or nanoliposomes.

The liposomes, lipid nanoparticles (LNPs), lipoplexes, and/or nanoliposomes - incorporated coding RNA may be completely or partially located in the interior space of the liposomes, lipid nanoparticles (LNPs), lipoplexes, and/or nanoliposomes, within the membrane, or associated with the exterior surface of the membrane. The incorporation of an RNA is also referred to herein as "encapsulation" wherein the coding RNA is entirely contained within the interior space of the liposomes, lipid nanoparticles (LNPs), lipoplexes, and/or nanoliposomes. The purpose of incorporating a coding RNA into liposomes, lipid nanoparticles (LNPs), lipoplexes, and/or nanoliposomes is to protect the RNA from an environment which may contain enzymes or chemicals that degrade RNA and/or systems or receptors that cause the rapid excretion of the RNA. Moreover, incorporating an RNA into liposomes, lipid nanoparticles (LNPs), lipoplexes, and/or nanoliposomes may promote the uptake of the RNA, and hence, may enhance the therapeutic effect of the RNA after SCS administration

In this context, the terms "complexed" or "associated" refer to the essentially stable combination of coding RNA as defined herein with one or more lipids into larger complexes or assemblies without covalent binding.

The term "lipid nanoparticle", also referred to as "LNP", is not restricted to any particular morphology, and include any morphology generated when a cationic lipid and optionally one or more further lipids are combined, e.g. in an aqueous environment and/or in the presence of RNA. E.g., a liposome, a lipid complex, a lipoplex and the like are within the scope of an LNP.

Liposomes, lipid nanoparticles (LNPs), lipoplexes, and/or nanoliposomes can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50nm in diameter, and a large unilamellar vesicle (LUV) which may be between 50nm and 500nm in diameter.

LNPs of the invention are suitably characterized as microscopic vesicles having an interior aqua space sequestered from an outer medium by a membrane of one or more bilayers. Bilayer membranes of LNPs are typically formed by amphiphilic molecules, such as lipids of synthetic or natural origin that comprise spatially separated hydrophilic and hydrophobic domains. Bilayer membranes of the liposomes can also be formed by amphophilic polymers and surfactants (e.g., polymerosomes, niosomes, etc.). In the context of the present invention, an LNP typically serves to transport the coding RNA of the composition for use to a target cell or tissue in the eye.

Accordingly, in preferred embodiments, the at least one coding RNA of the composition for use is complexed with one or more lipids thereby forming lipid nanoparticles (LNP).

LNPs typically comprise a cationic lipid and one or more excipient selected from neutral lipids, charged lipids, steroids and polymer conjugated lipids (e.g. PEGylated lipid). The at least one coding RNA may be encapsulated in the lipid portion of the LNP or an aqueous space enveloped by some or the entire lipid portion of the LNP. The at least one coding RNA or a portion thereof may also be associated and complexed with the LNP. An LNP may comprise any lipid capable of forming a particle to which the nucleic acids are attached, or in which the one or more nucleic acids are encapsulated. Preferably, the LNP comprising coding RNA comprises one or more cationic lipids, and one or more stabilizing lipids. Stabilizing lipids include neutral lipids and PEGylated lipids.

The cationic lipid of an LNP may be cationisable, i.e. it becomes protonated as the pH is lowered below the pK of the ionizable group of the lipid, but is progressively more neutral at higher pH values. At pH values below the pK, the lipid is then able to associate with negatively charged nucleic acids. In certain embodiments, the cationic lipid comprises a zwitterionic lipid that assumes a positive charge on pH decrease.

The LNP may comprise any further cationic or cationisable lipid, i.e. any of a number of lipid species which carry a net positive charge at a selective pH, such as physiological pH.

Such lipids include, but are not limited to, DSDMA, N,N-dioleyl-N,N-dimethylammonium chloride (DODAC), N,N-distearyl-N,N-dimethylammonium bromide (DDAB), 1,2-dioleoyltrimethyl ammonium propane chloride (DOTAP) (also known as N-(2,3-dioleoyloxy)propyl)-N,N,N-trimethylammonium chloride and 1,2-Dioleyloxy-3-trimethylaminopropane chloride salt), N-(1-(2,3-dioleyloxy)propyl)-N,N,N-trimethylammonium chloride (DOTMA), N,N-dimethyl-2,3-dioleyloxy)propylamine (DODMA), ckk-E12, ckk, 1,2-DiLinoleyloxy-N,N-dimethylaminopropane (DLinDMA), 1,2-Dilinolenyloxy-N,N-dimethylaminopropane (DLenDMA), 1,2-di-y-linolenyloxy-N,N-dimethylaminopropane (γ-DLenDMA), 98N12-5, 1,2-Dilinoleylcarbamoyloxy-3- dimethylaminopropane (DLin-C-DAP), 1,2-Dilinoleyoxy-3-(dimethylamino)acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S- DMA), 1-Linoleoyl-2-linoleyloxy-3-dimethylaminopropane (DLin-2-DMAP), 1,2-Dilinoleyloxy-3-trimethylaminopropane chloride salt (DLin-TMA.Cl), ICE (Imidazol-based), HGT5000, HGT5001, DMDMA, CLinDMA, CpLinDMA, DMOBA, DOcarbDAP, DLincarbDAP, DLinCDAP, KLin-K-DMA, DLin-K-XTC2-DMA, XTC (2,2-Dilinoleyl-4-dimethylaminoethyl-[l,3]-dioxolane) HGT4003, 1,2-Dilinoleoyl-3-trimethylaminopropane chloride salt (DLin-TAP.CI), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N- dimethylamino)ethoxypropane (DLin-EG-DM A), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine, (6Z,9Z,28Z,31Z)-heptatriaconta-6,9,28,31-tetraen-19-yl-4-(dimethylamino)butanoate (MC3), ALNY-100 ((3aR,5s,6aS)- N,N-dimethyl-2,2-di((9Z,12Z)-octadeca-9,12-dienyl)tetrahydro-3aH-cyclopenta[d] [1 ,3]dioxol- 5-amine)), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (C12-200), 2,2-dilinoleyl-4-(2-dimethylaminoethyl)-[1,3]-dioxolane (DLin-K-C2-DMA), 2,2-dilinoleyl-4-dimethylaminomethyl-[1,3]-dioxolane (DLin-K-DMA), NC98-5 (4,7, 13-tris(3-oxo-3-(undecylamino)propyl)-NI ,N 16-diundecyl-4,7, 10,13-tetraazahexadecane-l,16-diamide), (6Z,9Z,28Z,31Z)-heptatriaconta-6,9,28,31-tetraen-19-yl 4-(dimethylamino) butanoate (DLin-M-C3-DMA), 3-((6Z,9Z,28Z,31Z)-heptatriaconta-6,9,28,31-tetraen-19-yloxy)-N,N-dimethylpropan-1-amine (MC3 Ether), 4-((6Z,9Z,28Z,31Z)-heptatriaconta-6,9,28,31-tetraen-19-yloxy)-N,N-dimethylbutan-l-amine (MC4 Ether), LIPOFECTIN^{®} (commercially available cationic liposomes comprising DOTMA and 1,2-dioleoyl-sn-3phosphoethanolamine (DOPE), from GIBCO/BRL, Grand Island, N.Y.); LIPOFECTAMINE^{®} (commercially available cationic liposomes comprising N-(1-(2,3dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.) or any combination of any of the foregoing. Further suitable cationic lipids for use in the compositions and methods of the invention include those described in international patent publications WO2010/053572 (and particularly, Cl 2-200 described at paragraph [00225]) and WO2012/170930, both of which are incorporated herein by reference, HGT4003, HGT5000, HGTS001, HGT5001, HGT5002 (see US20150140070A1).

In some embodiments, the lipid is selected from the group consisting of 98N12-5, C12-200, and ckk-E12.

In one embodiment, the further cationic lipid is an amino lipid.

Representative amino lipids include, but are not limited to, 1,2-dilinoleyoxy-3-(dimethylamino)acetoxypropane (DLin-DAC), 1,2-dilinoleyoxy-3morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3dimethylaminopropane (DLin-2-DMAP), 1,2-dilinoleyloxy-3-trimethylaminopropane chloride salt (DLin-TMA.CI), 1,2-dilinoleoyl-3-trimethylaminopropane chloride salt (DLin-TAP.CI), 1,2-dilinoleyloxy-3-(N-methylpiperazino)propane (DLin-MPZ), 3-(N,Ndilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA), 2,2-dilinoleyl-4-(2-dimethylaminoethyl)-[l,3]-dioxolane (DLin-KC2-DMA); dilinoleyl-methyl-4-dimethylaminobutyrate (DLin-MC3-DMA); MC3 (US20100324120).

In one embodiment, the at least one RNA may be formulated in an aminoalcohol lipidoid. Aminoalcohol lipidoids which may be used in the present invention may be prepared by the methods described in U.S. Patent No. 8,450,298, herein incorporated by reference in its entirety. Suitable (ionizable) lipids can also be the compounds as disclosed in Tables 1, 2 and 3 and as defined in claims 1-24 of published PCT patent application WO2017/075531A1, the specific disclosure hereby incorporated by reference.

In another embodiment, ionizable lipids can also be the compounds as disclosed in published PCT patent application WO2015/074085A1 (i.e. ATX-001 to ATX-032 or the compounds as specified in claims 1-26), U.S. Appl. Nos. 61/905,724 and 15/614,499 or U.S. Patent Nos. 9,593,077 and 9,567,296, the specific disclosure hereby incorporated by reference.

In that context, any lipid derived from generic formula (X1) 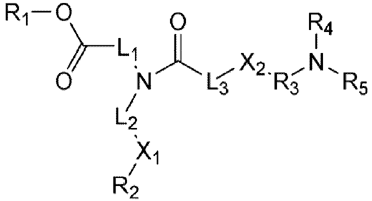 wherein, Ri and R2 are the same or different, each a linear or branched alkyl consisting of 1 to 9 carbons, an alkenyl or alkynyl consisting of 2 to 11carbons, Li and L2 are the same or different, each a linear alkylene or alkenylene consisting of 5 to 18 carbons, or forming a heterocycle with N, Xi is a bond, or is -CO-O- whereby -L2-CO-O-R2 is formed, X2 is S or O, L3 is a bond or a linear or branched alkylene consisting of 1 to 6 carbons, or forming a heterocycle with N, R3 is a linear or branched alkylene consisting of 1 to 6 carbons, and R4 and R 5 are the same or different, each hydrogen or a linear or branched alkyl consisting of 1 to 6 carbons; or a pharmaceutically acceptable salt thereof may be suitably used.

In other embodiments, suitable cationic lipids can also be the compounds as disclosed in published PCT patent application WO2017/117530A1 (i.e. lipids 13, 14, 15, 16, 17, 18, 19, 20, or the compounds as specified in the claims), the specific disclosure hereby incorporated by reference.

In that context, any lipid derived from generic formula (X2) 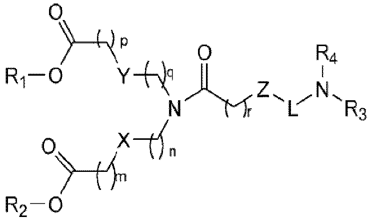 wherein
X is a linear or branched alkylene or alkenylene, monocyclic, bicyclic, or tricyclic arene or heteroarene;
Y is a bond, an ethene, or an unsubstituted or substituted aromatic or heteroaromatic ring; Z is S or 0;
L is a linear or branched alkylene of 1 to 6 carbons;
R-3 and R4 are independently a linear or branched alkyl of 1 to 6 carbons;
Ri and R2 are independently a linear or branched alkyl or alkenyl of 1 to 20 carbons; r is 0 to 6; and
m, n, p, and q are independently 1 to 18;
wherein when n=q, m=p, and Ri=R2, then X and Y differ;
wherein when X=Y, n=q, m=p, then Ri and R2 differ;
wherein when X=Y, n=q, and Ri=R2, then m and p differ; and
wherein when X=Y, m=p, and Ri=R2, then n and q differ;
or a pharmaceutically acceptable salt thereof.

In preferred embodiments, a lipid may be used derived from formula (X2), wherein, X is a bond, linear or branched alkylene, alkenylene, or monocyclic, bicyclic, or tricyclic arene or heteroarene; Y is a monocyclic, bicyclic, or tricyclic arene or heteroarene; Z is S or O; L is a linear or branched alkylene of 1 to 6 carbons; R3 and R4 are independently a linear or branched alkyl of 1 to 6 carbons; Ri and R2 are independently a linear or branched alkyl or alkenyl of 1 to 20 carbons; r is 0 to 6; and m, n, p, and q are independently 1 to 18; or a pharmaceutically acceptable salt thereof may be suitably used.

In preferred embodiments, ionizable lipids may also be selected from the lipids disclosed in published PCT patent application WO2018/078053A1 (i.e. lipids derived form formula I, II, and III of WO2018078053A1, or lipids as specified in Claims 1 to 12 of WO2018/078053A1), the specific disclosure of WO2018/078053A1 relating thereto hereby incorporated by reference. In that context, lipids disclosed in Table 7 of WO2018/078053A1 (e.g. lipids derived from formula I-1 to 1-41) and lipids disclosed in Table 8 of WO2018/078053A1 (e.g. lipids derived from formula II-1 to II-36) may be suitably used in the context of the invention. Accordingly, formula I-1 to formula 1-41 and formula II-1 to formula II-36 of WO2018/078053A1, and the specific disclosure relating thereto, are herewith incorporated by reference.

In particularly preferred embodiments, the at least one coding RNA of the composition for use is complexed with one or more lipids thereby forming LNPs, wherein the cationic lipid of the LNP is selected from structures III-1 to III-36 of Table 9 of published PCT patent application WO2018/078053A1. Accordingly, formula III-1 to III-36 of WO2018/078053A1, and the specific disclosure relating thereto, are herewith incorporated by reference.

In particularly preferred embodiment, the at least one coding RNA of the composition is complexed with one or more lipids thereby forming LNPs, wherein the LNP comprises the following cationic lipid:

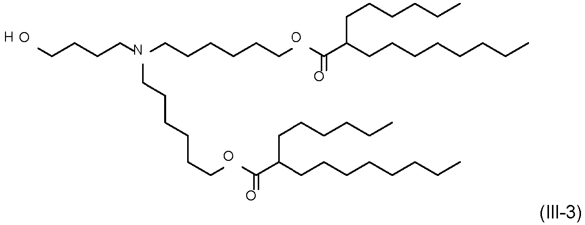

In certain embodiments, the cationic lipid is present in the LNP in an amount from about 30 to about 95 mole percent, relative to the total lipid content of the LNP. If more than one cationic lipid is incorporated within the LNP, such percentages apply to the combined cationic lipids.

In one embodiment, the cationic lipid is present in the LNP in an amount from about 30 to about 70 mole percent. In one embodiment, the cationic lipid is present in the LNP in an amount from about 40 to about 60 mole percent, such as about 40, 41, 42, 43, 44, 45, 46, 47, 48, 49, 50, 51, 52, 53, 54, 55, 56, 57, 58, 59 or 60 mole percent, respectively. In embodiments, the cationic lipid is present in the LNP in an amount from about 47 to about 48 mole percent, such as about 47.0, 47.1, 47.2, 47.3, 47.4, 47.5, 47.6, 47.7, 47.8, 47.9, 50.0 mole percent, respectively, wherein 47.7 mole percent are particularly preferred.

In some embodiments, the cationic lipid is present in a ratio of from about 20mol% to about 70 or 75mol% or from about 45 to about 65mol% or about 20, 25, 30, 35, 40, 45, 50, 55, 60, 65, or about 70mol% of the total lipid present in the LNP. In further embodiments, the LNPs comprise from about 25% to about 75% on a molar basis of cationic lipid, e.g., from about 20 to about 70%, from about 35 to about 65%, from about 45 to about 65%, about 60%, about 57.5%, about 57.1%, about 50% or about 40% on a molar basis (based upon 100% total moles of lipid in the LNP). In some embodiments, the ratio of cationic lipid to RNA is from about 3 to about 15, such as from about 5 to about 13 or from about 7 to about 11.

In some embodiments of the invention the LNP comprises a combination or mixture of any the lipids described above.

Other suitable (cationic or ionizable) lipids are disclosed in published patent applications WO2009/086558, WO2009/127060, WO2010/048536, WO2010/054406, WO2010/088537, WO2010/129709, WO2011/153493, WO 2013/063468, US2011/0256175, US2012/0128760, US2012/0027803, US8158601, WO2016/118724, WO2016/118725, WO2017/070613, WO2017/070620, WO2017/099823, WO2012/040184, WO2011/153120, WO2011/149733, WO2011/090965, WO2011/043913, WO2011/022460, WO2012/061259, WO2012/054365, WO2012/044638, WO2010/080724, WO2010/21865, WO2008/103276, WO2013/086373, WO2013/086354, and US Patent Nos. 7,893,302, 7,404,969, 8,283,333, 8,466,122 and 8,569,256 and US Patent Publication No. US2010/0036115, US2012/0202871, US2013/0064894, US2013/0129785, US2013/0150625, US20130178541, US2013/0225836, US2014/0039032 and WO2017/112865. In that context, the disclosures of WO2009/086558, WO2009/127060, WO2010/048536, WO2010/054406, WO2010/088537, WO2010/129709, WO2011/153493, WO 2013/063468, US2011/0256175, US2012/0128760, US2012/0027803, US8158601, WO2016/118724, WO2016/118725, WO2017/070613, WO2017/070620, WO2017/099823, WO2012/040184, WO2011/153120, WO2011/149733, WO2011/090965, WO2011/043913, WO2011/022460, WO2012/061259, WO2012/054365, WO2012/044638, WO2010/080724, WO2010/21865, WO2008/103276, WO2013/086373, WO2013/086354, US Patent Nos. 7,893,302, 7,404,969, 8,283,333, 8,466,122 and 8,569,256 and US Patent Publication No. US2010/0036115, US2012/0202871, US2013/0064894, US2013/0129785, US2013/0150625, US20130178541, US2013/0225836 and US2014/0039032 and WO2017/112865 specifically relating to (cationic) lipids suitable for LNPs are incorporated herewith by reference.

In some embodiments, amino or cationic lipids as defined herein have at least one protonatable or deprotonatable group, such that the lipid is positively charged at a pH at or below physiological pH (e.g. pH 7.4), and neutral at a second pH, preferably at or above physiological pH. It will, of course, be understood that the addition or removal of protons as a function of pH is an equilibrium process, and that the reference to a charged or a neutral lipid refers to the nature of the predominant species and does not require that all of lipids have to be present in the charged or neutral form. Lipids having more than one protonatable or deprotonatable group, or which are zwitterionic, are not excluded and may likewise suitable in the context of the present invention.

In some embodiments, the protonatable lipids have a pKa of the protonatable group in the range of about 4 to about 11, e.g., a pKa of about 5 to about 7.

LNPs can comprise two or more (different) cationic lipids. The cationic lipids may be selected to contribute different advantageous properties. E.g., cationic lipids that differ in properties such as amine pKa, chemical stability, half-life in circulation, half-life in tissue, net accumulation in tissue, or toxicity can be used in the LNP. In particular, the cationic lipids can be chosen so that the properties of the mixed-LNP are more desirable than the properties of a single-LNP of individual lipids.

The amount of the permanently cationic lipid or lipidoid may be selected taking the amount of the nucleic acid cargo into account. In one embodiment, these amounts are selected such as to result in an N/P ratio of the nanoparticle(s) or of the composition in the range from about 0.1 to about 20. In this context, the N/P ratio is defined as the mole ratio of the nitrogen atoms ("N") of the basic nitrogen-containing groups of the lipid or lipidoid to the phosphate groups ("P") of the RNA which is used as cargo. The N/P ratio may be calculated on the basis that e.g. 1µg RNA typically contains about 3nmol phosphate residues, provided that the RNA exhibits a statistical distribution of bases. The "N"-value of the lipid or lipidoid may be calculated on the basis of its molecular weight and the relative content of permanently cationic and - if present - cationisable groups.

LNP in vivo characteristics and behavior can be modified by addition of a hydrophilic polymer coating, e.g. polyethylene glycol (PEG), to the LNP surface to confer steric stabilization. Furthermore, LNPs can be used for specific targeting by attaching ligands (e.g. antibodies, peptides, and carbohydrates) to its surface or to the terminal end of the attached PEG chains (e.g. via PEGylated lipids or PEGylated cholesterol).

In some embodiments, the LNPs comprise a polymer conjugated lipid. The term "polymer conjugated lipid" refers to a molecule comprising both a lipid portion and a polymer portion. An example of a polymer conjugated lipid is a PEGylated lipid. The term "PEGylated lipid" refers to a molecule comprising both a lipid portion and a polyethylene glycol portion. PEGylated lipids are known in the art and include 1-(monomethoxy-polyethyleneglycol)-2,3-dimyristoylglycerol (PEG-s- DMG) and the like.

In certain embodiments, the LNP comprises an additional, stabilizing-lipid which is a polyethylene glycol-lipid (PEGylated lipid). Suitable polyethylene glycol-lipids include PEG-modified phosphatidylethanolamine, PEG-modified phosphatidic acid, PEG-modified ceramides (e.g. PEG-CerC14 or PEG-CerC20), PEG-modified dialkylamines, PEG-modified diacylglycerols, PEG-modified dialkylglycerols. Representative polyethylene glycol-lipids include PEG-c-DOMG, PEG-c-DMA, PEG-s-DMG, PEG-DMG, PEG-DSG, PEG-DSPE, PEG-DOMG. In one embodiment, the polyethylene glycol-lipid is N-[(methoxy polyethylene glycol)2000)carbamyl]-1,2-dimyristyloxlpropyl-3-amine (PEG-c-DMA). In a preferred embodiment, the polyethylene glycol-lipid is PEG-2000-DMG. In one embodiment, the polyethylene glycol-lipid is PEG-c-DOMG). In other embodiments, the LNPs comprise a PEGylated diacylglycerol (PEG-DAG) such as 1-(monomethoxy-polyethyleneglycol)-2,3-dimyristoylglycerol (PEG-DMG), a PEGylated phosphatidylethanoloamine (PEG-PE), a PEG succinate diacylglycerol (PEG-S-DAG) such as 4-O-(2',3'-di(tetradecanoyloxy)propyl-1-O-(ω-methoxy(polyethoxy)ethyl)butanedioate (PEG-S-DMG), a PEGylated ceramide (PEG-cer), or a PEG dialkoxypropylcarbamate such as ω-methoxy(polyethoxy)ethyl-N-(2,3di(tetradecanoxy)propyl)carbamate or 2,3-di(tetradecanoxy)propyl-N-(ω-methoxy(polyethoxy)ethyl)carbamate.

In preferred embodiments the at least one coding RNA of the composition for use is complexed with one or more lipids thereby forming LNPs, wherein the LNP additionally comprises a PEGylated lipid that is preferably derived from formula (IV) of published PCT patent application WO2018/078053A1. Accordingly, PEGylated lipid derived from formula (IV) of published PCT patent application WO2018/078053A1, and the respective disclosure relating thereto, is herewith incorporated by reference.

In a particularly preferred embodiments, the at least one coding RNA of the composition for use is complexed with one or more lipids thereby forming LNPs, wherein the LNP additionally comprises a PEGylated lipid, wherein the PEG lipid is preferably derived from formula (IVa) of published PCT patent application WO2018/078053A1. Accordingly, PEGylated lipid derived from formula (IVa) of published PCT patent application WO2018/078053A1, and the respective disclosure relating thereto, is herewith incorporated by reference.

In a particularly preferred embodiment the PEG lipid is of formula (IVa) 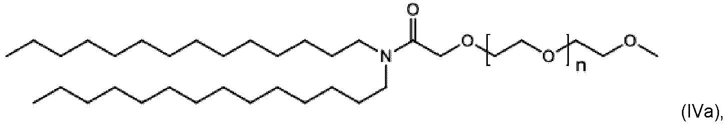 wherein n has a mean value ranging from 30 to 60, such as about 30±2, 32±2, 34±2, 36±2, 38±2, 40±2, 42±2, 44±2, 46±2, 48±2, 50±2, 52±2, 54±2, 56±2, 58±2, or 60±2. In a most preferred embodiment n is about 49.

Further examples of PEG-lipids suitable in that context are provided in US2015/0376115A1 and WO2015/199952, each of which is incorporated by reference in its entirety.

In some embodiments, LNPs include less than about 3, 2, or 1 mole percent of PEG or PEG-modified lipid, based on the total moles of lipid in the LNP. In further embodiments, LNPs comprise from about 0.1% to about 20% of the PEG-modified lipid on a molar basis, e.g., about 0.5 to about 10%, about 0.5 to about 5%, about 10%, about 5%, about 3.5%, about 3%, about 2,5%, about 2%, about 1.5%, about 1%, about 0.5%, or about 0.3% on a molar basis (based on 100% total moles of lipids in the LNP). In preferred embodiments, LNPs comprise from about 1.0% to about 2.0% of the PEG-modified lipid on a molar basis, e.g., about 1.2 to about 1.9%, about 1.2 to about 1.8%, about 1.3 to about 1.8%, about 1.4 to about 1.8%, about 1.5 to about 1.8%, about 1.6 to about 1.8%, in particular about 1.4%, about 1.5%, about 1.6%, about 1.7%, about 1.8%, about 1.9%, most preferably 1.7% (based on 100% total moles of lipids in the LNP). In various embodiments, the molar ratio of the cationic lipid to the PEGylated lipid ranges from about 100:1 to about 25:1.

In preferred embodiments, the LNP additionally comprises one or more additional lipids which stabilize the formation of particles during their formation (e.g. neutral lipid and/or one or more steroid or steroid analogue).

In preferred embodiments, the coding RNA of the composition for use is complexed with one or more lipids thereby forming LNPs, wherein the LNP additionally comprises one or more neutral lipid and/or one or more steroid or steroid analogue.

Suitable stabilizing lipids include neutral lipids and anionic lipids. The term "neutral lipid" refers to any one of a number of lipid species that exist in either an uncharged or neutral zwitterionic form at physiological pH. Representative neutral lipids include diacylphosphatidylcholines, diacylphosphatidylethanolamines, ceramides, sphingomyelins, dihydro sphingomyelins, cephalins, and cerebrosides.

In embodiments, the LNP additionally comprises one or more neutral lipids, wherein the neutral lipid is selected from the group comprising distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE), or mixtures thereof.

In some embodiments, the LNPs comprise a neutral lipid selected from DSPC, DPPC, DMPC, DOPC, POPC, DOPE and SM. In various embodiments, the molar ratio of the cationic lipid to the neutral lipid ranges from about 2:1 to about 8:1. In preferred embodiments, the neutral lipid is 1,2-distearoyl-sn-glycero-3-phosphocholine (DSPC). The molar ratio of the cationic lipid to DSPC may be in the range from about 2:1 to 8:1. In preferred embodiments, the steroid is cholesterol. The molar ratio of the cationic lipid to cholesterol may be in the range from about 2:1 to 1:1. In some embodiments, the cholesterol may be PEGylated.

The sterol can be about 10 mol % to about 60 mol % or about 25 mol % to about 40 mol % of the lipid particle. In one embodiment, the sterol is about 10, 15, 20, 25, 30, 35, 40, 45, 50, 55, or about 60 mol % of the total lipid present in the lipid particle. In another embodiment, the LNPs include from about 5% to about 50% on a molar basis of the sterol, e.g., about 15% to about 45%, about 20% to about 40%, about 48%, about 40%, about 38.5%, about 35%, about 34.4%, about 31.5% or about 31% on a molar basis (based upon 100% total moles of lipid in the LNP).

Preferably, LNPs comprise: (a) at least one coding RNA, (b) a cationic lipid, (c) an aggregation reducing agent or aggregation-reducing lipid (such as polyethylene glycol (PEG) lipid or PEG-modified lipid), (d) optionally a non-cationic lipid (such as a neutral lipid), and (e) optionally, a sterol.

In some embodiments, the cationic lipids (as defined above), non-cationic lipids (as defined above), cholesterol (as defined above), and/or PEG-modified lipids (as defined above) may be combined at various relative molar ratios. E.g., the ratio of cationic lipid to non-cationic lipid to cholesterol-based lipid to PEGylated lipid may be between about 30-60:20-35:20-30:1-15, or at a ratio of about 40:30:25:5, 50:25:20:5, 50:27:20:3, 40:30:20:10, 40:32:20:8, 40:32:25:3 or 40:33:25:2, or at a ratio of about 50:25:20:5, 50:20:25:5, 50:27:20:3 40:30:20: 10,40:30:25:5 or 40:32:20:8, 40:32:25:3 or 40:33:25:2, respectively.

In particularly preferred embodiments, the lipid is lipid compound III-3, the neutral lipid is DSPC, the steroid is cholesterol, and the PEGylated lipid is the compound of formula (IVa).

In a preferred embodiments, wherein the the liposomes, lipid nanoparticles, lipoplexes, and/or nanoliposomes preferably consist of (i) at least one cationic lipid; (ii) at least one neutral lipid; (iii) at least one steroid or steroid analogue; and (iv) at least one aggregation reducing-lipid, wherein, preferably, (i) to (iv) are in a molar ratio of about 20-60% cationic lipid, 5-25% neutral lipid, 25-55% sterol, and 0.5-15% PEG-lipid.

In particularly preferred embodiments, the at least one coding RNA of the composition for use is complexed with one or more lipids thereby forming LNPs, wherein the LNP essentially consists of
(i) at least one cationic lipid as defined herein, preferably lipid III-3;
(ii) a neutral lipid as defined herein, preferably 1,2-distearoyl-sn-glycero-3-phosphocholine (DSPC);
(iii) a steroid or steroid analogue as defined herein, preferably cholesterol; and
(iv) a PEG-lipid as defined herein, e.g. PEG-DMG or PEG-cDMA, preferably a PEGylated lipid of formula (IVa),
wherein (i) to (iv) are in a molar ratio of about 20-60% cationic lipid: 5-25% neutral lipid: 25-55% sterol; 0.5-15% PEG-lipid.

In a particular preferred embodiment, the at least one coding RNA of the composition for use is complexed with one or more lipids thereby forming LNPs, wherein LNPs have a molar ratio of approximately 50:10:38.5:1.5, preferably 47.5:10:40.8:1.7 or more preferably 47.4:10:40.9:1.7 (i.e. proportion (mol%) of cationic lipid (preferably lipid III-3), DSPC, cholesterol and PEG-lipid ((preferably PEG-lipid of formula (IVa) with n = 49); solubilized in ethanol).

The total amount of coding RNA in the LNPs may vary and is defined depending on the e.g. RNA to total lipid w/w ratio. In one embodiment of the invention the RNA to total lipid ratio is less than 0.06 w/w, preferably between 0.03 w/w and 0.04 w/w.

In various embodiments, the LNP as defined herein have a mean diameter of from about 50nm to about 200nm, from about 60nm to about 200nm, from about 70nm to about 200nm, from about 80nm to about 200nm, from about 90nm to about 200nm, from about 90nm to about 190nm, from about 90nm to about 180nm, from about 90nm to about 170nm, from about 90nm to about 160nm, from about 90nm to about 150nm, from about 90nm to about 140nm, from about 90nm to about 130nm, from about 90nm to about 120nm, from about 90nm to about 100nm, from about 70nm to about 90nm, from about 80nm to about 90nm, from about 70nm to about 80nm, or about 30nm, 35nm, 40nm, 45nm, 50nm, 55nm, 60nm, 65nm, 70nm, 75nm, 80nm, 85nm, 90nm, 95nm, 100nm, 105nm, 110nm, 115nm, 120nm, 125nm, 130nm, 135nm, 140nm, 145nm, 150nm, 160nm, 170nm, 180nm, 190nm, or 200nm and are substantially nontoxic. As used herein, the mean diameter may be represented by the z-average as determined by dynamic light scattering as commonly known in the art.

The polydispersity index (PDI) of the nanoparticles is typically in the range of 0.1 to 0.5. In a particular embodiment, a PDI is below 0.2. Typically, the PDI is determined by dynamic light scattering as commonly known in the art.

In another preferred embodiment of the invention the lipid nanoparticles have a hydrodynamic diameter in the range from about 50nm to about 300nm, or from about 60nm to about 250nm, from about 60nm to about 150nm, or from about 60nm to about 120nm, respectively.

According to various suitable embodiments, suitable carriers for SCS delivery may include polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semisynthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic poly conjugates).

In other embodiments, the at least one coding RNA of the composition for use may be formulated in amphiphilic macromolecules (AMs). AMs comprise biocompatible amphiphilic polymers which have an alkylated sugar backbone covalently linked to polyethylene glycol). In aqueous solution, the AMs self-assemble to form micelles. Non-limiting examples of methods of forming AMs and AMs are described in US Patent Publication No. US20130217753, the contents of which are herein incorporated by reference in its entirety.

In other embodiments, the at least one coding RNA of the composition for use may be formulated in inorganic nanoparticles (U.S. Pat. No. 8,257,745, herein incorporated by reference in its entirety). The inorganic nanoparticles may include, but are not limited to, clay substances that are water swellable. As a non-limiting example, the inorganic nanoparticle may include synthetic smectite clays which are made from simple silicates (See e.g., U.S. Pat. No. 5,585,108 and 8,257,745 each of which are herein incorporated by reference in their entirety).

In other embodiments, the at least one coding RNA of the composition for use may be formulated in water-dispersible nanoparticle comprising a semiconductive or metallic material (U.S. Pub. No. 20120228565; herein incorporated by reference in its entirety) or formed in a magnetic nanoparticle (U.S. Pub. No. 20120265001 and 20120283503; each of which is herein incorporated by reference in its entirety). The water-dispersible nanoparticles may be hydrophobic nanoparticles or hydrophilic nanoparticles.

In other embodiments, the at least one coding RNA of the composition for use may be formulated in high density lipoprotein-nucleic acid particles. As a non-limiting example, the particles may comprise a nucleic acid component and a polypeptide comprising a positively charged region which associates with the nucleic acid component as described in US Patent No. 8,734,853, the contents of which is herein incorporated by reference in its entirety.

In other embodiments, the at least one coding RNA of the composition for use may be formulated in a micelle or coated on a micelle for delivery, or may be encapsulated into any hydrogel known in the art which may form a gel when injected into a subject, or may be formulated in and/or delivered using a nanolipogel.

In other embodiments, the at least one coding RNA of the composition for use may be formulated in exosomes. The exosomes may be loaded with at least one polynucleotide and delivered to cells, tissues and/or organisms. As a non-limiting example, the RNA may be loaded in the exosomes described in International Publication No. WO2013084000, herein incorporated by reference in its entirety. In embodiments, the exosome are obtained from cells that have been induced to undergo oxidative stress such as, but not limited to, the exosomes described in International Patent Publication No. WO2014028763, the contents of which are herein incorporated by reference in its entirety.

Accordingly, in preferred embodiments, the at least one coding RNA of the composition for use is complexed or associated with or at least partially complexed or partially associated with one or more exosomes.

Accordingly, the pharmaceutically acceptable carrier as used herein preferably includes the liquid or non-liquid basis of the inventive composition. If the inventive composition is provided in liquid form, the carrier will be water, typically pyrogen-free water; isotonic saline or buffered (aqueous) solutions, e.g. phosphate, citrate etc. buffered solutions. Preferably, Ringer- or Ringer-Lactate solution as described in WO2006/122828 is used as a liquid basis for the composition for use according to the invention.

Accordingly, in preferred embodiments, the composition for use is administered in Ringer- or Ringer-Lactate solution. In embodiments, the composition for use may be provided in lyophilized or dried form (using e.g. lyophilisation or drying methods as described in WO2016/165831, WO2011/069586, WO2016/184575 or WO2016/184576). Preferably, the lyophilized or dried composition is reconstituted in a suitable buffer, advantageously based on an aqueous carrier, prior to SCS administration, e.g. Ringer- or Ringer-Lactate solution or a phosphate buffer solution.

In preferred embodiments, the composition for use as specified herein is administered into the SCS, wherein administration into the SCS may be performed by an injection needle, a microneedle, an injection device, a catheter, an implant delivery device, or a microcannula to a subject in need of treatment. Preferably, administration into the SCS is performed by a microinjection device.

Preferably, the injection needle or microneedle has a length that does not exceed about 2000µm and a diameter that does not exceed about 600µm.

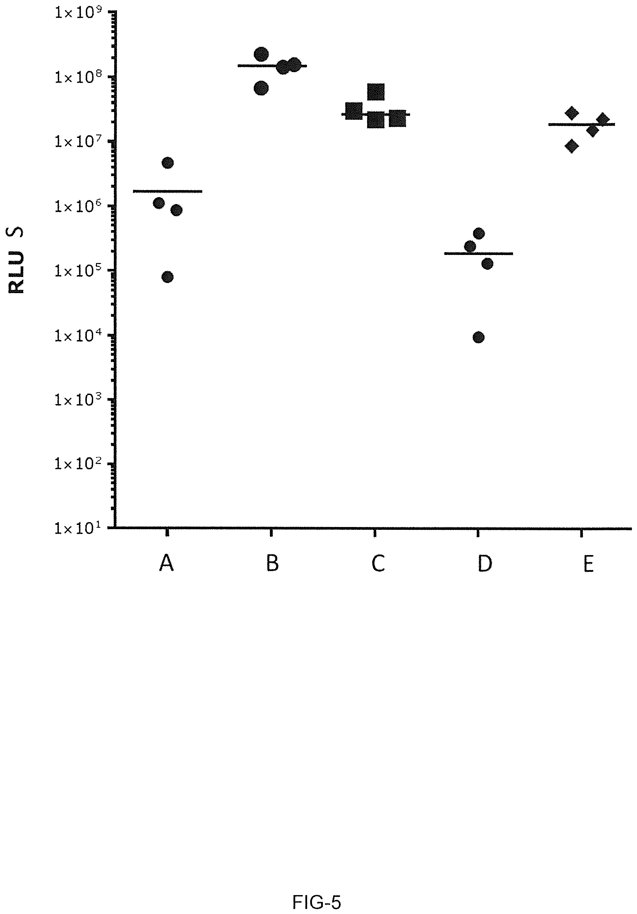

A suitable injection device for SCS administration is an SCS microinjection device. Such a microinjection device is for example described in published PCT patent application WO2014/074823, the whole disclosure incorporated herein by reference. In particular, the features of Claims 1 to 32 of WO2014/074823, as well as the disclosure relating thereto, are particularly suitable in the context of the invention, and are herein incorporated by reference. Another suitable microinjection device for SCS delivery is described in published PCT patent application WO2017/192565, the whole disclosure incorporated herein by reference. In particular, the features of Claims 1 to 44 of WO2017/192565, or the device as shown in Figures 5 to 41 of WO2017/192565, as well as the specific disclosure relating to these claims and Figures, are particularly suitable in the context of the invention, and are herein incorporated by reference.

Further suitable delivery devices for SCS administration are described in published PCT patent application WO2014151070A1, the whole disclosure incorporated herein by reference. In particular, the features of Claims 1 to 24 of WO2014151070A1, as well as the disclosure relating thereto, are particularly suitable in the context of the invention, and are herein incorporated by reference. A further suitable delivery device for SCS administration are described in published PCT patent application WO2017158365, the whole disclosure incorporated herein by reference. In particular, the features of Claims 1-38 of WO2017158365, as well as the disclosure relating thereto, are particularly suitable in the context of the invention, and are herein incorporated by reference. A further suitable delivery device for SCS administration are described in published PCT patent application WO2017046358, the whole disclosure incorporated herein by reference. In particular, the features of Claims 1-16 of WO2017046358, as well as the disclosure relating thereto, are particularly suitable in the context of the invention, and are herein incorporated by reference. A further suitable delivery device for SCS administration are described in published PCT patent application WO2016042162, the whole disclosure incorporated herein by reference. In particular, the features of Claims 1 to 29 of WO2016042162, as well as the disclosure relating thereto, are particularly suitable in the context of the invention, and are herein incorporated by reference.

In a preferred embodiment, the effective amount of the composition for use when administered to the SCS as described above provides higher efficacy or expression or a greater therapeutic effect compared to the identical amount of coding RNA when administered via state of the art methods including intravitreally, intracamerally, topically, subretinally, parenterally, or orally.

Methods to evaluate the expression of the coding RNA in the eye or in specific organs/tissues of the eye, and methods to determine the therapeutic effect (neovascularization, subretinal exudation, inflammation etc.) of a treatment are well known in the art for the skilled artisan. In embodiments, the intraocular pressure of the eye remains substantially constant during administration of the composition to the SCS. In another embodiment, administration of the composition as defined herein to the SCS results in a decreased number of side effects, or a reduced severity of one or more side effects, compared to administration of the same composition intravitreally or subretinally.

### Kit or kit of parts

In a **second aspect**, the present invention provides a kit or kit of parts, preferably a kit or kit of parts **for use** in treatment or prevention of an ophthalmic disease, disorder or condition, wherein said kit or kit of parts is administered into the SCS to a subject in need of treatment.

Notably, embodiments relating to the first aspect of the invention are likewise applicable to embodiments of the second aspect of the invention, and embodiments relating to the second aspect of the invention are likewise applicable to embodiments of the first aspect of the invention.

The kit or kit of parts for use may comprise at least one coding RNA as defined in the context of the first aspect, and/or the composition of the first aspect, and optionally, a liquid vehicle for solubilising, and optionally technical instructions providing information on administration and dosage of the components. Embodiments and features disclosed in the context of coding RNA suitable for ocular delivery or embodiments and features disclosed in the context of the composition for use (e.g., LNP formulation, delivery devices) are likewise applicable for the RNA and/or the composition of the kit or the kit of parts.

The kit or kit of parts for use may further comprise additional components as described in the context of the composition, in particular, pharmaceutically acceptable carriers, excipients, buffers and the like.

The kit or kit of parts for use may further comprise an anti-inflammatory drug, a vascular endothelial growth factor (VEGF) modulator, a platelet derived growth factor (PDGF) modulator, an angiogenesis inhibitor, an immunosuppressive agent, a vascular permeability inhibitor, or a combination thereof, to the SCS of the patient in need of treatment. Further, the kit or kit of parts for use may comprise modulators and/or activators of any of the provided protein targets of **List 1**. In other embodiments, the kit or kit of parts for use may comprise at least one of the agents provided in paragraph [1263] to [1276] of published PCT application WO2017/192565.

The technical instructions of said kit or kit of parts for use may comprise information about administration and dosage and patient groups. Such kits, preferably kits of parts, may be applied e.g. for any of the applications or medical uses mentioned herein.

Preferably, the individual components of the kit or kit of parts for use may be provided in lyophilised form. The kit may further contain as a part a vehicle (e.g. pharmaceutically acceptable buffer solution) for solubilising the coding RNA or the composition of the first aspect.

In preferred embodiments, the kit or kit of parts for use comprises Ringer- or Ringer lactate solution.

In preferred embodiments, the kit or kit of parts for may comprise an injection needle suitable for SCS delivery, a microneedle suitable for SCS delivery, an injection device suitable for SCS delivery, a catheter suitable for SCS delivery, an implant delivery device suitable for SCS delivery, or a microcannula suitable for SCS delivery.

Any of the above kits may be used in applications or medical uses as defined in the context of the invention.

### Methods of treatment and delivery:

In a **third aspect**, the present invention relates to a method of treating or preventing an ophthalmic disease, disorder, or condition.

Notably, embodiments relating to the first or second aspect of the invention are likewise applicable to embodiments of the third aspect, and embodiments relating to the third aspect of the invention are likewise applicable to embodiments relating to the first or second aspect of the invention.

In preferred embodiments of the third aspect, the method comprises a step of applying or administering to a subject the composition of the first aspect, or the kit or kit of parts of the second aspect, wherein said composition or kit or kit of parts is administered into the suprachoroidal space (SCS).

In preferred embodiments, the coding RNA of the composition and/or the encoded peptide (provided by the coding RNA) is substantially localized to (therapeutically relevant) cells and/or tissues, preferably to (therapeutically relevant) cells and/or tissues of the posterior segment of the eye upon SCS administration.

In particular, such the method may comprise the steps of:
a) providing the composition of the first aspect, or the kit or kit of parts of the second aspect;
b) administering said composition or kit or kit of parts to the SCS;
c) optionally, administering a further substance (e.g. an anti-inflammatory drug, a vascular endothelial growth factor modulator, a platelet derived growth factor modulator, an angiogenesis inhibitor, an immunosuppressive agent, a vascular permeability inhibitor, or a combination thereof), preferably to the SCS or intravitreally;
d) optionally, administering a further coding RNA and/or guideRNA, preferably to the SCS or intravitreally.

In a preferred embodiment of the third aspect, the invention relates to a method for gene therapy of an ophthalmic disease, disorder, or condition.

In preferred embodiments, the method for gene therapy comprises a step of applying or administering to a subject a composition of the first aspect, or the kit or kit of parts of the second aspect, wherein said composition or kit or kit of parts is administered into the suprachoroidal space (SCS), wherein said composition or kit or kit of parts comprises a coding RNA encoding at least one CRISP-associated endonuclease and at least one guide RNA.

In preferred embodiments, the coding RNA encoding at least one CRISP-associated endonuclease and/or the CRISP-associated endonuclease is substantially localized to (therapeutically relevant) cells and/or tissues, preferably to (therapeutically relevant) cells and/or tissues of the posterior segment of the eye upon SCS administration.

In particular, such the method for gene therapy may comprise the steps of:
a) providing a composition, or the kit or kit of parts, comprising at least one coding RNA encoding at least one CRISP-associated endonuclease and, optionally, at least one guide RNA;
b) applying or administering said composition, or kit or kit of parts to the eye of a subject in need of gene therapy, wherein applying or administering is performed by administering to the SCS;
c) optionally, administering a further substance (e.g. an anti-inflammatory drug, a vascular endothelial growth factor modulator, a platelet derived growth factor modulator, an angiogenesis inhibitor, an immunosuppressive agent, a vascular permeability inhibitor, or a combination thereof), *preferably* to the SCS or intravitreally;
d) optionally, administering a further coding RNA and/or guideRNA, *preferably* to the SCS or intravitreally,

"Gene therapy" preferably involves modulating (i.e. restoring, enhancing, decreasing or inhibiting) gene expression in a subject in order to achieve a therapeutic effect. To this end, gene therapy typically encompasses the introduction of nucleic acids into cells. The term generally refers to the manipulation of a genome for therapeutic purposes and includes the use of genome-editing technologies (e.g. CRISPR/Cas) for correction of mutations that cause disease, the addition of therapeutic genes to the genome, the removal of deleterious genes or genome sequences, and the modulation of gene expression. Gene therapy may involve *in vivo* or *ex vivo* transformation of the host cells.

In a **fourth aspect**, the present invention relates to a method of delivering a coding RNA molecule to cells and/or tissues of the eye of a subject, said method comprising the steps of providing a composition comprising said coding RNA *preferably* as defined herein, or a composition, *preferably* as defined herein, or kit or kit of parts *preferably* as defined herein, and delivering said coding RNA, composition, or kit or kit of parts to cells and/or tissues of the eye of the subject, wherein delivering is performed by administering said composition to the SCS.

Notably, embodiments relating to the first, second, or third aspect of the invention are likewise applicable to embodiments of the fourth aspect, and embodiments relating to the fourth aspect of the invention are likewise applicable to embodiments relating to the first, second, or third aspect of the invention.

In preferred embodiments of the fourth aspect, delivering via SCS leads to expression, that is translation, of the encoded protein in cells and/or tissues of the eye, *preferably* in therapeutically relevant cells or tissues of the eye (as defined above).

In preferred embodiments of the third and fourth aspect, SCS administration is preferably performed in a non-surgical manner, e.g. via an injection needle, a microneedle, an injection device, a microinjection device, a catheter, an implant delivery device, or a microcannula to a subject, preferably to a subject in need of treatment.

In preferred embodiments of the third and fourth aspect, the administering step comprises inserting a hollow microneedle into the sclera at an insertion site, the microneedle having a tip end with an opening, and infusing the composition as defined in the context of the first aspect into the SCS through the inserted microneedle.

Suitably, the microneedle has a length suitable for SCS delivery, e.g. of from about 500µm to about 1500µm.

Suitably, the microneedle has a diameter suitable for SCS delivery, e.g. of from about 200µm to about 600µm.

In preferred embodiments of the third and fourth aspect, the microneedle is inserted into the sclera without penetrating through the sclera and/or the microneedle is inserted into the sclera without penetrating through the choroid, thereby administering the composition comprising coding RNA into the SCS.

In preferred embodiments of the third and fourth aspect, the microneedle is inserted into the surface of the sclera at an angle of from about 70 degrees to about 110 degrees, wherein, preferably, the microneedle is inserted into the surface of the sclera at an angle of about 90 degrees.

The methods outlined in the context of the third and fourth aspect may be applied for laboratory, for research, for diagnostic, for commercial purposes and/or for therapeutic purposes. Preferably, the methods may be carried out in the context of the treatment or prevention of ophthalmic diseases, disorders or conditions as defined herein.

### Brief description of lists and tables

**List 1:** Suitable therapeutic proteins provided by the coding RNA of the invention
**Table 1:** Suitable therapeutic proteins and sequences provided by the coding RNA of the composition
**Table 2:** Human codon usage table with frequencies indicated for each amino acid
**Table 3:** SCS administration of coding RNA compositions, experimental setup1
**Table 4:** Suprachoroidal administration of coding RNA compositions, experimental setup 2
**Table 5:** Suprachoroidal administration of coding RNA compositions (Example 3)

### Brief description of the drawings

- **Figure 1**: cross-sectional view of an illustration of the human eye, comprising anterior region or segment 12, posterior region or segment 14, cornea 16, lens 18, sclera 20, anterior chamber 22, iris 24, cornea 16, posterior chamber 26, limbus 38, conjunctiva 45, choroid 28, retina 27, retinachoroidal tissue (choroid 28, retina 27), vitreous 30, ciliary body 32, ora serrata 34, suprachoroidal space (SCS) 36. Further explanations are provided in the description.
- **Figure 2**: shows a cross-sectional view of a portion of the human eye, illustrating the suprachoroidal space 36, Sclera 20, Tenon's Capsule 46, conjunctiva 45, choroid 28, and retina 27. As indicated, the sclera 20 has a typical thickness between about 500µm and 700µm. Further explanations are provided in the description.

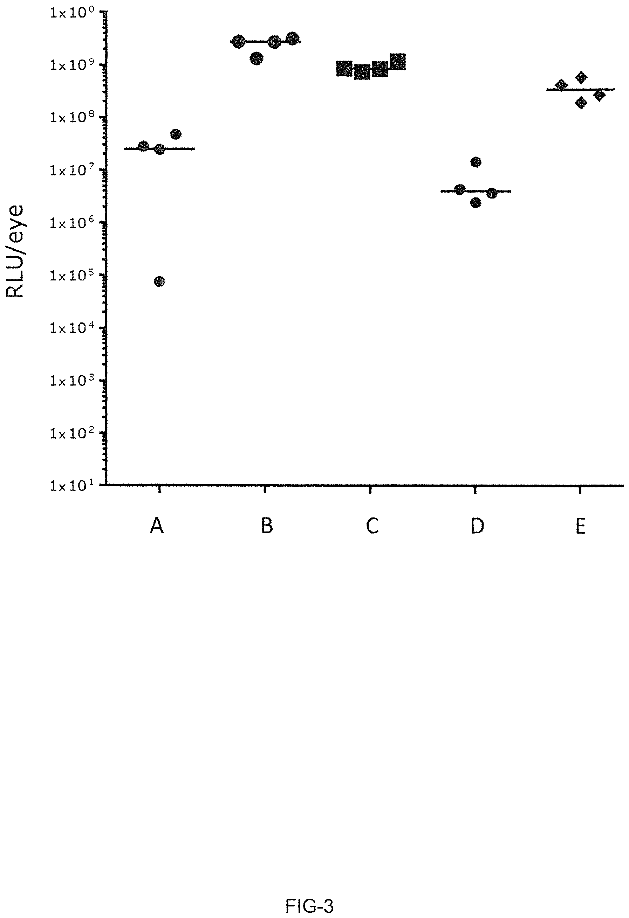

- **Figure 3**: shows the transfection efficiency analysis 24h post SCS injection. Shown are luminescence values, expressed as relative light units (RLU), for pooled lysate samples derived from different sections of the eye. Values represent the luciferase levels in the whole eye (see experimental setup 1, Table 3, **Example 2**).

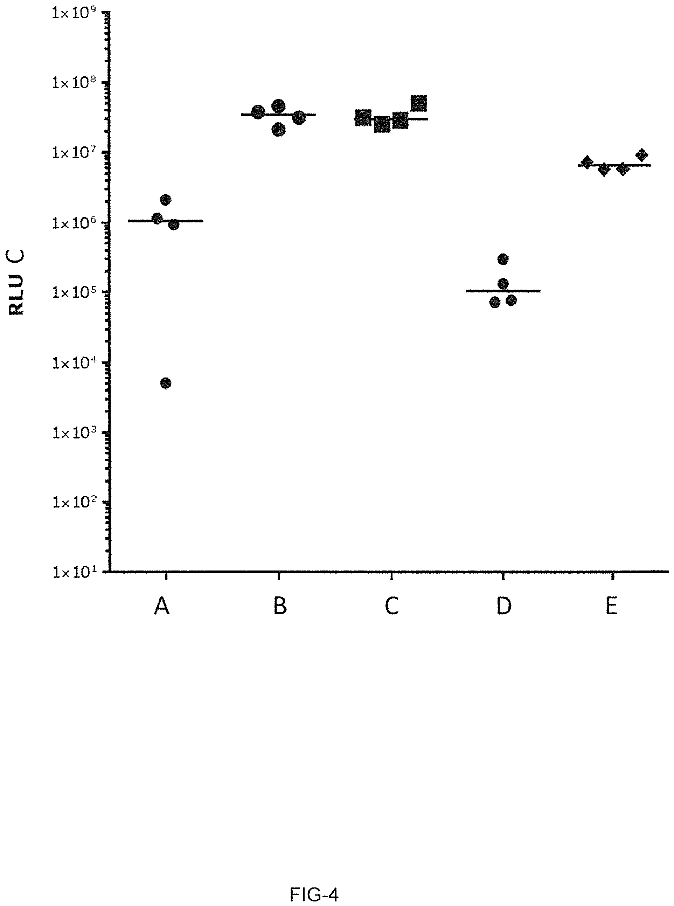

- **Figure 4**: shows the transfection efficiency analysis 24h post SCS injection. Shown are luminescence values, expressed as relative light units (RLU), for cell lysates (C=cells) (see experimental setup 1, Table 3, **Example 2**).
- **Figure 5**: shows the transfection efficiency analysis 24h post SCS injection. Shown are luminescence values, expressed as relative light units (RLU), for sclera lysates (S=sclera) (see experimental setup 1, Table 3, **Example 2**).

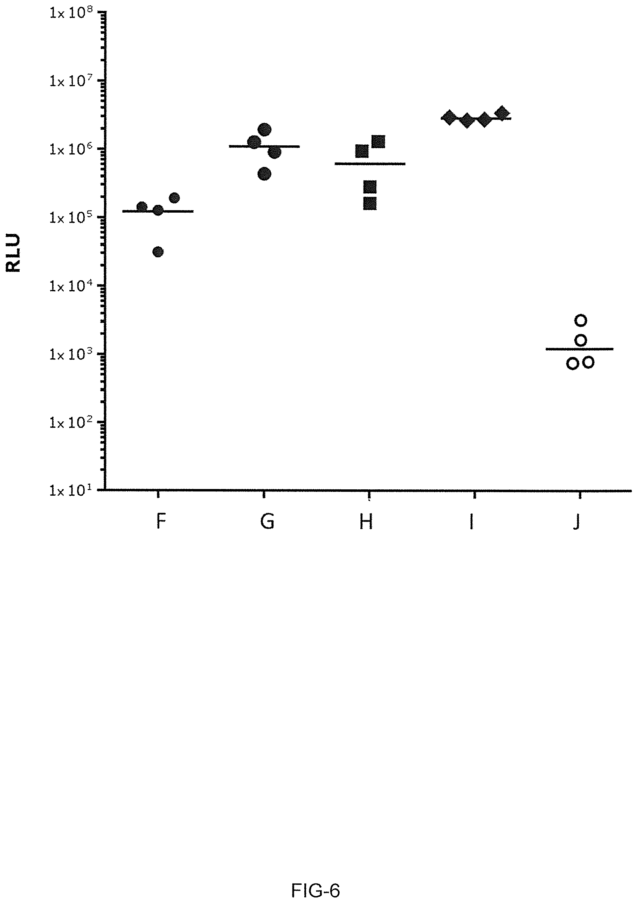

- **Figure 6**: shows the transfection efficiency analysis 72h post SCS injection. Shown are luminescence values, expressed as relative light units (RLU), for pooled lysate samples deriving from different sections of the eye. Values represent the luciferase levels in the whole eye (see experimental setup 2, Table 4, **Example 2**).

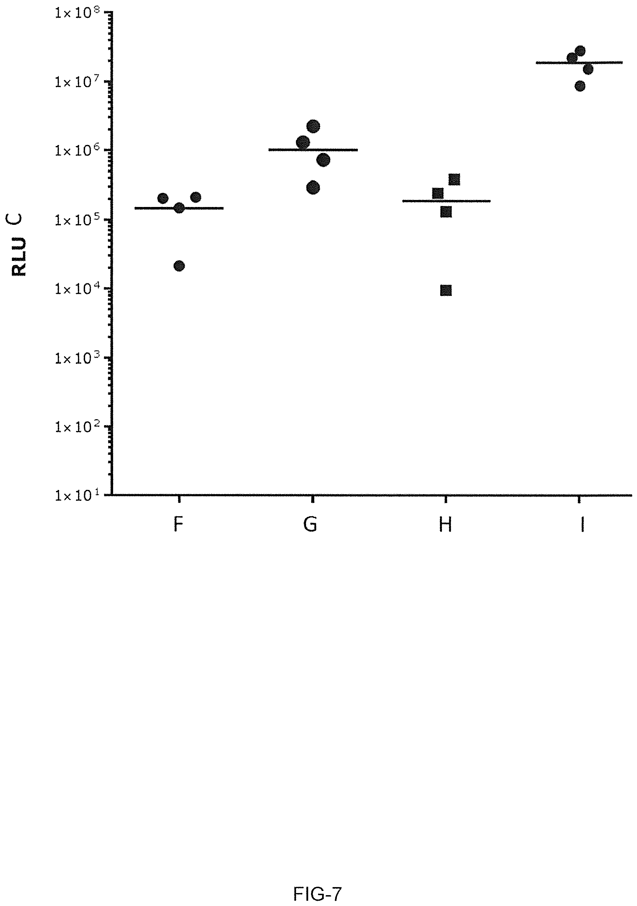

- **Figure 7**: shows the transfection efficiency analysis 72h post SCS injection. Shown are luminescence values, expressed as relative light units (RLU), for cell lysates (C=cells) (see experimental setup 2, Table 4, **Example 2**).

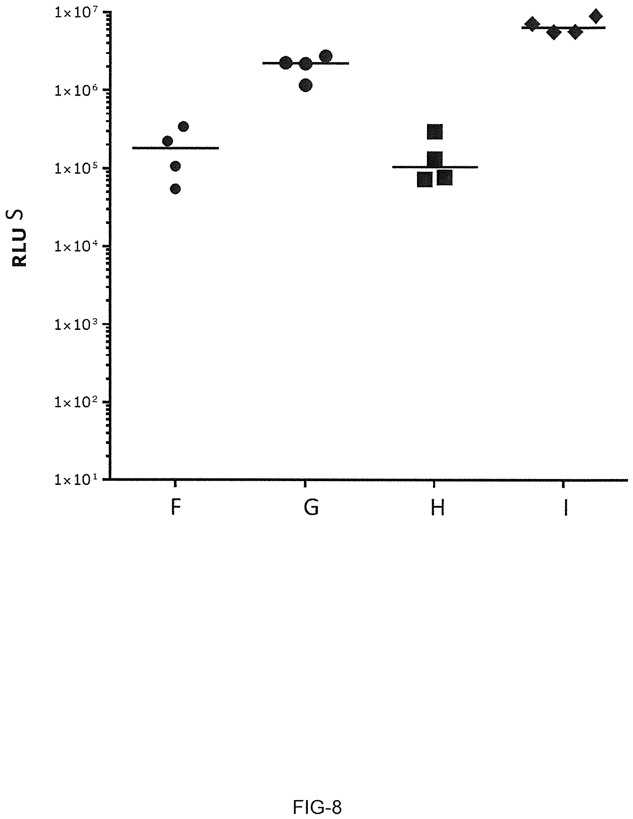

- **Figure 8**: shows the results of the expression analysis 72h post SCS injection. Shown are luminescence values, expressed as relative light units (RLU), for sclera lysates (S=sclera) (see experimental setup 2, Table 4, **Example 2**).

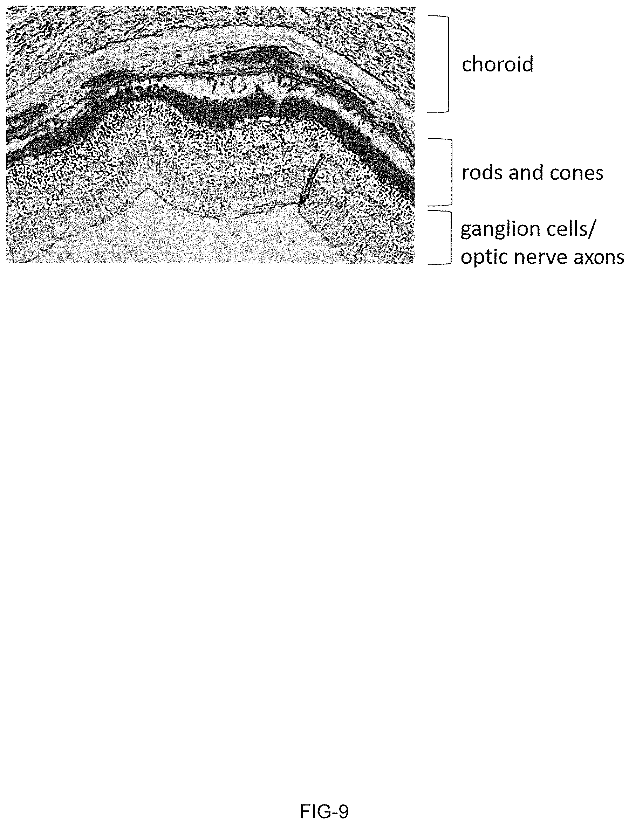

- **Figure 9**: shows the results of the expression analysis 72h post SCS injection. Microscopy image of a tissue sample of the eye (A). SCS delivery of 50µg coding RNA led to a strong expression of the reporter protein in photoreceptor cells (fluorescent signal in black). For experimental details, see **Example 3**.

### Examples

The following examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1: Preparation compositions for in vivo experiments

The present Example provides methods of obtaining the coding RNA as well as methods of generating a composition of the invention.

### 1.1. Preparation of DNA and RNA constructs:

A DNA sequence encoding luciferase was prepared and used for subsequent RNA *in vitro* transcription. Said DNA sequence was prepared by modifying the wild type cds sequences by introducing a GC optimized. Sequences were introduced into a plasmid vector to comprising UTR sequences, a stretch of adenosines, a histone-stem-loop structure, and a stretch of 30 cytosines. Obtained plasmid DNA was transformed and propagated in bacteria using common protocols and plasmid DNA was extracted, purified, and used for subsequent RNA *in vitro* transcription.

### 1.2. RNA in vitro transcription from plasmid DNA templates:

DNA plasmids were enzymatically linearized using EcoRI and used for DNA dependent RNA *in vitro* transcription using T7 RNA polymerase in the presence of a nucleotide mixture (ATP/GTP/CTP/UTP) and cap analog (e.g., m7GpppG) under suitable buffer conditions. The obtained coding RNA constructs were purified using RP-HPLC (PureMessenger^{®}, CureVac AG, Tübingen, Germany; according to WO2008/077592). The obtained m7G capped coding RNA was additionally methylated using Cap specific 2'-O-methyltransferase enzyme, and enzymatically polyadenylated using a commercial Polyadenylation kit (coding RNA: **SEQ ID NO: 1**)

### 1.3. Preparation of a composition comprising LNP formulated coding RNA:

Lipid nanoparticles (LNP) were prepared according to the general procedures described in WO2015/199952, WO2017/004143 and WO2017/075531. LNP formulated coding RNA was prepared using an ionizable amino lipid (cationic lipid), phospholipid, cholesterol and a PEGylated lipid. Briefly, lipid compounds were solubilized in ethanol at a molar ratio (%) of approximately 47.4:10:40.9:1.7 (DC-cholesterol:DSPC:cholesterol:PE-PEG). RNA was diluted to 0.05mg/mL in 10mM to 50mM citrate buffer, pH4. Syringe pumps were used to mix the ethanolic lipid solution with the RNA aqueous solution at a ratio of about 1:3 (vol/vol) with total flow rates above 15ml/min. The ethanol was then removed and the external buffer replaced with a PBS buffer by dialysis. Finally, the lipid nanoparticles were filtered through a 0.2µm pore sterile filter and the LNP-formulated RNA composition was adjusted to about 1mg/ml total RNA. The LNP solution was stored in the presence of trehalose at -80°C till used. The obtained LNP-formulated RNA composition (1mg/ml total RNA) was diluted to 0.25µg/µl RNA target concentration using PBS before *in vivo* application.

### 1.4. Preparation of a composition comprising Liposome formulated coding RNA:

Liposomes were prepared using a 50:100:2.5 ratio of DC-cholesterol, DOPE, and PEG-PE using methods as described in paragraph 1.3. Liposomes were dried over night, re-hydrated, and pre-formed Liposomes were mixed with the coding RNA. A composition comprising liposome-formulated coding RNA was used form in vivo experiments.

### 1.5. Preparation of a composition comprising polymer-lipidoid complexed coding RNA:

Coding RNA constructs were complexed with a polymer-lipidoid prior to use *in vivo.* 20mg peptide (CGHHHHHRRRRHHHHHGC-NH2; **SEQ ID NO: 437**) TFA salt was dissolved in 2mL borate buffer pH 8.5 and stirred at room temperature for approx. 18h. Then, 12.6mg PEG-SH 5000 (Sunbright) dissolved in N-methylpyrrolidone was added to the peptide solution and filled up to 3mL with borate buffer pH 8.5. After 18h incubation at room temperature, the reaction mixture was purified and concentrated by centricon procedure (MWCO 10kDa), washed against water, and lyophilized. The obtained lyophilisate is dissolved in ELGA water and the concentration of the polymer was adjusted to 10mg/mL. The obtained polyethylene glycol/peptide polymers (HO-PEG 5000-S-(S-CHHHHHHRRRRHHHHHHC-S-)7-S-PEG 5000-OH) were used for further formulation. Ringer lactate buffer (RiLa; alternatively e.g. saline (NaCl) or PBS buffer may be used), respective amounts of lipidoid ("lipidoid 1" or "lipidoid 2"), and respective amounts of said polymer were mixed to prepare compositions comprising a lipidoid and a peptide or polymer. Then, the polymer-lipidoid carrier compositions were used to assemble nanoparticles with the coding RNA by mixing the coding RNA with respective amounts of polymer-lipidoid carrier by incubation of 10min at RT. Size measurements were performed to evaluate whether the obtained nanoparticles have a uniform size profile.

### Example 2: Suprachoroidal injection of compositions comprising coding RNA

The present example shows that compositions comprising coding RNA were successfully delivered via ocular delivery into the suprachoroidal space (SCS), and that the encoded Protein, provided by the coding RNA of the composition, is strongly expressed in various ocular tissues.

### 2.1. Experimental setup:

In a first experiment, New Zeeland white rabbits were injected by suprachoroidal injection via an injection needle into the eye (2 eyes per rabbit), using different compositions comprising coding RNA (**SEQ ID NO: 1**; compositions generated as described in **Example 1**) at day 0, analysis was performed 24h post SCS injection (see section 2.2). Experimental setup is provided in **Table 3.**

In a second experiment, New Zeeland white rabbits were injected by suprachoroidal injection via an injection needle into the eye (2 eyes per rabbit), using different compositions comprising coding RNA (**SEQ ID NO: 1**; compositions generated as described in **Example 1**) at day 0, analysis was performed 72h post SCS injection (see section 2.2). In addition, a control group received NaCl (without coding RNA) to exclude any background. Experimental setup is provided in **Table 4.**

**Table 3: Suprachoroidal administration of coding RNA compositions, experimental setup 1**

| | **Rabbit** | **Formulation** | **administration route** | **volume** | **µg RNA** |
|---|---|---|---|---|---|
| A | 1, 2 | RiLa | SCS, left and right eye | 100µl | 25 |
| B | 3, 4 | lipidoid 1 | SCS, left and right eye | 100µl | 25 |
| C | 5, 6 | lipidoid 2 | SCS, left and right eye | 100µl | 25 |
| D | 7, 8 | LNP (see 1.3) | SCS, left and right eye | 100µl | 25 |
| E | 9, 10 | Liposomes (see 1.4) | SCS, left and right eye | 100µl | 25 |

**Table 4: Suprachoroidal administration of coding RNA compositions, experimental setup 2**

| | **Rabbit** | **Formulation** | **B** | **volume per injection** | **µg RNA** |
|---|---|---|---|---|---|
| F | 1, 2 | RiLa | SCS, left and right eye | 100µl | 25 |
| G | 3, 4 | lipidoid 1 | SCS, left and right eye | 100µl | 25 |
| H | 5, 6 | lipidoid 2 | SCS, left and right eye | 100µl | 25 |
| I | 7, 8 | LNP (see 1.3) | SCS, left and right eye | 100µl | 25 |
| J | 9, 10 | NaCl | SCS, left and right eye | 100µl | 0 |

### 2.1. Expression analysis:

24h post injection (experimental setup 1) and 72h post injection (experimental setup 2), eyes were dissected and subsequently used for transfection efficiency analysis using a standard luciferase assay.

Luciferase assay: Eyes were cut through the pupil and vitreous was removed. After cutting the rest in two pieces, cell material (retinal cells etc.) loosely attached was scraped out of sclera with a scalpel (C= cells). 500µl lysis buffer (1mM PMSF and 2mM DTT) was added to each fraction. The solid eye tissues, mainly containing sclera and cell strongly attached to it, were placed in another 500µl of lysis buffer (S= sclera). After adding a steel bead, samples were placed in a commercially available tissue lyser. Samples were lysed at full speed for 6min. Lysed samples were centrifuged at 13500rpm at 4°C for 10min. Collected supernatant were transferred to a new vial. 50µl of each of the individual fractions were placed in duplicate in a 96-well plate for luciferase measurement. To estimate total levels of luciferase activity per eye 20µl of lysates deriving from different eye sections were pooled together. The values of relative light units deriving from these samples were multiplied by 33.3. Luciferase measurement: PpLuc measurement was performed with 20µl of each fraction/well (sample or standard) using common protocols and reagents. In short, the luminescence assay was performed in a TriStar2S LB942 plate reader, using freshly prepared luciferase substrate (Promega), and 20sec counting time. The results of the Luciferase measurement for individual tissue samples (C= cells, S= sclera), and total eye (pooled samples) are shown in **Figures 3-5** (experimental setup 1), and **Figures 6-8** (experimental setup 1)

### Results:

As shown in **Figures 3-8**, all tested compositions comprising coding RNA that were injected into the SCS led to a surprisingly strong and durable expression of the encoded luciferase protein in ocular tissues. In particular, strong expression was detected in sclera ("S") and cell samples ("C"). The data demonstrates, for the first time, that coding RNA is delivered via the SCS to reach ocular tissues, leading to expression of the protein in different cell types of the eye (e.g. retinal cells). That finding opens as yet unexpected treatment options for RNA therapeutics, and that SCS delivery can be used to address ocular diseases associated with diseases or disorders of the posterior segment of the eye.

### Example 3: Suprachoroidal injection of coding RNA results in strong expression in photoreceptor cells

The present example shows that a composition comprising coding RNA was successfully delivered via ocular delivery into the suprachoroidal space (SCS), and that the encoded protein, provided by the coding RNA of the composition, was strongly expressed in therapeutically relevant cell types, such as retinal cells, e.g. photoreceptors (rods, cones). The data demonstrates for the first time that SCS delivery of a (long) coding RNA results in expression of the encoded protein in therapeutically relevant cell types.

### 3.1. Experimental setup:

New Zeeland white rabbits were injected with coding RNA by suprachoroidal injection via an injection needle into the eye, using compositions comprising coding RNA, encoding a fluorescent reporter (construct **SEQ ID NO: 438**; compositions generated as described in **Example 1**). Tissue samples of the SCS injected eyes were taken and analyzed for spacial (cellular) expression of the reporter construct using fluorescence microscopy. An exemplary microscopy image such a sample is shown in **Figure 9**.

### Results:

As shown in **Figures 9**, SCS delivery of a coding RNA led to strong expression of the protein in therapeutically relevant cell types, including photoreceptor cells (fluorescent signal in photoreceptor cells in black).

The data demonstrates, for the first time, that coding RNA delivered via the SCS to reaches therapeutically relevant cell types, and that those cell types express the encoded protein. That finding opens as yet unexpected treatment options for RNA therapeutics, and that SCS delivery is suitable for addressing ocular diseases associated with diseases or disorders of the posterior segment of the eye. These unexpected results demonstrate that various ocular diseases associated with photoreceptor cells can now be addressed using SCS delivery of RNA therapeutics.

### Items

**1.** A composition comprising at least one coding RNA, wherein said at least one coding RNA comprises at least one coding sequence encoding at least one peptide or protein **for use in** treatment or prevention of an ophthalmic disease, disorder or condition, wherein said composition is administered into the suprachoroidal space (SCS) to a subject in need thereof.
**2.** Composition for use of **item 1**, wherein administration into the SCS results in expression and/or activity of the encoded peptide or protein in cells and/or tissues of the eye, preferably in cells and/or tissues located in the posterior segment of the eye.
**3.** Composition for use of **item 2**, wherein the cells and/or tissues of the eye are selected from sclera, choroid, retina, optic nerve, macula, scleral cells, choroid plexus epithelial cells, retinal cells, retinal pigment epithelium, bruch's membrane, and retinal blood vessels.
**4.** Composition for use of **item 2 or 3**, wherein the cells from retina are selected from photoreceptor cells, bipolar cells, ganglion cells, horizontal cells, and amacrine cells.
**5.** Composition for use of **any one of the preceding items**, wherein administered into the SCS leads to translation of the encoded peptide or protein, thereby exerting a therapeutic effect.
**6.** Composition for use of **item 5**, wherein the therapeutic effect is selected from reduced neovascularization, reduced inflammation, neuroprotection, complement inhibition, reduced drusen formation, reduced scar formation, and/or reduction in choriocapillaris.
**7.** Composition for use of **any one of the preceding items**, wherein the ophthalmic disease, disorder or condition is selected from neovascularization, retinal degenerative disease, diabetic eye disease, retinal detachment, optic nerve disease, endocrine disorders, cancer disease, infectious disease, parasitic disease, pigmentary uveitis, branch retinal vein occlusion, central retinal vein occlusion, macular edema, cystoid macular edema, uveitic macular edema, cytomegalovirus retinitis, endophthalmitis, scleritis, choriotetinitis, dry eye syndrome, Norris disease, Coat's disease, persistent hyperplastic primary vitreous, familial exudative vitreoretinopathy, Leber congenital amaurosis, X-linked retinoschisis, Leber's hereditary optic neurophathy, uveitis, refraction and accommodation disorders, keratoconus, amblyopia, conjunctivitis, corneal ulcers, dacryocystitis, Duane retraction syndrome, optic neuritis, ocular inflammation, glaucoma, macular degeneration, and uveitis, or any disease, disorder or condition related or associated thereto.
**8.** Composition for use of **any one of the preceding items**, wherein the at least one peptide or protein is or is derived from a therapeutic peptide or protein.
**9.** Composition for use of **item 8**, wherein the therapeutic peptide or protein is or is derived from an antibody, an antibody fragment, an intrabody, a receptor, a binding protein, a CRISPR-associated endonuclease, a transcription factor, an enzyme, a growth factor, a structural protein, a cytoplasmic or cytoskeletal protein, or fragments, variants, or combinations of any of these.
**10.** Composition for use of **item 9**, wherein the CRISPR-associated endonuclease, is or is derived from Cas9, Cpf1, C2c1, C2c3, and C2c2, Cas13, CasX and CasY, or fragments, variants, or combinations of any of these.
**11.** Composition for use of **item 9**, wherein the antibody, or antibody fragment, is or is derived from an antibody or antibody fragment against a platelet derived growth factor (PDGF), or an antibody or antibody fragment against vascular endothelial growth factor (VEGF).
**12.** Composition for use of **any one of the preceding items**, wherein the at least one peptide or protein is selected from ABCA4, ADRB1, ANGPT1, ANGPT2, BEST1, CCR3, CD276, CD59, CFD, CHM, CHST4, CNR1, CNTF, CRYAA, CRYAB, CSF3R, DCN, DICER1, DRD2, EGFR, EGLN1, ENG, FLT1, FLT1(1-758), FLT1-iso3(sFlt1-14), FLT1-iso2(sFlt1), FLT1-iso4, GUCY1A1, GUCY1A2, GUCY1B1, GUCY1B2, GUCY2D, GUCY2F, HEY1, HEPH, HEPHL1, IL1RN, KDR, MAG, MERTK, MYO7A, MYOC, NOTCH4, NR3C1, NXNL1, OPA1, OPA3, OPTN, PDGFA, PDGFB, PDGFC, PDGFD, PDGFRA, PDGFRB, PGF, PLXND1, PPP3CA, PPP3CB, PPP3CC, PPP3R1, PPP3R2, PRPH2, PTGDR, PTGDR2, PTGER1, PTGER2, PTGER3, PTGER4, PTGFR, PTGIR, RHO, RLBP1, ROCK1, ROCK2, RPE65, RPGR, RS1, SEMA3E, SEMA3A, SERPINF1, SIRT1, SIRT2, SIRT3, SIRT4, SIRT5, SIRT6, SIRT7, SOD1, TBXA2R, TEK, TIE1, TNF, TNFRSF10C, UNC5B, USH2A, VEGFA, VEGFA-isoVEGF165, VEGFA-isoVEGF165B, VEGFB, VEGFC, VEGFD, WFS1, aflibercept, etanercept, bevacizumab, ranibizumab, anti-CCL11 antibody, anti-PDGFB antibody, anti-PDGFA antibody, bersanlimab, clazakizumab, olokizumab, sarilumab, siltuximab, sirukumab, DPP4-Fc, IL6R-Fc, PDGFRB-Fc, TNFRSF10A-Fc, TNFRSF10B-Fc, TNFRSF1A-Fc, TNFRSF1B-Fc, UNC5B-Fc, PDGFRB-trap(lgG4), PDGFRB-trap(lgG1), Cas9, Cpf1, C2c1, C2c3, and C2c2, Cas13, CasX and CasY, or a fragment or variant thereof
**13.** Composition for use of **any one of the preceding items**, wherein the at least one coding sequence encodes at least one peptide or protein comprising or consisting of an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 2-365, 436 or a fragment or variant thereof of any of these sequences.
**14.** Composition for use of **any one of the preceding items**, wherein the at least one coding sequence encodes at least one CRISPR-associated endonuclease comprising or consisting of an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 428-1345, 10999-11001 of published PCT patent application WO2018/172556.
**15.** Composition for use of **any one of the preceding items**, wherein the at least one coding RNA and/or the at least one coding sequence comprise at least one modified nucleotide preferably selected from pseudouridine (ψ), N1- methylpseudouridine (m1ψ), 5-methylcytosine, and 5-methoxyuridine.
**16.** Composition for use of **any one of the preceding items**, wherein the at least one coding sequence is a codon modified coding sequence, wherein the amino acid sequence encoded by the at least one codon modified coding sequence is preferably not being modified compared to the amino acid sequence encoded by the corresponding wild type coding sequence.
**17.** Composition for use of **item 16**, wherein the at least one codon modified coding sequence is selected from C maximized coding sequence, CAI maximized coding sequence, human codon usage adapted coding sequence, G/C content modified coding sequence, and G/C optimized coding sequence, or any combination thereof.
**18.** Composition for use of **any one of the preceding items**, wherein the least one coding RNA is an mRNA, a self-replicating RNA, a circular RNA, a viral RNA, or a replicon RNA, preferably an mRNA.
**19.** Composition for use of **any one of the preceding items**, wherein the least one coding RNA comprises a 5'-cap structure, preferably m7G, cap0, cap1, cap2, a modified capO or a modified cap1 structure.
**20.** Composition for use of **any one of the preceding items**, wherein the least one coding RNA comprises at least one poly(A) sequence, and/or at least one poly(C) sequence, and/or at least one histone stem-loop.
**21.** Composition for use of **any one of the preceding items**, wherein the at least one coding RNA comprises at least one heterologous 5'-UTR and/or at least one heterologous 3'-UTR.
**22.** Composition for use of **item 21**, wherein the at least one heterologous 3'-UTR comprises a nucleic acid sequence derived from a 3'-UTR of a gene selected from PSMB3, ALB7, alpha-globin, CASP1, COX6B1, GNAS, NDUFA1 and RPS9, or from a homolog, a fragment or a variant of any one of these genes.
**23.** Composition for use of **item 21**, wherein the at least one heterologous 5'-UTR comprises a nucleic acid sequence derived from a 5'-UTR of a gene selected from HSD17B4, RPL32, ASAH1, ATP5A1, MP68, NDUFA4, NOSIP, RPL31, SLC7A3, TUBB4B and UBQLN2, or from a homolog, a fragment or variant of any one of these genes.
**24.** Composition for use of **any one of the preceding items**, additionally comprising at least one pharmaceutically acceptable carrier or excipient, wherein the carrier or excipient is suitable for ocular administration, preferably for SCS administration.
**25.** Composition for use of **any one of the preceding items**, comprising at least one coding RNA encoding a CRISPR-associated protein, preferably at least one RNA which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 11011-11042; 11249-11280; 11131-11162; 11367-11398; 11485-11516; 11603-11634; 11721-11752; 11839-11870; 11044-11116; 11282-11354; 11164-11236; 11400-11472; 11518-11590; 11636-11708; 11754-11826; 11872-11944; 11011-11042; 11249-11280; 11044-11116; 11282-11354; 11131-11162; 11367-11398; 11485-11516; 11603-11634; 11721-11752; 11839-11870; 11164-11236; 11400-11472; 11518-11590; 11636-11708; 11754-11826; 11872-11944; 11120-11122; 11240; 11241; 11358; 11359; 11476; 11477; 11594; 11595; 11712; 11713; 11830; 11831; 11948; 11949; 11123-11130; 11360-11366; 11242-11248; 11478-11484; 11596-11602; 11714-11720; 11832-11838; 11950-11956, of WO2018/172556, and additionally, at least one guide RNA.
**26.** Composition for use of **any one of the preceding items**, wherein the at least one coding RNA is complexed or associated with or at least partially complexed or partially associated with one or more cationic or polycationic compound, preferably cationic or polycationic polymer, cationic or polycationic polysaccharide, cationic or polycationic lipid, cationic or polycationic protein, or cationic or polycationic peptide, or any combinations thereof.
**27.** Composition for use of **item 26**, wherein the one or more cationic or polycationic peptides are selected from SEQ ID NO: 432 to 435, or 437, or any combinations thereof.
**28.** Composition for use of **item 26**, wherein the cationic or polycationic polymer is a polyethylene glycol/peptide polymer comprising HO-PEG5000-S-(S-CHHHHHHRRRRHHHHHHC-S-)7-S-PEG5000-OH (SEQ ID NO: 435 of the peptide monomer).
**29.** Composition for use of **item 26**, wherein the cationic or polycationic polymer is a polyethylene glycol/peptide polymer comprising HO-PEG5000-S-(S-CGHHHHHRRRRHHHHHGC-S-)4-S-PEG5000-OH (SEQ ID NO: 437 of the peptide monomer).
**30.** Composition for use of **item 28 or 29**, wherein the composition comprises a lipid component or a lipidoid component.
**31.** Composition for use of **item 27**, wherein the at least one coding RNA is complexed, partially complexed, encapsulated, partially encapsulated, or associated with one or more lipids, thereby forming liposomes, lipid nanoparticles, lipoplexes, and/or nanoliposomes.
**32.** Composition for use of **any one of the preceding items**, wherein the composition is administered in Ringer or Ringer-Lactate solution.
**33.** Composition for use of **any one of the preceding items**, wherein administration into the SCS is performed by an injection needle, a microneedle, an injection device, a catheter, an implant delivery device, or a microcannula.
**34.** A kit or kit of parts comprising a composition as defined in items 1 to 33 **for use in treatment** of an ophthalmic disease, disorder or condition, wherein said kit or kit of parts is administered into the SCS to a subject in need of treatment.
**35.** Kit or kit of parts for use of **item 30**, comprising an injection needle, a microneedle, an injection device, a microinjection device, a catheter, an implant delivery device, or a microcannula.
**36.** A method of treating an ophthalmic disease, disorder, or condition comprising the steps of:
   a) providing a composition comprising at least one coding RNA, preferably as defined in items 1-33;
   b) administering said composition to the SCS of a subject;
   c) optionally, administering a further substance;
   d) optionally, administering a further coding RNA and/or guide RNA.
**37.** A method of treating of **item 36**, wherein the method is for gene therapy, comprising applying or administering to a subject in need thereof a composition comprising a coding RNA encoding at least one CRISPR-associated endonuclease, and at least one guide RNA, via administration into the SCS of a subject.
**38.** A method of delivering a coding RNA to cells and/or tissues of the eye of a subject, said method comprising the steps of providing a composition comprising said coding RNA, and delivering said composition to cells and/or tissues of the eye of the subject, wherein delivering is performed by administering said composition to the SCS of the subject.
**39.** A method of delivering of **item 38**, wherein the composition is as defined in items 1 to 33.
**40.** A method of treating of **items 36 to 37**, or a method of delivering of items **38 to 39**, wherein administration to the SCS is performed by an injection needle, a microneedle, an injection device, a microinjection device, a catheter, an implant delivery device, or a microcannula.

## Claims

1. A composition comprising at least one coding RNA, wherein said at least one coding RNA comprises at least one coding sequence encoding at least one peptide or protein for use in treatment or prevention of an ophthalmic disease, disorder or condition, wherein said composition is administered into the suprachoroidal space (SCS) to a subject in need thereof.

2. Composition for use of claim 1, wherein administration into the SCS results in expression and/or activity of the encoded peptide or protein in cells and/or tissues of the eye, preferably in cells and/or tissues located in the posterior segment of the eye,
wherein the cells and/or tissues of the eye are preferably selected from sclera, choroid, retina, optic nerve, macula, scleral cells, choroid plexus epithelial cells, retinal cells, retinal pigment epithelium, bruch's membrane, and retinal blood vessels.

3. Composition for use of claim 2, wherein the cells from retina are selected from photoreceptor cells, bipolar cells, ganglion cells, horizontal cells, and amacrine cells.

4. Composition for use of any one of the preceding claims, wherein administered into the SCS leads to translation of the encoded peptide or protein, thereby exerting a therapeutic effect,
wherein the therapeutic effect is preferably selected from reduced neovascularization, reduced inflammation, neuroprotection, complement inhibition, reduced drusen formation, reduced scar formation, and/or reduction in choriocapillaris.

5. Composition for use of any one of the preceding claims, wherein the ophthalmic disease, disorder or condition is selected from neovascularization, retinal degenerative disease, diabetic eye disease, retinal detachment, optic nerve disease, endocrine disorders, cancer disease, infectious disease, parasitic disease, pigmentary uveitis, branch retinal vein occlusion, central retinal vein occlusion, macular edema, cystoid macular edema, uveitic macular edema, cytomegalovirus retinitis, endophthalmitis, scleritis, choriotetinitis, dry eye syndrome, Norris disease, Coat's disease, persistent hyperplastic primary vitreous, familial exudative vitreoretinopathy, Leber congenital amaurosis, X-linked retinoschisis, Leber's hereditary optic neurophathy, uveitis, refraction and accommodation disorders, keratoconus, amblyopia, conjunctivitis, corneal ulcers, dacryocystitis, Duane retraction syndrome, optic neuritis, ocular inflammation, glaucoma, macular degeneration, and uveitis, or any disease, disorder or condition related or associated thereto.

6. Composition for use of any one of the preceding claims, wherein the at least one peptide or protein is or is derived from a therapeutic peptide or protein,
wherein the therapeutic peptide or protein preferably is or is derived from an antibody, an antibody fragment, an intrabody, a receptor, a binding protein, a CRISPR-associated endonuclease, a transcription factor, an enzyme, a growth factor, a structural protein, a cytoplasmic or cytoskeletal protein, or fragments, variants, or combinations of any of these.

7. Composition for use of claim 6, wherein the CRISPR-associated endonuclease, is or is derived from Cas9, Cpf1, C2c1, C2c3, and C2c2, Cas13, CasX and CasY, or fragments, variants, or combinations of any of these
or
wherein the antibody, or antibody fragment, is or is derived from an antibody or antibody fragment against a platelet derived growth factor (PDGF), or an antibody or antibody fragment against vascular endothelial growth factor (VEGF).

8. Composition for use of any one of the preceding claims, wherein the at least one peptide or protein is selected from ABCA4, ADRB1, ANGPT1, ANGPT2, BEST1, CCR3, CD276, CD59, CFD, CHM, CHST4, CNR1, CNTF, CRYAA, CRYAB, CSF3R, DCN, DICER1, DRD2, EGFR, EGLN1, ENG, FLT1, FLT1 (1-758), FLT1-iso3(sFlt1-14), FLT1-iso2(sFlt1), FLT1-iso4, GUCY1A1, GUCY1A2, GUCY1B1, GUCY1B2, GUCY2D, GUCY2F, HEY1, HEPH, HEPHL1, IL1RN, KDR, MAG, MERTK, MYO7A, MYOC, NOTCH4, NR3C1, NXNL1, OPA1, OPA3, OPTN, PDGFA, PDGFB, PDGFC, PDGFD, PDGFRA, PDGFRB, PGF, PLXND1, PPP3CA, PPP3CB, PPP3CC, PPP3R1, PPP3R2, PRPH2, PTGDR, PTGDR2, PTGER1, PTGER2, PTGER3, PTGER4, PTGFR, PTGIR, RHO, RLBP1, ROCK1, ROCK2, RPE65, RPGR, RS1, SEMA3E, SEMA3A, SERPINF1, SIRT1, SIRT2, SIRT3, SIRT4, SIRT5, SIRT6, SIRT7, SOD1, TBXA2R, TEK, TIE1, TNF, TNFRSF10C, UNC5B, USH2A, VEGFA, VEGFA-isoVEGF165, VEGFA-isoVEGF165B, VEGFB, VEGFC, VEGFD, WFS1, aflibercept, etanercept, bevacizumab, ranibizumab, anti-CCL1 1 antibody, anti-PDGFB antibody, anti-PDGFA antibody, bersanlimab, clazakizumab, olokizumab, sarilumab, siltuximab, sirukumab, DPP4-Fc, IL6R-Fc, PDGFRB-Fc, TNFRSF10A-Fc, TNFRSF1 0B-Fc, TNFRSF1A-Fc, TNFRSF1B-Fc, UNC5B-Fc, PDGFRB-trap(lgG4), PDGFRB-trap(lgG1), Cas9, Cpf1, C2c1, C2c3, and C2c2, Cas13, CasX and CasY, or a fragment or variant thereof.

9. Composition for use of any one of the preceding claims, wherein the at least one coding sequence encodes at least one peptide or protein comprising or consisting of an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 2-365, 436 or a fragment or variant thereof of any of these sequences
or
wherein the at least one coding sequence encodes at least one CRISPR-associated endonuclease comprising or consisting of an amino acid sequences being identical or at least 70%, 80%, 85%, 86%, 87%, 88%, 89%, 90%, 91%, 92%, 93%, 94%, 95%, 96%, 97%, 98%, or 99% identical to any one of SEQ ID NOs: 428-1345, 10999-11001 of published PCT patent application WO 2018/172556.

10. Composition for use of any one of the preceding claims, wherein the at least one coding RNA and/or the at least one coding sequence comprise at least one modified nucleotide preferably selected from pseudouridine (ψ), N1-methylpseudouridine (m1ψ), 5-methylcytosine, and 5-methoxyuridine.

11. Composition for use of any one of the preceding claims, wherein the at least one coding sequence is a codon modified coding sequence, wherein the amino acid sequence encoded by the at least one codon modified coding sequence is preferably not being modified compared to the amino acid sequence encoded by the corresponding wild type coding sequence,
wherein the at least one codon modified coding sequence is preferably selected from C maximized coding sequence, CAI maximized coding sequence, human codon usage adapted coding sequence, G/C content modified coding sequence, and G/C optimized coding sequence, or any combination thereof.

12. Composition for use of any one of the preceding claims, wherein the least one coding RNA is an mRNA, a self-replicating RNA, a circular RNA, a viral RNA, or a replicon RNA, preferably an mRNA.

13. Composition for use of any one of the preceding claims, wherein the least one coding RNA comprises a 5'-cap structure, preferably m7G, cap0, cap1, cap2, a modified capO or a modified cap1 structure
and/or
wherein the least one coding RNA comprises at least one poly(A) sequence, and/or wherein the at least one coding RNA comprises at least one heterologous 5'-UTR and/or at least one heterologous 3'-UTR.

14. Composition for use of any one of the preceding claims, additionally comprising at least one pharmaceutically acceptable carrier or excipient, wherein the carrier or excipient is suitable for ocular administration, preferably for SCS administration.

15. Composition for use of any one of the preceding claims, comprising at least one coding RNA encoding a CRISPR-associated protein, preferably at least one RNA which is identical or at least 70%, 80%, 85%, 86%, 87%, 88%, 89%, 90%, 91%, 92%, 93%, 94%, 95%, 96%, 97%, 98%, or 99% identical to a nucleic acid sequence selected from the group consisting of SEQ ID NOs: 11011-11042; 11249-11280; 11131-11162; 11367-11398; 11485-11516; 11603-11634; 11721-11752; 11839-11870; 11044-11116; 11282-11354; 11164-11236; 11400-11472; 11518-11590; 11636-11708; 11754-11826; 11872-11944; 11011-11042; 11249-11280; 11044-11116; 11282-11354; 11131-11162; 11367-11398; 11485-11516; 11603-11634; 11721-11752; 11839-11870; 11164-11236; 11400-11472; 11518-11590; 11636-11708; 11754-11826; 11872-11944; 11120-11122; 11240; 11241; 11358; 11359; 11476; 11477; 11594; 11595; 11712; 11713; 11830; 11831; 11948; 11949; 11123-11130; 11360-11366; 11242-11248; 11478-11484; 11596-11602; 11714-11720; 11832-11838; 11950-11956, of WO2018/172556, and additionally, at least one guide RNA.

16. Composition for use of any one of the preceding claims, wherein the at least one coding RNA is complexed or associated with or at least partially complexed or partially associated with one or more cationic or polycationic compound, preferably cationic or polycationic polymer, cationic or polycationic polysaccharide, cationic or polycationic lipid, cationic or polycationic protein, or cationic or polycationic peptide, or any combinations thereof.

17. Composition for use of claim 16, wherein the at least one coding RNA is in association or in complexation with a polymeric carrier, a polycationic protein or peptide, a lipid nanoparticle, or a liposome.

18. Composition for use of claim 16, wherein the at least one coding RNA is complexed, partially complexed, encapsulated, partially encapsulated, or associated with one or more lipids, thereby forming liposomes, lipid nanoparticles, lipoplexes, and/or nanoliposomes.

19. Composition for use of any one of the preceding claims, wherein the composition is administered in Ringer or Ringer-Lactate solution and/or wherein administration into the SCS is performed by an injection needle, a microneedle, an injection device, a catheter, an implant delivery device, or a microcannula.

20. A kit or kit of parts comprising a composition as defined in claims 1 to 19 for use in treatment of an ophthalmic disease, disorder or condition, wherein said kit or kit of parts is administered into the SCS to a subject in need of treatment, preferably comprising an injection needle, a microneedle, an injection device, a microinjection device, a catheter, an implant delivery device, or a microcannula.
